(19) Europäisches Patentamt
European Patent Office
Office européen des brevets

(11) EP 0 850 921 B1

(12) FASCICULE DE BREVET EUROPEEN

(45) Date de publication et mention de la délivrance du brevet: **25.09.2002 Bulletin 2002/39**

(21) Numéro de dépôt: **97403189.0**

(22) Date de dépôt: **30.12.1997**

(51) Int Cl.[7]: **C07C 317/44**, C07C 255/17, C07C 255/65, C07C 255/27, C07C 255/05, C07C 255/35, C08F 220/44, C07C 255/31, C08G 65/48, C08G 73/06, C08G 77/44, C08G 73/02, C07F 17/02, C07F 7/18, C07C 311/02, C09K 3/00, H01M 6/16, H01M 10/40, C07B 41/00, C08F 4/00, C08J 3/24

# (54) Matériaux à conduction ionique comprenant un composé ionique dérivé du malononitrile et leurs utilisations

Ionisch leitfähige Materialien, die eine von Malononitril abgeleitete ionische Verbindung enthalten und ihre Verwendungen

Ionic conductive materials comprising an ionic compound, derived from malononitrile and uses thereof

(84) Etats contractants désignés:
**DE FR GB IT**

(30) Priorité: **30.12.1996 CA 2194127**
**05.03.1997 CA 2199231**

(43) Date de publication de la demande:
**01.07.1998 Bulletin 1998/27**

(60) Demande divisionnaire:
**01129670.4 / 1 201 650**

(73) Titulaires:
- **CENTRE NATIONAL DE LA RECHERCHE SCIENTIFIQUE (CNRS) 75016 Paris (FR)**
- **HYDRO-QUEBEC Montréal Québec H2Z 1A4 (CA)**

(72) Inventeurs:
- **Armand, Michel Montreal, Québec H3T 1N2 (CA)**
- **Choquette, Yves Sainte-Julie, Québec J3E 1P4 (CA)**
- **Gauthier, Michel La Prairie, Québec J5R 1E6 (CA)**
- **Michot, Christophe 38000 Grenoble (FR)**

(74) Mandataire: **Sueur, Yvette et al**
**Cabinet SUEUR & L'HELGOUALCH, 109, boulevard Haussmann 75008 Paris (FR)**

(56) Documents cités:
**WO-A-93/09092**
**DD-A- 118 433**
**US-A- 4 966 954**
**US-A- 5 232 940**

- **A. DORNOW ET AL.: "Über Umsetzungen von alpha-Ketonitrilen, V. Über die Verwendung von alpha-Ketonitrilen zur C-Alkylierung" CHEMISCHE BERICHTE, vol. 91, no. 9, 1958, WEINHEIM, DE, pages 1824-29, XP002061822**
- **H. HIROTA ET AL.: "facile carbon-carbon bond heterolysis. SN1-E1 Reactions of t-cumyl and t-butyl substituted tricyanomethanes" CHEMISTRY LETTERS, no. 5, mai 1990, TOKYO, JP, pages 803-6, XP002061823**
- **H.W. ROESKY ET AL.: "Synthese und Struktur des Trifluoracetyldicyanomethanids" ZEITSCHRIFT FÜR NATURFORSCHUNG, TEIL B, ANORGANISCHE CHEMIE, ORGANISCHE CHEMIE, vol. 40b, no. 7, juillet 1985, TÜBINGEN, DE, pages 883-5, XP002061824**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 850 921 B1

- J.A. ELVIDGE ET AL.: "Preparation of some highly polarised ethenes by the addition of amines to suitable carbonitriles" JOURNAL OF THE CHEMICAL SOCIETY, PERKIN TRANSACTIONS I, no. 8, 1983, LONDON, GB, pages 1741-4, XP002061825
- E. OTT ET AL.: "Zur Kenntnis einfacher Cyanverbindungen, IV. Mitteil.: Über das Dibrom-malonitril und seine Umwandlung in Natrium-azidomalonitril und in ein dimolekulares Cyan-azid C2N8" CHEMISCHE BERICHTE, vol. 70, no. 8, 1937, WEINHEIM, DE, pages 1829-34, XP002061826
- J.-P. FLEURY ET AL.: "Carboacides polycyanés. II. Sulfonylmalodinitriles. Préparation, structure et propriétés" BULLETIN DE LA SOCI T CHIMIQUE DE FRANCE, 1966, PARIS, FR, pages 1966-70, XP002061827
- B.K. FREED ET AL.: "(Alpha-perfluoroheptyl-beta,beta-dicyanovinyl)aminostyrenes: a novel class of perfluorocarbon-soluble dyes" JOURNAL OF FLUORINE CHEMISTRY, vol. 47, no. 2, mai 1990, LAUSANNE, CH, pages 219-25, XP002061828
- DATABASE CROSSFIRE Beilstein Informationssysteme GmbH, Frankfurt DE XP002061829 & J. ORG. CHEM. USSR (ENGL. TRANSL.), vol. 27, no. 6.1, 1991, page 1042-8

## Description

La présente invention a pour objet des matériaux à conduction ionique comprenant un composé ionique dérivé du malononitrile dans lequel la charge anionique est délocalisée, et leurs utilisations.

Il est connu et particulièrement intéressant d'introduire des groupements ioniques dans les molécules ou les polymères organiques possédant des fonctions variées. Les forces coulombiennes correspondent, en effet, aux interactions les plus fortes disponibles au niveau moléculaire, et les groupements ioniques modifient de la manière la plus marquée les molécules auxquelles ils sont attachés. On peut citer les colorants qui sont rendus solubles dans l'eau à l'aide de fonctions sulfonates ou carboxylates.

Cependant les groupements de ce type $-CO_2^-$ $1/mM^{m+}$ ou $-SO_3^-$ $1/mM^{m+}$ ne sont pas dissociés, et ils n'induisent pas de solubilité dans les solvant autres que l'eau ou certains solvants protiques très polaires comme les alcools légers, ce qui restreint considérablement la portée de leur utilisation.

On connaît par ailleurs **(EP-A-0 290 511)** les sels des composés $[R_FSO_2\text{-}N\text{-}SO_2R_F]^-$ $1/mM^{m+}$ dans lesquels $R_F$ est un groupement perfluoré et $M^{m+}$ un cation à la valence m+ qui sont solubles et dissociés dans les milieux aprotiques organiques ou les polymères solvatants. Il est cependant considéré que l'existence de deux groupements perfluoroalkylsulfonyles (en particulier l'existence d'atomes de fluor sur l'atome de carbone en $\alpha$ de chacun des groupements sulfonyles) qui exercent un pouvoir attracteur important sur les électrons de la charge anionique, est une condition nécessaire à l'obtention des propriétés de solubilité et dissociation. Pour exemple, le $pK_a$ de l'acide H $[CF_3SO_2\text{-}N\text{-}SO_2CF_3]$ n'est que de 1,95, comparable à celui de l'acide non fluoré $CH_3SO_3H$ ($pK_a$ = 0,3) et nettement inférieur à celui de l'acide perfluoré $CF_3SO_3H$ ($pK_a$ < -9) du fait de la basicité de l'atome d'azote central.

On connaît en outre par WO93/09092 des composés répondant à la formule $(R_FSO_2)_2CY^-M^+$ et des matériaux à conduction ionique qui les contiennent. $R_F$ représente un groupe perfluoroalkyle, M une espèce cationique minérale ou organique et Y peut être un groupe nitrile.

D'une manière surprenante, les inventeurs ont trouvé que les composés contenant des groupements ioniques $-C(CN)_2^-$ avaient d'excellentes propriétés de solubilité et de dissociation, même lorsqu'ils ne contiennent pas de groupements perfluorés fortement électroattracteurs.

La présente invention a par conséquent pour but de fournir une famille de composés ioniques possédant une bonne solubilité et une bonne dissociation, sans qu'il soit nécessaire de faire appel à des modifications complexes de la molécule de départ. Les précurseurs des molécules de l'invention sont pour la plupart des produits industriels et/ou facilement accessibles. Il est à noter en outre que l'absence, ou tout au moins la diminution de la fraction perfluorée dans les composés de l'invention permet de réduire les coûts de production de composés et par conséquent le coût des applications qui en sont faites.

Un objet de la présente invention est un matériau à conduction ionique comprenant un composé ionique en solution dans un solvant, ledit composé ionique étant un dérivé du malononitrile comprenant une partie anionique associée à au moins une partie cationique $M^{+m}$ en nombre suffisant pour assurer la neutralité électronique de l'ensemble, caractérisé en ce que M est un hydroxonium, un nitrosonium $NO^+$, un ammonium $-NH_4^+$, un cation métallique ayant la valence m, un cation organique ayant la valence m ou un cation organométallique ayant la valence m, et en ce que la partie anionique répond à l'une des formules $R_D\text{-}Y\text{-}C(C\equiv N)_2^-$ ou $Z\text{-}C(C\equiv N)_2^-$ dans lesquelles :

- Z représente un radical électro-attracteur ayant un paramètre de Hammett au moins égal à celui d'un radical phényle, choisi parmi :

  j) -CN, $-NO_2$, -SCN, $-N_3$, $FSO_2$-, $-CF_3$, $R'_FCH_2$- ($R'_F$ étant un radical perfluoré, de préférence $CF_3$-), les radicaux fluoroalkyloxy, fluoroalkylthioxy, fluoroalkényloxy, fluoroalkénylthioxy ;

  jj) les radicaux comprenant un ou plusieurs noyaux aromatiques contenant éventuellement au moins un atome d'azote, d'oxygène, de soufre ou de phosphore, les dits noyaux pouvant être éventuellement des noyaux condensés et/ou lesdits noyaux pouvant éventuellement porter au moins un substituant choisi parmi les halogènes, -CN, $-NO_2$, -SCN, $-N_3$, $CF_2$=CF-O-, les radicaux $R_F$- et $R_FCH_2$- dans lesquels $R_F$ est un radical perfluoroalkyle ayant de 1 à 12 atomes de carbone, les groupes fluoroalkyloxy, les groupes fluoroalkylthioxy, les radicaux alkyles, alkényles, oxa-alkyles, oxaalkényles, azaalkyles, azaalkényles, thiaalkyles, thiaalkényles, les radicaux polymères, les radicaux possédant au moins un groupement ionophore cationique et/ou au moins un groupement ionophore anionique ;

- Y représente un groupement carbonyle, un groupement thiocarbonyle, un groupement sulfonyle, un groupement sulfinyle ou un groupement phosphonyle et :
- $R_D$ est un radical choisi parmi :

  a) les radicaux alkyle ou alkényle ayant de 1 à 24 atomes de carbone, et les radicaux aryle, arylalkyle, alkylaryle

ou alkénylaryle ayant de 5 à 24 atomes de **carbone,** les radicaux alicycliques ou hétérocycliques, y compris les radicaux polycycliques ;

b) les radicaux alkyle ou alkényle ayant de 1 à 12 atomes de carbone et comprenant éventuellement au moins un hétéroatome O, N ou S dans la chaîne principale ou dans une chaîne latérale, et/ou comprenant au moins un groupe fonctionnel éther, thioéther, amine, imine, amide, carboxyle, carbonyle, thiocarbonyle, isocyanate, isothiocyanate, hydroxy ; les radicaux oxa-alkyle, oxa-alkényle, aza-alkyle, aza-alkényle, thia-alkyle ou thia-alkényle ayant de 1 à 24 atomes de carbone ;

c) les radicaux aryle, arylalkyle, arylalkényle, alkylaryle ou alkénylaryle, dans lesquels les noyaux aromatiques et/ou au moins un substituant du noyau comprennent des hétéroatomes tels que l'azote, l'oxygène, le soufre ;

d) les radicaux comprenant des cycles aromatiques condensés qui comprennent éventuellement au moins un hétéroatome choisi parmi l'azote, l'oxygène, le soufre ;

e) les radicaux halogénés ou perhalogénés alkyle, alkényle, aryle, arylalkyle, alkylaryle, lesdits radicaux comprenant éventuellement des groupes fonctionnels éther, thioéther, imine, amine, carboxyle, carbonyle ou hydroxy ;

f) les radicaux $R_CC(R')(R'')$-O- dans lesquels $R_C$ est un radical alkylperfluoré et R' et R'' sont indépendamment l'un de l'autre un atome d'hydrogène ou un radical tel que défini en a), b), c) ou d) ci-dessus [par exemple $CF_3CH_2O$-, $(CF_3)_3CO$-, $(CF_3)_2CHO$-, $CF_3CH(C_6H_5)O$-, $-CH_2(CF_2)_2CH_2$-] ;

g) les radicaux $(R_B)_2N$-, dans lesquels les radicaux $R_B$ identiques ou différents sont tels que définis en a), b), c), d) et e) ci-dessus, l'un des $R_B$ pouvant être un atome d'hydrogène, ou bien les deux radicaux $R_B$ forment ensemble un radical divalent qui forme un cycle avec N ;

h) les radicaux poly(oxyalkylène) et poly(oxyalkylène)-alkyléther,

i) les radicaux possédant un ou plusieurs groupements ionophores cationiques et/ou un ou plusieurs groupements ionophores anioniques ;

étant entendu que :

- un substituant Z peut être un radical monovalent, une partie d'un radical ayant une valence égale à 2 portant deux groupements $-C^-(C{\equiv}N)_2$' ou un segment d'un polymère ;
- un substituant $R_D$ peut être un radical monovalent, une partie d'un radical ayant une valence supérieure à 2 portant plusieurs groupements $-Y-C^-(C{\equiv}N)_2$' ou un segment d'un polymère ;
- lorsque Y est un carbonyle et $R_D$ est un radical perfluoroalkyle ayant de 1 à 3 atomes de carbone, ou lorsque Z est -CN, M est différent d'un métal alcalin ;
- "segment d'un polymère" signifie unité récurrente éthylène portant éventuellement un substituant alkylène ou un substituant oxyalkénylène, unité récurrente oxyalkylène portant éventuellement un groupe méthylène, unité récurrente styrène portant éventuellement un substituant polyoxyéthylène, unité récurrente de 2,3-époxypropane, unité récurrente azaéthylène, unité récurrente 2-(triéthoxysilyl)éthyl-co-O-[2-(triméthoxysilyl)éthyl]-O'-méthylpolyéthylène glycol, unité récurrente bis[3-triméthoxysilyl)propyl]amino, unité récurrente méthyléthylsiloxane, unité récurrente pyrrole, unité récurrente acrylonitrile-co-4-styrényle, ou unité récurrente aniline portant un substituant fluoroalkyle ;
- "groupement ionophore cationique" signifie un groupement iodonium, sulfonium, oxonium, ammonium, amidinium, guanidinium, pyridinium, imidazolium, imidazolinium, triazolium, phosphonium ou carbonium, ledit groupement ionique jouant totalement ou partiellement le rôle du cation $M^+$ ;
- "groupement ionophore anionique" signifie une fonction carboxylate ($-CO_2^-$), une fonction sulfonate ($-SO_3^-$), une fonction sulfonimide ($-SO_2NSO_2$-) ou une fonction sulfonamide ($-SO_2N$-).

Dans un mode de réalisation de l'invention, le cation est un cation métallique choisi parmi les cations de métaux alcalins, les cations de métaux alcalino-terreux, les cations de métaux de transition, les cations de métaux trivalents, les cations de terres rares. A titre d'exemple, on peut citer $Na^+$, $Li^+$, $K^+$, $Sm^{3+}$, $La^{3+}$, $Ho^{3+}$, $Sc^{3+}$, $Al^{3+}$, $Y^{3+}$, $Yb^{3+}$, $Lu^{3+}$, $Eu^{3+}$.

Le cation peut également être un cation organométallique, notamment un métallocénium. A titre d'exemple, on peut citer les cations dérivés du ferrocène, du titanocène, du zirconocène, d'un indénocénium ou d'un arène métallocénium, les cations des métaux de transition complexés par des ligands de type phosphine possédant éventuellement une chiralité, les cations organométalliques possédant un ou plusieurs groupements alkyles ou aryles fixés d'une manière covalente à un atome ou un groupe d'atome. Comme exemples particuliers, on peut citer les cations méthylzinc, phénylmercure, trialkylétain ou trialkylplomb, chloro[éthylènebis(indényl)] zirconium(IV) ou tétrakis(acétonitrile)-palladium(II). Le cation organo-métallique peut faire partie d'une chaîne polymère.

Dans un autre mode de réalisation particulier de l'invention, le cation organique est un cation onium choisi dans le groupe constitué par les cations $R_3O^+$ (oxonium), $NR_4^+$ (ammonium), $RC(NR_2)_2^+$ (amidinium), $C(NR_2)_3^+$ (gua-

nidinium), $C_5R_6N^+$ (pyridinium), $C_3R_5N_2^+$ (imidazolium), $C_3R_7N_2^+$ (imidazolinium), $C_2R_4N_3^+$ (triazolium), $SR_3^+$ (sulfonium), $PR_4^+$ (phosphonium), $IR_2^+$ (iodonium), $(C_6R_5)_3C^+$ (carbonium). Dans un cation onium donné, les radicaux R peuvent être tous identiques. Mais un cation onium peut aussi comporter des radicaux R différents les uns des autres. Un radical R peut être un H ou bien il est choisi parmi les radicaux suivants :

- les radicaux alkyles, alkényles, oxa-alkyles, oxa-alkényles, aza-alkyles, aza-alkényles, thia-alkyles, thia-alkényles, aryles, arylalkyles, alkylaryles, alkénylaryles, les radicaux dialkylamino et les radicaux dialkylazo ;
- les radicaux cycliques ou hétérocycliques comprenant éventuellement au moins une chaîne latérale comprenant des hétéroatomes tels que l'azote, l'oxygène, le soufre ;
- les radicaux cycliques ou hétérocycliques comprenant éventuellement des hétéroatomes dans le noyau aromatique ;
- les groupes comprenant plusieurs noyaux aromatiques ou hétérocycliques, condensés ou non, contenant éventuellement au moins un atome d'azote, d'oxygène, de soufre ou de phosphore.

Lorsqu'un cation onium porte au moins deux radicaux R différents de H, ces radicaux peuvent former ensemble un cycle aromatique ou non, englobant éventuellement le centre portant la charge cationique.

Lorsque la partie cationique d'un composé de l'invention est un cation onium, il peut se présenter soit sous la forme d'un groupe cationique indépendant qui n'est lié à la partie cationique que par la liaison ionique entre la charge positive du cation et la charge négative de la partie anionique. Dans ce cas, la partie cationique peut faire partie d'une unité récurrente d'un polymère.

Un cation onium peut également faire partie du radical Z ou du radical $R_D$ porté par le centre anionique. Dans ce cas, un composé de l'invention constitue un zwitterion. Lorsque le cation d'un composé de l'invention est un cation onium, il peut être choisi de telle sorte à introduire dans le composé des substituants permettant de conférer audit composé des propriétés spécifiques. Par exemple, le cation $M^+$ peut être un hétérocycle cationique à caractère aromatique, comportant au moins un atome d'azote alkylé dans le cycle. A titre d'exemple, on peut citer un imidazolium, un triazolium, un pyridinium, un 4-diméthylamino-pyridinium, lesdits cations portant éventuellement un substituant sur les atomes de carbone du cycle. Parmi ces cations, ceux dont les sels ont un point de fusion inférieur à 150°C, plus particulièrement inférieur à 25°C.

Un composé de l'invention dans lequel le cation M est un groupement cationique possédant un groupement diazoïque -N=N-, $-N=N^+$, un groupement sulfonium, un groupement iodonium, un groupement phosphonium ou un cation arène-ferrocénium substitué ou non, éventuellement incorporé dans une trame polymérique, est intéressant dans la mesure où il est activable par une source d'énergie actinique de longueur d'onde appropriée. Comme exemples particuliers de tels composés, on peut citer ceux dans lesquels le cation est un cation diaryliodonium, un cation dialkylaryliodonium, un cation triarylsulfonium, un cation trialkylaryle sulfonium, ou un cation phénacyl-dialkyl sulfonium substitué ou non. Les cations précités peuvent faire partie d'une chaîne polymère.

Le cation M d'un composé de l'invention peut être un cation organique incorporant un groupement 2,2'[Azobis (2-2'-imidazolinio-2-yl)propane]$^{2+}$ ou 2,2'-Azobis(2-amidiniopropane)$^{2+}$. Le composé de l'invention est alors particulièrement intéressant comme initiateur radicalaire, activable thermiquement et non volatil, soluble dans les solvants organiques polaires et dans les monomères et polymères solvatants aprotiques.

Une famille particulière de composés de l'invention est celle qui comprend un groupe $R_DY$-. Les composés dans lesquels Y est $-SO_2$- ou -CO- sont spécialement préférés.

Le choix du substituant $R_D$ permet d'ajuster les propriétés d'un composé de l'invention. Dans un mode de réalisation, $R_D$ est choisi parmi les radicaux alkyles, alkényle, oxa-alkyle, oxa-alkényle, aza-alkyle, aza-alkényle, thia-alkyle ou thia-alkényle ayant de 1 à 24 atomes de carbone, ou parmi les radicaux aryle, arylalkyle, alkylaryle ou alkénylaryle ayant de 5 à 24 atomes de carbones.

Dans un autre mode de réalisation, $R_D$ est choisi parmi les radicaux alkyles ou alkényles ayant de 1 à 12 atomes de carbone et comprenant éventuellement au moins un hétéroatome O, N ou S dans la chaîne principale ou dans une chaîne latérale, et/ou portant éventuellement un groupe hydroxy, un groupe carbonyle, un groupe amine ou un groupe carboxyle.

Dans un autre mode de réalisation, $R_D$ est choisi parmi les radicaux aryle, arylalkyle, alkylaryle ou alkénylaryle, dans lesquels les noyaux aromatiques et/ou leurs substituants comprennent des hétéroatomes tels que l'azote, l'oxygène, le soufre.

Un substituant $R_D$ peut être comme radical polymère, un radical poly(oxyalkylène) ou poly(oxyalklène) alkyléther. Le composé de l'invention se présente alors sous la forme d'un polymère portant un groupe ionique $-Y-C(CN)_2^-$, $M^+$.

$R_D$ peut être une unité récurrente d'un polymère, par exemple une unité oxyalkylène ou une unité styrène. Le composé de l'invention se présente alors sous la forme d'un polymère dans lequel une partie au moins des unités récurrentes portent un groupe latéral sur lequel est fixé un groupe ionique $-Y-C(CN)_2^-$, $M^+$. A titre d'exemple, on peut

citer un poly(oxyalkylène) dans lequel au moins certaines unités oxyalkylène portent un substituant $-Y-C(CN)_2^-$, $M^+$, ou un polystyrène dans lequel au moins certaines unités styrène portent un substituant $-Y-C(CN)_2^-$, $M^+$.

Une catégorie particulière de composés selon l'invention comprend les composés dans lesquels le substituant $R_D$ possède au moins un groupement ionophore anionique et /ou au moins un groupement ionophore cationique. Le groupement anionique peut par exemple être une fonction carboxylate ($-CO_2^-$), une fonction sulfonate ($-SO_3^-$), une fonction sulfonimide ($-SO_2NSO_2-$) ou une fonction sulfonamide ($-SO_2N-$). Le groupement ionophore peut par exemple être un groupement iodonium, sulfonium, oxonium, ammonium, amidinium, guanidinium, pyridinium, imidazolium, imidazolinium, triazolium, phosphonium ou carbonium. Le groupement ionophore cationique peut jouer totalement ou partiellement le rôle du cation M.

Lorsque $R_D$ comporte au moins une insaturation éthylénique et/ou un groupe condensable et/ou un groupe dissociable par voie thermique, par voie photochimique ou par dissociation ionique, les composés de l'invention sont des composés réactifs qui peuvent être soumis à des polymérisations, des réticulations ou des condensations, éventuellement avec d'autres monomères. Ils peuvent également être utilisés pour fixer des groupes ionophores sur les polymères portant la fonction réactive appropriée.

Un substituant $R_D$ peut être un groupe mésomorphe ou un groupe chromophore ou un polymère conducteur électronique autodopé ou un alcoxysilane hydrolysable.

Un substituant $R_D$ peut comporter un groupement susceptible de piéger les radicaux libres, par exemple un phénol encombré ou une quinone.

Un substituant $R_D$ peut aussi comporter un dipôle dissociant, par exemple une fonction amide, une fonction sulfonamide ou une fonction nitrile.

Un substituant $R_D$ peut aussi comporter un couple rédox, par exemple un groupe disulfure, un groupe thioamide, un groupe ferrocène, un groupe phénothiazine, un groupe bis(dialkylaminoaryle), un groupe nitroxyde ou un groupe imine aromatique.

Un substituant $R_D$ peut aussi comporter un ligand complexant, ou un groupe optiquement actif.

Une autre catégorie de composés de l'invention comprend les composés dans lesquels Y est un groupe carbonyle, $R_D$-CO-représentant un acide aminé, ou un polypeptide optiquement ou biologiquement actif.

Selon une autre variante, un composé selon l'invention comprend un substituant $R_D$ qui représente un radical ayant une valence v supérieure à deux, comportant lui-même au moins un groupe $-Y-C(CN)_2^-$, $M^+$. Dans ce cas, les charges négatives présentes sur la partie anionique du composé de l'invention devront être compensées par le nombre approprié de cations ou de groupes ionophores cationiques M.

Lorsqu'un composé de la présente invention répond à la formule $Z-C(CN)_2^-$, $M^+$ dans laquelle Z est un groupement électroattracteur non lié à l'azote portant la charge négative par un groupe $-SO_x-$, Z est avantageusement choisi dans le groupe constitué par $-OC_nF_{2n+1}$, $-OC_2F_4H$, $-SC_nF_{2n+1}$ et $-SC_2F_4H$, $-OCF=CF_2$, $-SCF=CF_2$, n étant un nombre entier de 1 à 8. Z peut également être un radical $C_nF_{2n+1}CH_2-$, n étant un nombre entier de 1 à 8.

Les composés de la présente invention peuvent être obtenus par un procédé dans lequel on fait réagir un composé $R_D$-Y-L ou Z-L avec un composé $[A-C(CN)_2]^{n-}{}_m\ nM'^{m+}$,

- Z et $R_D$ étant tels que définis précédemment,
- M' étant H ou un cation tel que défini précédemment pour M,
- L représentant un groupe partant électronégatif tel qu'un halogène, un radical N-imidazoyle, un radical N-triazoyle, un composé donnant un ester activé (par exemple un succinimidyloxy, un benzotriazoloxy ou une O-acylurée), un groupement alcoxyde, un groupement $R_D$-Y-O- ou un groupement $R_D$-Y-S-, et
- A représentant un cation $M^{m+}$, un groupe trialkylsilyle, un groupe trialkyle germanyle, un groupe trialkylstannyle ou un groupe tertioalkyle, dans lesquels les substituants alkyles ont de 1 à 6 atomes de carbone.

A titre d'exemple, on peut citer la réaction d'un fluorure de fluorosulfonyle avec un di sel de malononitrile selon le schéma réactionnel suivant :

$$FSO_2\text{-}F + [NaC(CN)_2]^- \ Na^+ \Rightarrow NaF + [FSO2\text{-}C(CN)_2]^- \ Na^+.$$

L'utilisation d'un composé $[A-C(CN)_2]^{n-}{}_m\ nM'^{m+}$ dans lequel A est un groupement tertioalkyle est avantageuse, car un tel groupement est précurseur de proton par formation de l'alcène correspondant. L'utilisation du groupe trialkylsilyle est spécialement intéressante lorsque le groupe partant est un fluor, du fait de la très grande stabilité de la liaison F-Si.

Lorsque l'on utilise un composé $[A-C(CN)_2]^{n-}{}_m\ nM'^{m+}$ dans lequel A est le proton, il est avantageux d'effectuer la réaction en présence d'une base tertiaire ou d'une base encombrée T susceptible de former le sel $L^-(HT^+)$ par combinaison avec le proton, afin de favoriser la formation du composé de l'invention. La base peut être choisie parmi

les alkylamines (par exemple la triéthylamine, la di-isopropylamine, la quinuclidine), le 1,4-diazobicyclo[2,2,2]octane (DABCO) ; les pyridines (par exemple la pyridine, les alkylpyridines, les dialkylaminopyridines) ; les imidazoles (par exemple les N-alkylimidazoles, l'imidazo[1,1-a]pyridine) ; les amidines (par exemple le 1,5-diazabicyclo-[4,3,0]non-5-ène (DBN), le 1,8-diazabicyclo[5,4,0]undec-7-ène (DBU)) ; les guanidines (par exemple la tétraméthyl guanidine, la 1,3,4,7,8-hexahydro-1-méthyl-2H-pyrimido[1,2-a]pyrimidine (HPP)). On peut également utilisé un sel de métal alcalin du malononitrile comme base.

A titre d'exemple d'un tel procédé, on peut citer le procédé dans lequel on fait réagir un chlorure de carbonyle $R_DCOCl$ avec le malononitrile en présence de DABCO.

Un composé selon l'invention peut également être obtenu par couplage direct entre un sel du malononitrile et un acide carboxylique à l'aide d'un agent de couplage. Lorsque Z = CO, il est avantageux d'utiliser un composé RZX du type pseudohalogénure préparé directement *in-situ* (X = $RCO_2$, SCO, PTO, BzO) à partir de RCOOH par action des agents de condensation utilisés dans la synthèse des peptides (déshydratants moléculaires). De tels agents sont décrits par exemple dans *Synthesis p.* 453 (1972) et dans *Ann. Rev. Biochem* 39, 841 (1970). Les composés de l'invention sont alors préparés à partir de RCOOH auquel est ajouté le déshydratant moléculaire, puis le composé $(1/nM)[(NC)_2CH]$ en proportions stoechiométriques, dans un solvant polaire. De préférence, l'agent de condensation est choisi parmi les carbodiimides, par exemple le cyclohexyl carbodiimide ou le diisopropyl carbodiimide ; les carbonates et les oxalates de succinimidyle, de phtalimidyle, de 1-benzotriazolyle, de nitro-, de dinitro- ou de perhalo-phénols, de trifluoroéthyle, de trichloroéthyle ; le mélange $P\Phi_3$-diéthylazodicarboxylate (DEAD) ou $P\Phi_3$-dithiodipyridine ; le carbonyldiimidazole $(Im)_2CO$ ou le phénylphosphoro-diimidazole $\Phi PO(Im)_2$ ; les acétals d'amides, par exemple, le diméthylformamide di-néopentyl acétal $(CH_3)_2NCH[OCH_2C(CH_2)_2]_2$ ; les 2-alcoxy-1-alcoxycarbonyl-1,2-dihydroquinoline ; les sels de O-benzotriazol-1-yl-N,N,N',N'-tétraméthyluronium ou de O-benzo triazol-1-yloxytrisdiméthylaminophosphonium ; les sultones aromatiques, par exemple, l'oxyde de 2,2-(6-nitronapht[1,8-cd]-1,2-oxathiole), le chloroformiate d'isobutyle, le diphé-nylphosphochloroiridate, le chlorophosphite d'éthylène, le pyrophosphite de diéthyléthylène ; le chlorure de bis(2-oxo-3-oxazolidinyl)phosphinyle ; les sels de 2-*ter*-butyl-5-méthyl isooxazolium (Woodward's reagent L).

Le cation d'un composé obtenu selon l'un ou l'autre des procédés décrits ci-dessus peut être remplacé par les procédés classiques d'échange de cation, soit par des précipitations ou des extractions sélectives, soit par l'utilisation de résines échangeuses d'ions.

En outre, le substituant $R_D$ d'un composé de l'invention peut être modifié par les réactions connues. Par exemple, un substituant $R_D$ qui comprend un groupe allyle peut être transformé par réaction avec un peroxyde pour obtenir un substituant $R_D$ époxydé. Un groupe -NHR peut être transformé en groupe vinylester par réaction avec du tert-butoxyde de potassium et le vinylchloroformate. Les procédés pour réaliser ces modifications et d'autres sont à la portée de l'homme de métier.

Les composés ioniques de la présente invention comprennent au moins un groupement ionophore sur lequel sont fixés des substituants qui peuvent être très variés. Compte tenu du grand choix possible pour les substituants, les composés de l'invention permettent d'induire des propriétés de conduction ionique dans la plupart des milieux organiques, liquides ou polymères possédant une polarité, même faible. Les applications sont importantes dans le domaine de l'électrochimie, en particulier du stockage de l'énergie dans des générateurs primaires ou secondaires, dans les supercapacités, dans les piles à combustibles et dans les diodes électroluminescentes. La compatibilité des composés ioniques de l'invention avec les polymères ou les liquides organiques permet d'induire des propriétés antistatiques marquées, même lorsque la teneur en composé ionique est extrêmement faible. Les composés de l'invention qui sont des polymères, de même que des composés polymères obtenus à partir de composés de l'invention ayant la propriété de se polymériser ou de se copolymériser, présentent les propriétés énumérées ci-dessus avec l'avantage d'avoir d'une charge anionique immobile.

Dans un mode de réalisation, le composé ionique utilisé pour l'élaboration d'un matériau à conduction ionique est choisi parmi les composés dont le cation est l'ammonium, ou un cation dérivé d'un métal, en particulier le lithium ou le potassium, le zinc, le calcium, les métaux des terres rares, ou un cation organique, tel qu'un ammonium substitué, un imidazolium, un triazolium, un pyridinium, un 4-diméthylamino-pyridinium, lesdits cations portant éventuellement un substituant sur les atomes de carbone du cycle. Le matériau à conduction ionique ainsi obtenu présente une conductivité et une solubilité dans les solvants élevées, du fait des interactions faibles entre la charge positive et la charge négative. Son domaine de stabilité électrochimique est étendu, et il est stable dans des milieux aussi bien réducteurs qu'oxydants. De plus, les composés qui ont un cation organique et un point de fusion inférieur à 150°C, en particulier les composés de imidazolium, de triazolium, de pyridinium, de 4-diméthylamino-pyridinium présentent une conductivité élevée intrinsèque, même en l'absence de solvant, lorsqu'ils sont en phase fondue.

Les propriétés du matériau à conduction ionique peuvent également être adaptées par le choix du substituant Y ou $R_D$.

Le choix pour $R_D$ d'un groupe alkyle, d'un groupe aryle, d'un groupe alkylaryle ou d'un groupe arylalkyle, permet d'induire dans le matériau à conduction ionique des propriétés de type mésogène, en particulier les groupements alkyles de 6 à 20 atomes de carbones, les groupements arylealkyle, en particulier ceux contenant l'entité biphényle qui forment des phases de type cristal liquide. Des propriétés de conduction dans des phases de type cristal liquide, nématique, cholestérique ou discotique, sont intéressantes pour les applications relatives à l'affichages optique ou pour réduire la mobilité des anions dans les électrolytes, en particulier dans les électrolytes polymères, sans affecter la mobilité des cations. Cette particularité est importante pour les applications dans les générateurs électrochimiques, en particulier ceux mettant en jeu les cations lithium.

Lorsque le substituant $R_D$ contient un groupe mésomorphe ou un groupe comprenant au moins une insaturation éthylénique et/ou un groupe condensable et/ou un groupe dissociable par voie thermique, par voie photochimique ou par dissociation ionique, le matériau à conduction ionique forme aisément des polymères ou copolymères qui sont des polyélectrolytes, soit intrinsèques quand le polymère porte des groupements solvatants, soit par addition d'un solvant polaire de type liquide ou polymère, ou par mélange avec un tel solvant. Ces produits ont une conductivité uniquement due au cations, ce qui constitue une propriété très utile dans les applications de type générateur électrochimique. En faible fraction molaire dans un copolymère, ils induisent des propriétés antistatiques stables et peu dépendantes de l'humidité et favorisent la fixation de colorants cationiques, cette propriété étant utile pour les fibres textiles et les lasers à colorants.

La présence d'un substituant $R_D$ qui est un polymère conducteur électronique autodopé, améliore la stabilité du matériau à conduction ionique par rapport aux agents extérieurs. La conductivité est stable dans le temps même à des températures élevées. En contact avec les métaux, ces matériaux donnent des résistances d'interface très faibles et protègent en particulier les métaux ferreux ou l'aluminium de la corrosion.

Lorsque le substituant $R_D$ est un alcoxysilane hydrolysable, le matériau à conduction ionique peut former des polymères stables par simple mécanisme d'hydrolyse-condensation en présence d'eau, permettant ainsi de traiter les surfaces d'oxydes, de silice, de silicates, en particulier le verre, pour induire des propriétés de conduction de surface, des propriétés antistatiques, ou pour favoriser l'adhésion de polymères polaires.

Lorsque le substituant $R_D$ est un groupe comprenant un piège à radicaux libres tel qu'un phénol encombré, ou une quinone, le matériau à conduction ionique présente les avantages et propriétés suivantes : il agit comme antioxydant ne présentant pas de volatilité et compatible avec les monomères et polymères polaires, auquel il confère de surcroît des propriétés antistatiques.

Lorsque le substituant $R_D$ comprend un dipôle dissociant tel qu'une amide, une sulfonamide ou un nitrile, le matériau à conduction ionique a une conductivité améliorée dans des milieux de faible et moyenne polarité, en particulier dans les polymères solvatants, ce qui permet de minimiser, voire de supprimer l'addition de solvants ou de plastifiants volatils.

La présence d'un substituant $R_D$ qui contient un couple rédox tel qu'un disulfure, une thioamide, un ferrocène, une phéno-thiazine, un groupe bis(dialkylaminoaryle), un nitroxyde, un imide aromatique, permet d'induire dans le matériau à conduction ionique des propriétés de navette rédox utiles comme élément de protection et d'égalisation de charge des générateurs électrochimiques, dans les systèmes photoélectrochimiques, en particulier de conversion de la lumière en électricité, dans les systèmes de modulation de la lumière de type électrochrome.

La présence d'un substituant $R_D$ qui est un ligand complexant dans un matériau à conduction ionique permet de chélater les cations métalliques, en particulier ceux qui possèdent un charge élevée (2, 3 et 4), sous forme de complexe soluble dans les milieux organiques, y compris dans les milieux aprotiques, et permet le transport de ces cations en particulier sous forme de complexe anionique, dans les polymères solvatants. Les cations métalliques de charge élevée sont en effet immobiles dans les polymères solvatants. Ce type de complexant donne avec certains cations des métaux de transition (Fe, Co) ou de certaines terres rares (Ce, Eu,) des couples rédox particulièrement stables.

Les matériaux à conduction ionique contenant un composé de l'invention dans lequel $R_D$ est un substituant alkyle ou alkényle qui contient au moins un hétéroatome choisi parmi O, N et S ont un pouvoir complexant, et plastifiant, en particulier dans les polymères polaires et tout spécialement les polyéthers. Les hétéroatomes N et S sont sélectivement complexants pour les cations des métaux de transition, Zn et Pb.

Lorsqu'un substituant $R_D$ alkyle ou alkényle porte en outre un groupe hydroxy, un groupe carbonyle, un groupe amine, un groupe carboxyle, le composé ionique de l'invention peut donner par polycondensation un polymère ou un copolymère et le matériau à conduction ionique qui contient un tel polymère ou copolymère présente des propriétés de polyélectrolyte.

La présence, dans le matériau à conduction ionique de l'invention, d'un composé dans lequel $R_D$ est choisi parmi les radicaux aryle, arylalkyle, alkylaryle ou alkénylaryle, dans lesquels les chaînes latérales et/ou les noyaux aromatiques comprennent des hétéroatomes tels que l'azote, l'oxygène, le soufre, améliore la dissociation et augmente la possibilité de former des complexes suivant la position de l'hétéroatome (pyridine) ou de donner par oxydation duplicative des polymères ou copolymères conjugués (pyrrole, thiophène).

Lorsque le matériau à conduction ionique contient un composé de l'invention dans lequel $R_D$ représente une unité récurrente d'une chaîne polymère, le matériau constitue un polyélectrolyte.

Un composé de l'invention dans lequel le substituant Z est choisi dans le groupe constitué par $-OC_nF_{2n+1}$, $-OC_2F_4H$, $-SC_nF_{2n+1}$ et $-SC_2F_4H$, $-OCF=CF_2$, $-SCF=CF_2$, n étant un nombre entier de 1 à 8, est un précurseur de monomères et polymères stables, en particulier vis-à-vis de l'oxygène même à des températures supérieures à 80°C lorsqu'il s'agit des polymères. Un matériau à conduction ionique qui contient un tel composé est donc particulièrement approprié comme électrolyte d'une pile à combustible.

Le solvant utilisé dans un matériau à conduction ionique de la présente invention peut être un solvant liquide aprotique, un polymère polaire ou un de leurs mélanges.

Le solvant liquide aprotique est choisi par exemple parmi les éthers linéaires et les éthers cycliques, les esters, les nitriles, les dérivés nitrés, les amides, les sulfones, les sulfolanes, les alkylsulfamides et les hydrocarbures partiellement hydrogénés. Les solvants particulièrement préférés sont le diéthyléther, le diméthoxyéthane, le glyme, le tétrahydrofurane, le dioxane, le diméthyltétrahydrofurane, le formiate de méthyle ou d'éthyle, le carbonate de propylène ou d'éthylène, les carbonates d'alkyle (notamment le carbonate de diméthyle, le carbonate de diéthyle et le carbonate de méthylpropyle), les butyrolactones, l'acétonitrile, le benzonitrile, le nitrométhane, le nitrobenzène, la diméthylformamide, la diéthylformamide, la N-méthylpyrrolidone, la diméthylsulfone, la tétraméthylène sulfone, la tétraméthylène sulfone et les tétraalkylsulfonamides ayant de 5 à 10 atomes de carbone.

Un matériau à conduction ionique de la présente invention peut comprendre simultanément un solvant liquide aprotique choisi parmi les solvants liquides aprotiques cités ci-dessus et un solvant polymère polaire comprenant des unités contenant au moins un hétéroatome choisi parmi le soufre, l'azote, l'oxygène et le fluor. Il peut comprendre de 2 à 98% de solvant liquide. A titre d'exemple d'un tel polymère polaire, on peut citer les polymères qui contiennent principalement de unités dérivées de l'acrylonitrile, du fluorure de vinylidène, de la N-vinylpyrrolidone ou du méthacrylate de méthyle. La proportion de liquide aprotique dans le solvant peut varier de 2% (correspondant à un solvant plastifié) à 98% (correspondant à un solvant gélifié).

Un matériau à conduction ionique de la présente invention peut contenir en outre un sel utilisé classiquement dans l'art antérieur pour l'élaboration d'un matériau à conduction ionique. Parmi les sels utilisables en mélange avec un composé ionique selon l'invention, on préfère tout particulièrement un sel choisi parmi les perfluoroalcanesulfonates, les bis(perfluoroalkylsulfonyl) imidures, les bis(perfluoroalkylsulfonyl) méthanes et les tris(perfluoroalkylsulfonyl)-méthanes.

Bien entendu, un matériau à conduction ionique de l'invention peut contenir en outre les additifs utilisés de manière classique dans ce type de matériau, et notamment des charges minérales ou organiques sous forme de poudre ou de fibres.

Un matériau à conduction ionique de l'invention peut être utilisé comme électrolyte dans un générateur électrochimique. La présente invention a ainsi pour autre objet un générateur électrochimique comprenant une électrode négative et une électrode positive séparées par un électrolyte, caractérisé en ce que l'électrolyte est un matériau à conduction ionique tel que défini ci-dessus. Selon un mode de réalisation particulier, un tel générateur comprend une électrode négative constituée par du lithium métallique, ou par l'un de ses alliages, éventuellement sous forme de dispersion manométrique dans de l'oxyde de lithium, ou par un nitrure double de lithium et d'un métal de transition, ou par un oxyde à bas potentiel ayant pour formule générale $Li_{1+y+x/3}Ti_{2-x/3}O_4$ ($0 \leq x \leq 1$, $0 \leq y \leq 1$), ou par le carbone et les produit carbonés issus de la pyrolyse de matières organiques. Selon un autre mode de réalisation, le générateur comprend une électrode positive choisie parmi les oxydes de vanadium $VO_x$ ($2 \leq x \leq 2{,}5$), $LiV_3O_8$, $Li_yNi_{1-x}Co_xO_2$, ($0 \leq x < 1$ ; $0 \leq y \leq 1$), les spinelles de manganèse $Li_yMn_{1-x}M_xO_2$ (M = Cr, Al, V, Ni, $0 \leq x < 0{,}5$ ; $0 \leq y < 2$), les polydisulfures organiques, FeS, $FeS_2$, le sulfate de fer $Fe_2(SO_4)_3$, les phosphates et phosphosilicates de fer et de lithium de structure olivine, ou leurs produits de substitution du fer par le manganèse, utilisés seuls ou en mélanges. Le collecteur de l'électrode positive est de préférence en aluminium.

Un matériau à conduction ionique de la présente invention peut également être utilisé dans une supercapacité. Un autre objet de la présente invention est par conséquent une supercapacité utilisant au moins une électrode de carbone à haute surface spécifique, ou une électrode contenant un polymère rédox, dans laquelle l'électrolyte est un matériau à conduction ionique tel que défini ci-dessus.

Un matériau à conduction ionique de la présente invention peut également être utilisé pour le dopage p ou n d'un polymère à conduction électronique et cette utilisation constitue un autre objet de la présente invention.

En outre, un matériau à conduction ionique de la présente invention peut être utilisé comme électrolyte dans un dispositif électrochrome. Un dispositif électrochrome dans lequel l'électrolyte est un matériau à conduction ionique selon l'invention est un autre objet de la présente invention.

Il a été observé que la forte dissociation des espèces ioniques des composés de l'invention se traduisait par une stabilisation des carbocations, en particulier ceux dans lesquels il existe une conjugaison avec l'oxygène ou l'azote et, d'une manière surprenante, par une forte activité de la forme protonée des composés l'invention sur certains monomères. La présente invention a donc également pour objet l'utilisation des composés ioniques comme photoinitiateurs sources d'acides de Brønsted catalyseurs de polymérisation ou de réticulation de monomères ou de prépolymères capables de réagir par voie cationique, ou comme catalyseurs pour la modification de polymères.

Les inventeurs ont également trouvé que la charge anionique portée par le groupement $-C(CN)_2^-$ exerçait un effet stabilisant sur les conducteurs électroniques de type polymères conjugués, et que l'utilisation d'un composé dans lequel le substituant Z comprenait une longue chaîne alkyle permettait, de rendre ces polymères solubles dans les solvants organiques usuels même à l'état dopé. Le greffage de ces charges sur le polymère lui-même donne des polymères à charge globale cationique, solubles et présentant, outre leur stabilité, des propriétés d'anticorrosion vis-à-vis des métaux, tels que l'aluminium et les métaux ferreux. La présente invention a pour objet des matériaux à conduction électronique comprenant un composé ionique de la présente invention dans lequel la partie cationique est un polycation constitué par un polymère conjugué dopé "p". Les composés ioniques préférés pour cette application sont ceux dans lesquels le substituant Z ou $R_D$ contient une chaîne alkyle ayant de 6 à 20 atomes de carbone.

Les colorants de type cationique (cyanines) sont utilisés de plus en plus fréquemment comme sensibilisateurs de films photographiques, pour le stockage optique d'information (disques optiques accessibles en écriture), pour les lasers. La tendance de ces molécules conjuguées à s'empiler lorsqu'elles sont en phases solides limite leur utilisation, par suite des variations des propriétés optiques par rapport à la molécule isolée. L'utilisation de composés ioniques de l'invention pour la fabrication de colorants cationiques dont les contre ions, éventuellement fixés à cette même molécule, correspondent aux fonctionnalités de l'invention, permet de réduire les phénomènes d'agrégation, y compris dans des matrices solides polymères et de stabiliser ces colorants.

Quelques exemples de composés selon l'invention sont donnés ci-après :

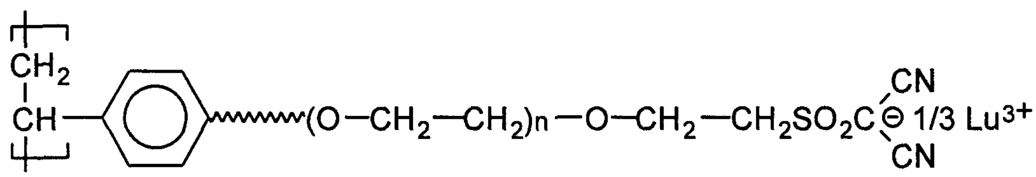

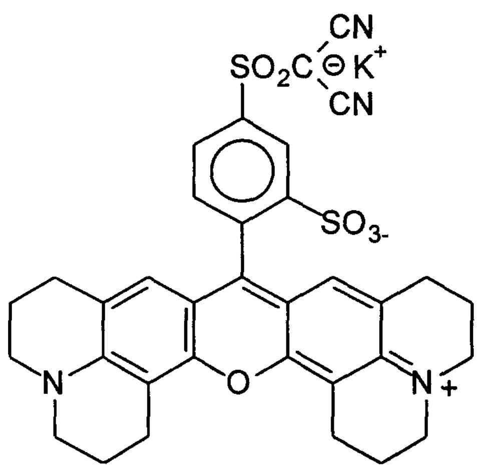

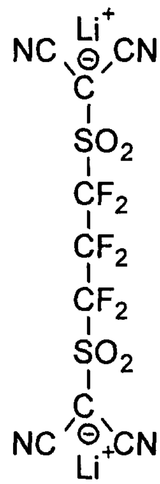

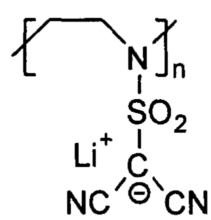

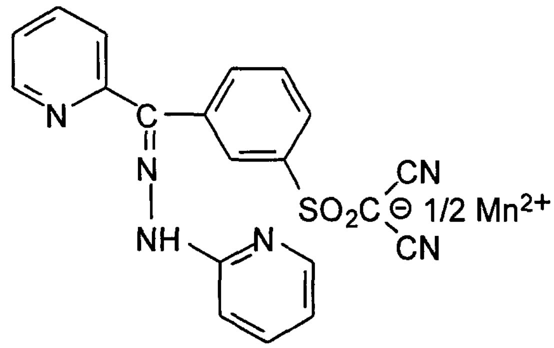

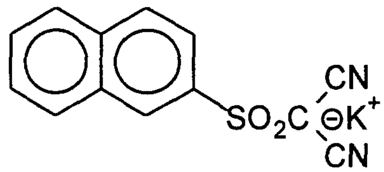

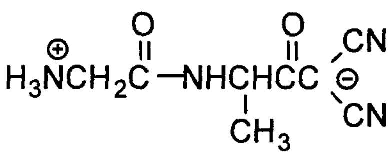

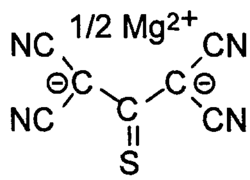

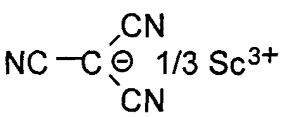

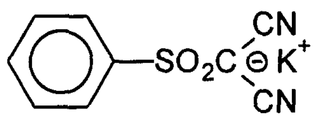

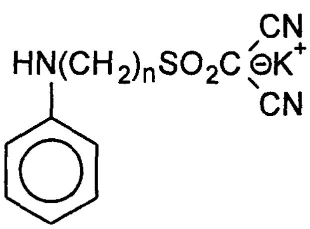

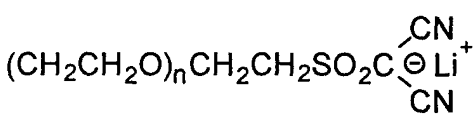

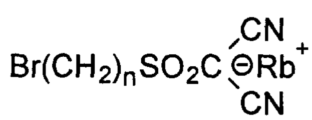

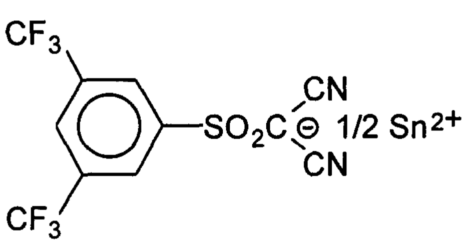

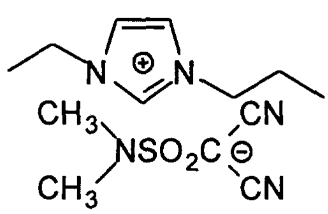

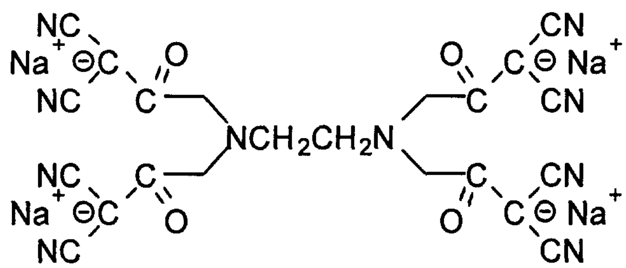

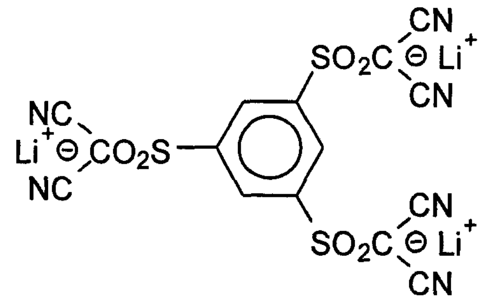

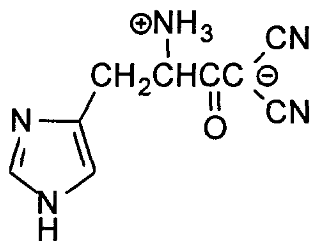

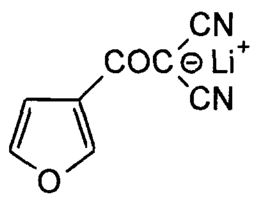

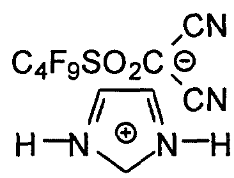

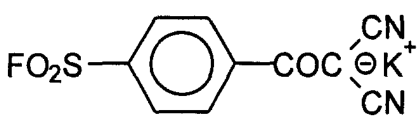

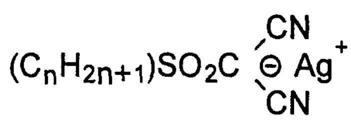

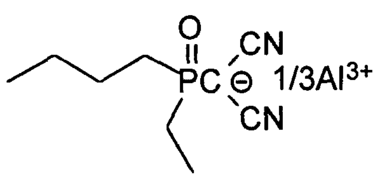

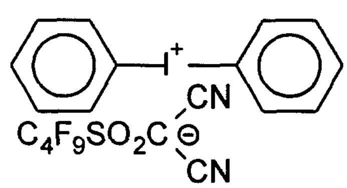

La présente invention est expliquée plus en détails par les exemples suivants, qui décrivent la préparation et diverses utilisations de composés de l'invention. L'invention n'est toutefois pas limitée à ces exemples.

Tous les composés selon l'invention ont été préparés à partir de sels de métal alcalin du malononitrile. Lesdits sels ont été obtenus à partir de malononitrile commercial purifié au préalable dans une cellule de sublimation à 40°C sous vide secondaire, le malononitrile étant récupéré après 48 heures sur le doigt froid de la cellule, sous forme de cristaux blancs qui sont ensuite conservés sous argon.

On a obtenu le sel de lithium en dosant une solution aqueuse de malononitrile par une solution titrée d'hydroxyde de lithium LiOH, le point de neutralisation étant déterminé à l'aide d'un pH-mètre. La solution aqueuse a ensuite été lyophilisée, puis le produit séché sous vide secondaire à l'ambiante pendant 72 heures. On a ainsi obtenu le sel de lithium qui, conservé sous argon, a une pureté déterminée par RMN du proton et du carbone supérieure à 99%.

On a obtenu par le même procédé les sels de sodium et de potassium en remplaçant l'hydroxyde de lithium respectivement par l'hydroxyde de sodium et l'hydroxyde de potassium.

**Exemple 1.**

3,03 g (10 mmoles) de chlorure de l'acide stéarique $C_{17}H_{35}COCl$ dans 25 ml de tétrahydrofurane (THF) et 5 ml de pyridine anhydre ont été ajoutés à 1,04 g (10 mmoles) de sel de potassium du malononitrile dans 25 ml de tétrahydrofurane. Après 24 heures sous agitation, la solution a été filtrée pour éliminer le précipité de chlorure de potassium, puis mise en contact avec 500 mg de carbonate de lithium $Li_2CO_3$. Le mélange a été agité pendant 24 heures, ensuite l'excès de carbonate a été éliminé par centrifugation, puis le solvant a été évaporé. On a obtenu 3,3 g du sel de lithium du stéaroyl de malononitrile, ayant une pureté, caractérisée par RMN du proton et du carbone, supérieure à 97%.

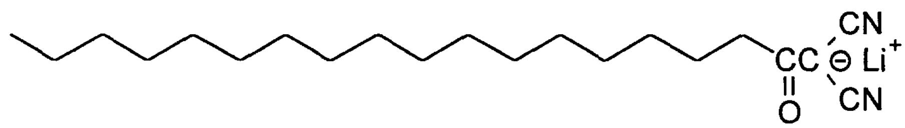

Le radical $R_D$ alkyle supérieur de ce sel lui confère des propriétés tensio-actives marquées, y compris dans les solvants et polymères solvatants aprotiques.

**Exemple 2.**

A 6,61 g (100 mmoles) de malononitrile en solution dans 50 ml de THF à -20°C, on a ajouté par portions 795 mg d'hydrure de lithium LiH. Après deux heures à -20°C, on a ajouté 20,14 g (100 mmoles) de 1-(trifluorométhanesulfonyl)-imidazole (commercialisé par Fluka). La réaction s'est poursuivie pendant 4 heures à -20°C, puis 48 heures à température ambiante. Le solvant a alors été évaporé et le solide résiduel blanc a été lavé par le dichlorométhane pour éliminer l'imidazole formé au cours de la réaction. On a obtenu $LiCF_3SO_2C(CN)_2$.

**Exemple 3.**

Sous atmosphère d'argon, on a ajouté 0,66 g (10 mmoles) de malononitrile et 180 mg d'hydrure de lithium à une solution de 3,02 g (10 mmoles) de fluorure de nonafluorobutanesulfonyle dans 20 ml de THF anhydre, maintenu à 0°C. Après quatre heures, le mélange réactionnel a été filtré, évaporé, repris dans 10 $cm^3$ d'eau, et versé dans une solution saturée de KCl. Le précipité de $KC_4F_9SO_2C(CN)_2$ a été purifié par cristallisation dans l'eau, puis dans un mélange pentanone /dichloroéthane. Le rendement en produit analytiquement pur est de 65%.

De la même manière, on a préparé $KC_6F_{13}SO_2C(CN)_2$ et $KC_{18}F_{17}SO_2C(CN)_2$. Les sels de lithium ont ensuite été obtenus par échange d'ions à l'aide de LiCl ou $LiBF_4$ dans le THF. Ces produits ont des propriétés tensioactives marquées, ils sont solubles dans les polymères solvatants en donnant des complexes conducteurs et ils confèrent des propriétés tensiostatiques.

**Exemple 4.**

324 mg (1 mmole) de chlorure de 4-(diméthylamino)-azobenzène-4'-sulfonyle dans 5 ml de tétrahydrofurane ont été ajoutés à 104 mg (1 mmole) du sel de potassium du malononitrile dans 5 ml de THF et 500 µl de triéthylamine. Après 24 heures sous agitation, le précipité de chlorure de potassium a été éliminé et, après évaporation du solvant, on a obtenu le sel de triéthylammonium qui a été mis en suspension dans 5 ml d'une solution aqueuse contenant 350 mg de bromure de tétrabutylammonium. Le mélange a été agité pendant 24 heures. On a obtenu une poudre de couleur orange qui a une pureté, caractérisée par RMN du proton et du carbone, supérieure à 98%, qui est soluble dans la plupart des solvants organiques, et qui répond à la formule suivante :

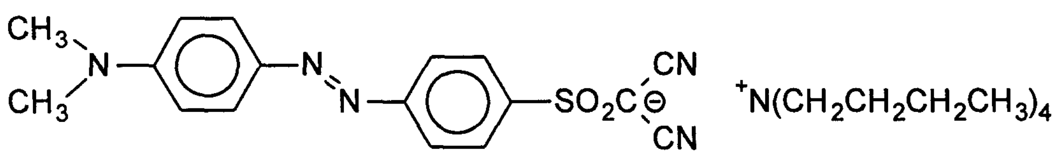

Ce colorant ionique est un indicateur de pH en milieu non aqueux (transition jaune-orange - rouge-violet dans

la zone de pH 1-4).

**Exemple 5.**

10,85 g (100 mmoles) de chlorure de l'acide méthoxyacétique ont été dilués dans 150 ml d'acétonitrile et 15 ml de pyridine anhydre. Le mélange a été maintenu sous atmosphère d'azote et agitation magnétique, et l'on a ajouté par portions 10,41 g (100 mmoles) de sel de potassium du malononitrile. Lorsque la précipitation du chlorure de potassium a été terminée (environ une heure), on a ajouté 25 g de phosphate tripotassique $K_3PO_4$ anhydre et le mélange a été agité pendant 24 heures. Le mélange a ensuite été évaporé à siccité. Le sel de potassium du méthoxyacétylmalononitrile obtenu a été purifié par recristallisation dans le mélange butanone / 1,2-dichloroéthane.

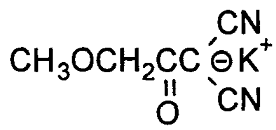

Ce composé est soluble dans la plupart des solvants organiques usuels (tétrahydrofurane, acétonitrile, diméthylformamide, acétate d'éthyle, glymes,) et dans les polymères solvatants aprotiques.
176,2 mg de ce composé ont été dissous dans 5 ml d'acétonitrile séché, auquel on a ajouté 31,7 mg de chlorure de magnésium anhydre. Le mélange a été agité pendant 4 heures et le précipité de chlorure de potassium a été éliminé par centrifugation. La solution surnageante contient le sel de potassium d'un complexe anionique du magnésium $\{Mg[CH_3OCH_2COC(CN)_2]_3\}^-K^+$.
A cette solution surnageante, on a ajouté 800 mg de copolymère statistique oxyde d'éthylène (80%) - méthylglycidyl-éther (20%) de masse $M_w = 2,5.10^5$. Par épandage et évaporation de la solution visqueuse, on a obtenu un film d'électrolyte polymère contenant le magnésium sous forme de complexe $\{Mg[CH_3OCH_2COC(CN)_2]_3\}^-$.
Un générateur primaire comprenant une anode de magnésium a été constitué comme suit :

| anode | Electrolyte | Cathode |
|---|---|---|
| Mg | électrolyte polymère à mécanisme véhiculaire anionique transportant les ions Mg | composite : fluorure de graphite électrolyte noir d'acétylène |

L'électrolyte est le film d'électrolyte polymère contenant le complexe $\{Mg[CH_3OCH_2COC(CN)_2]_3\}^-$ tel que décrit ci-dessus. L'électrode positive a été obtenue de la manière suivante : on a préparé une composition contenant 42% v/v d'un électrolyte identique à celui décrit ci-dessus, 8% v/v de noir d'acétylène et 50% v/v de fluorure de graphite $CF_x$ ($x \leq 1$) ; cette composition a été diluée dans l'acétonitrile, puis épandue sur une feuille de polypropylène de 8 μm d'épaisseur métallisée par 200 nm de cuivre, de manière à former une couche d'environ 80 μm d'épaisseur. L'électrode négative était une feuille de magnésium de 20 μm. La tension de la batterie après assemblage par laminage des éléments à 80°C était de 2,5 V et la capacité pour un débit de 400 $\mu A/cm^2$ était de 7 $mAh/cm^2$.

**Exemple 6.**

Un oligomère sulfoné de poly (oxyde d'éthylène) PEO a été préparé de la manière suivante : 10 g de POE de masse 600 ont été séchés par distillation azéotropique avec du benzène et lyophilisation. Après addition de 50 ml de THF, les groupements OH terminaux ont été métallés par le potassiumnaphtalène. La stoechiométrie a été déterminée par colorimétrie, la fin de la réaction étant indiquée par la persistance de la couleur verte intense du radical anion du naphtalène. On a alors ajouté 4,10 g de propanesultone. Après évaporation du solvant, on a obtenu le polymère $\alpha,\omega$-disulfoné sous forme de poudre, et le naphtalène résiduel a été éliminé par lavage à l'hexane.
5 g du polymère $\alpha,\omega$-disulfoné ainsi formé, mis en suspension dans 15 ml d'acétonitrile, ont été traités par 1,8 g de chlorure de (chlorométhylène)diméthylammonium $[(CH_3)_2N=CHCl]^+,Cl^-$. Un précipité de chlorure de potassium s'est formé après environ 1 heure. A cette suspension ont été ajoutés 1,26 g du sel de potassium du malononitrile et 3 ml de pyridine anhydre. Après filtration, le mélange réactionnel a été agité en présence de 2 g de phosphate de lithium $Li_3PO_4$. Une nouvelle filtration a permis de séparer une solution incolore qui, par concentration, a donné un fluide visqueux.

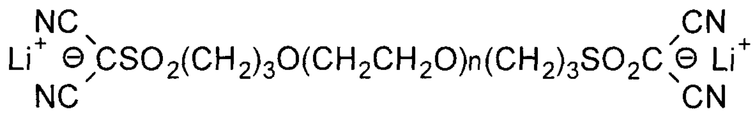

Ce matériau permet de plastifier un grand nombre de polymères contenant des unités polaires (éther, amide, nitrile, ester), tout en leur conférant une conductivité ionique élevée.

**Exemple 7.**

Un tensio-actif non-ionique, le polyoxyéthylène-23 lauryl éther $C_{12}H_{25}(OCH_2CH_2)_{23}OH$ (Brij® 35), a été sulfoné par une procédure similaire à celle de l'exemple 4. 12 g de Brij® 35 ont été séchés par distillation azéotropique avec du benzène et lyophilisation. Après addition de 50 ml de THF, les groupements OH terminaux ont été métallés par l'hydrure de sodium en présence de 5 mg de triphénylméthane. La stoechiométrie a été déterminée par colorimétrie, la fin de la réaction étant indiquée par la persistance de la couleur rouge intense de l'anion $\Phi_3C^-$. On a alors ajouté 1,4 g de 1,4-butanesultone. Après évaporation du solvant, l'oligomère sulfoné a été obtenu sous forme de poudre.

5 g de l'oligomère sulfoné ainsi formé en suspension dans 15 ml d'acétonitrile ont été traités par 1 ml de chlorure de thionyle et 20 µl de diméthylformamide. Un précipité de chlorure de sodium s'est formé en 20 min. Après filtration, le solvant et l'excès de $SOCl_2$ ont été évaporés sous pression réduite. Le résidu a été dissous dans 30 ml de pyridine, puis on a ajouté 350 mg du sel de sodium du malononitrile. Après filtration, le mélange réactionnel a été agité en présence de 1 g de phosphate le lithium $Li_3PO_4$. Une nouvelle filtration a permis de séparer une solution incolore qui, par concentration, a donné une cire.

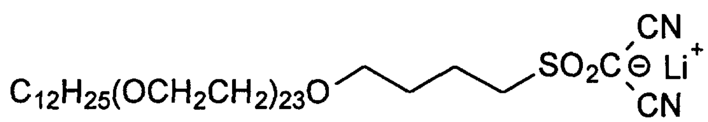

Ce matériau possède des propriétés tensioactives et plastifiantes.

**Exemple 8.**

380 mg (1 mmole) de l'acide éthylènebis(oxyéthylènenitrilo)tétraacétique dans 10 ml de pyridine ont été traités par 912 mg de carbonate d'hydroxysuccinimidyle pendant 24 heures. On a ajouté 416 mg (4 mmoles) du sel de potassium du malononitrile dans 15 ml d'un mélange équivolumique de pyridine et d'acétonitrile. Après 24 heures, le précipité a été séparé par filtration et lavé avec 2 portions de 30 ml de THF. On a obtenu des cristaux de :

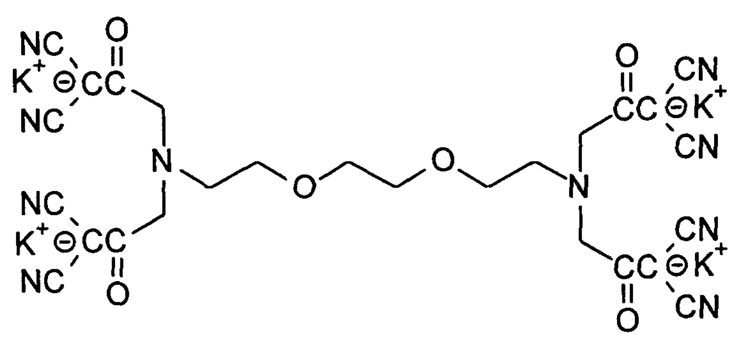

Ce composé est un ligand (L) des métaux divalents ($A^{II}$) et trivalents ($A^{III}$), en particulier des terres rares. Les complexes correspondants $[A^{II}L]^{2-}M^+$ et $[A^{III}L]^-M^+$ sont des sels solubles dans les milieux aprotiques polaires et dans les polymères polaires, en particulier les polyéthers. Ces complexes dans lesquels le métal central A est protégé des influences électrostatiques extérieures sont intéressants pour la constitution de lasers. Il permettent aussi des réactions rédox par changement du degré d'oxydation du métal A.

**Exemple 9.**

On a préparé une solution de 548 mg (2 mmoles) d'acide 1,1'-ferrocène-dicarboxylique et 824 mg (4 mmoles) de dicyclohexylcarbodiimide dans 5 ml d'un mélange équivolumique de pyridine et de méthanol anhydres. Le mélange a été maintenu sous agitation magnétique à température ambiante pendant 75 heures. On a ensuite ajouté 288 mg (4 mmoles) du sel de lithium du malononitrile. Le mélange a été maintenu sous agitation à température ambiante pendant 24 heures. Le précipité de dicyclohexylurée a été éliminé par centrifugation et la solution a été évaporée. On a obtenu le 1,1'-ferrocène-diacétylmalononitrile sous la forme d'un solide brun foncé hygroscopique, ayant une pureté, caractérisée par RMN du proton et du carbone, supérieure à 98%. Il est soluble dans la plupart des solvants organiques usuels (tétrahydrofurane, acétonitrile, diméthylformamide, acétate d'éthyle, glymes,) et dans les polymères solvatants aprotiques.

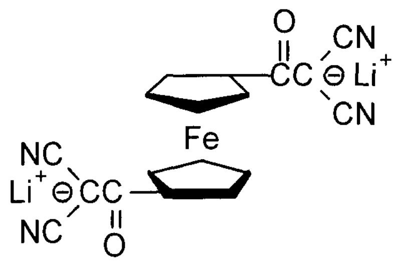

Ce sel présente un couple rédox réversible. Dans le polyoxyde d'éthylène, on a pu déterminer, sur une électrode de platine d'un diamètre de 125 µm, un potentiel réversible ≈ 3,8 V vis à vis d'une électrode de lithium.

Par dissolution dans un électrolyte liquide, gel ou polymère, il permet d'effectuer une protection en surcharge dans les batteries au lithium, jouant ainsi un rôle de navette rédox. Il permet aussi de réaliser des systèmes électrochromes à colorants.

**EXEMPLE 10.**

501 mg (2 mmoles) d'acide 6-hydroxy-2,5,7,8-tétraméthylchroman-2-carboxylique(Trolox) :

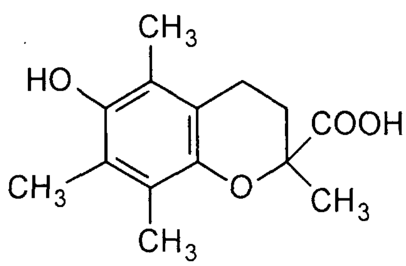

ont été mis en suspension dans 10 ml d'un mélange équivolumique de pyridine et de méthanol anhydres avec 313 µl (2 mmoles) de 1,3 diisopropylcarbodiimide. Après 24 heures, le précipité de diisopropylurée a été filtré et on a ajouté 208 mg (2 mmoles)du sel de potassium du malononitrile. Le mélange a été maintenu sous agitation en atmosphère neutre (azote) pendant 24 heures. Le volume de la solution a été réduit à 2 ml à l'aide d'un évaporateur rotatif. On a ajouté 20 ml de dioxane et le mélange a été refroidi à -10°C. Un précipité blanc a été collecté par filtration. L'analyse correspond à $C_{17}H_{17}N_2O_3K$.

Ce produit possède des propriétés anti-oxidantes, en particulier pour les polymères. Il en est de même pour les dérivés d'autres cations, y compris de cations organiques tels que les cations tétraalkylammonium.

**Exemple 11.**

2,8 g (10 mmoles) d'acide 4,4'-azobis(4-cyanovalérique), 3,24 g (20 mmoles) de carbonyldiimidazole et 100 mg de diméthylamino pyridine ont été mis en suspension dans 20 ml d'éther et maintenus à 0°C. Après cessation du dégagement de $CO_2$ (5 heures), on a ajouté 1,44 g (20 mmoles) du sel de lithium du malononitrile. Le mélange a été

maintenu sous agitation magnétique à 0°C pendant 1 heure. Par centrifugation, on a isolé le précipité cristallin de :

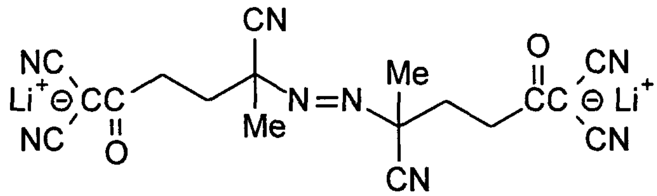

Ce sel est soluble en particulier dans l'acétone, l'acétonitrile, l'acétate d'éthyle, le tétrahydrofurane. Il peut servir d'initiateur radicalaire pour amorcer des réactions de polymérisation ou de réticulation dès 60°C.

**Exemple 12.**

479 mg (1 mmole) de Rhodamine B ont été mis en suspension dans 10 ml de pyridine et on a ajouté 104 mg (1 mmole) de sel de potassium du malononitrile et 206 mg (1 mmole) de dicyclohexylcarbodiimide. Après 48 h sous agitation, le mélange a été filtré pour éliminer la dicyclohexylurée et soumis à évaporation. Le composé obtenu est un zwitterion qui possède des propriétés colorantes intenses. Il est soluble dans les polymères polaires et permet la constitution de lasers à colorants. Le groupement acétylmalonitrile lui permet également de s'adsorber sur des oxydes, en particulier le dioxyde de titane nano-particulaire, il joue alors un rôle de sensibilisateur au rayonnement visible, en particulier dans les applications aux cellules photovoltaïques.

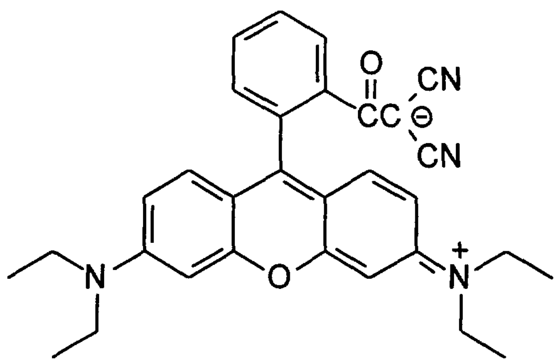

**EXEMPLE 13.**

L'acide pyrryl-3 acétique (M = 122) a été préparé selon la méthode de D. Delabouglise (Thèse Université de Paris-Nord, février 1991, "Contrôle Moléculaire des propriétés des polymères"). 488 mg de ce composé ont été dissous dans un mélange de 5 ml d'acétonitrile et 1 ml de pyridine, auquel ont été ajoutés 648 mg (40 mmoles) de carbonyldiimidazole. Après 24 heures et cessation du dégagement de $CO_2$, on a ajouté 417 mg (40 mmoles) du sel de potassium du malononitrile. Le mélange a été agité pendant 48 heures à température ordinaire. Le solvant a été évaporé et le sel de potassium a été purifié par recristallisation dans le mélange butanone - 1,2 dichloroéthane.

On a préparé 10 ml d'une solution $5.10^{-2}$ M dans l'acétonitrile du sel obtenu et l'on a effectué une électropolymérisation dans le compartiment anodique d'une cellule électrochimique sur électrode de platine. On a obtenu un film souple conducteur dont le dopage (oxydation) est assuré par échange de cations et d'électrons avec l'extérieur. La conductivité de ce matériau est de l'ordre de 10 $S.cm^{-1}$ et elle est stable à l'atmosphère ambiante. L'électropolymérisation effectuée en présence de pyrrole non substitué ou possédant des chaînons oxyéthylène en position N ou 3 donne des copolymères également stables dont le changement de couleur peut être utilisé pour la constitution de systèmes électrochromes†:

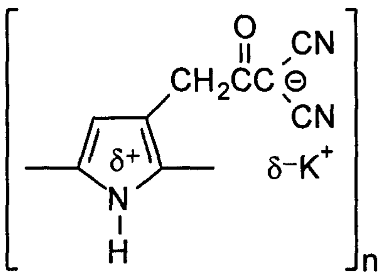

**Exemple 14.**

En opérant dans une boîte à gants sous argon, à 24,15 g (100 mmoles) de di-2-éthylhexylamine dans 100 ml de THF à -20°C, on a ajouté par portions 32 ml de butyllithium 2M dans le cyclohexane (100 mmoles). Après une heure, on a ajouté 11,85 g (100 mmoles) de fluorure de chlorosulfonyle $FSO_2Cl$. La réaction s'est poursuivie 4 heures à -20°C, puis pendant 24 heures à température ambiante. On a alors porté la température à 0°C et on ajouté 10,42 g (100 mmoles) du sel de potassium du malononitrile $KHC(CN)_2$ et 11,22 g (100 mmoles) de DABCO. Après 24 heures à 0°C, le milieu réactionnel a été filtré pour éliminer le précipité de chlorure de potassium et l'hydrochlorure de DABCO. Après évaporation du solvant et séchage, on a récupéré le sel de lithium du di-2-éthylhexylaminosulfonylmalononitrile, ayant une pureté, caractérisée par RMN du proton et du carbone, supérieure à 98%.

Suivant le même procédé, on a obtenu le sel de lithium du dibutylaminosulfonylmalononitrile à partir de la dibutylamine.

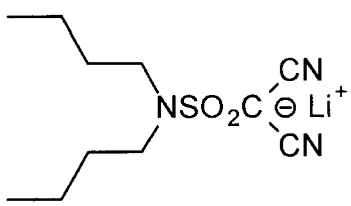

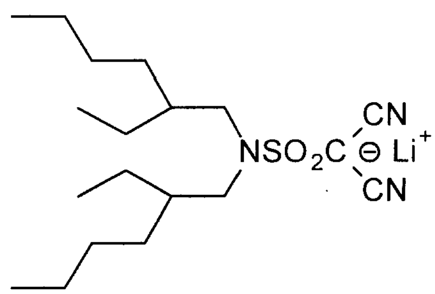

Les sels de potassium ont été obtenus en traitant les sels de lithium dans le minimum d'eau par le fluorure de potassium KF. Après filtration, évaporation et séchage, on a récupéré quantitativement les sels de potassium.

Ces sels sont solubles dans la plupart des solvants organiques usuels (tétrahydrofurane, acétonitrile, diméthylformamide, acétate d'éthyle, glymes,) et dans les polymères solvatants aprotiques.

**Exemple 15.**

5,44 g (10 mmoles) de iodure de 3,3'-diéthylthiatricarbocyanine (commercialisé par Aldrich, Milwaukee, USA) et 4,08 g (10 mmoles) du sel de potassium du di-2-éthylhexylaminosulfonylmalononitrile préparé selon l'exemple 14 ont été agités ensemble durant 24 heures dans l'eau. Par extraction de la phase aqueuse par du dichlorométhane, on a récupéré la 3,3'-diéthylthiatricarbocyanine du di-2-éthylhexylaminosulfonylmalononitrile, ayant une pureté, caractérisée par RMN du proton supérieure, à 99%.

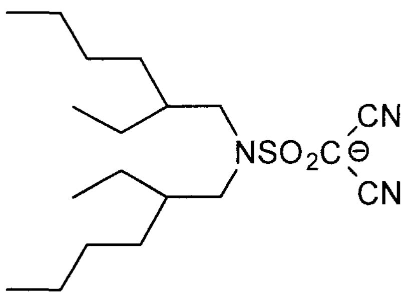

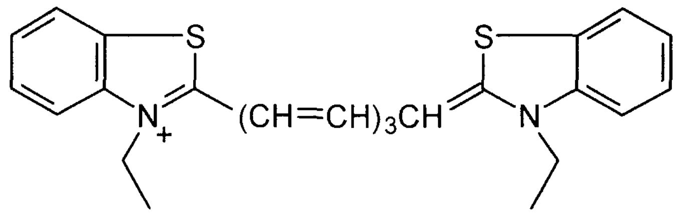

Ce sel est très soluble dans les solvants peu polaires comme le dichlorométhane ou le chlorure de méthylène ainsi que dans les matrices polymères peu polaires comme le polyméthacrylate de méthyle.

Lorsqu'il est utilisé comme colorant, on constate une très nette diminution de l'agrégation des molécules de colorant cationique entre elles, du fait du caractère "plastifiant" des différents groupements dialkylamino, ce qui constitue un avantage. En effet, le phénomène d'agrégation est préjudiciable au bon fonctionnement des systèmes utilisant des colorants, en particulier dans les disques optique de stockage d'informations, car il provoque un élargissement des bandes d'absorption optiques.

**Exemple 16.**

3,17 g (10 mmoles) de chlorure de diphényliodonium $(C_6H_5)_2ICl$ et 4,08 g (10 mmoles) de sel de potassium di-2-éthylhexylaminosulfonylmalononitrile préparé selon l'exemple 14 ont été agités ensemble durant 24 heures dans l'eau. Par extraction de la phase aqueuse par du dichlorométhane, on a récupéré le diphényliodonium du di-2-éthylhexylaminosulfonylmalononitrile, ayant une pureté, caractérisée par RMN du proton, supérieure à 99%.

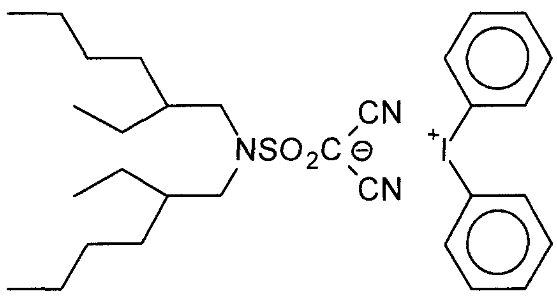

Ce sel permet d'amorcer, sous l'effet d'un rayonnement actinique (lumière, rayons $\gamma$, faisceaux d'électrons), la réaction de réticulation cationique de monomères riches en électrons (éthers vinyliques, propylvinyléthers,).

Il est soluble dans la plupart des solvants organiques usuels (tétrahydrofurane, acétonitrile, diméthylformamide, acétate d'éthyle, glymes,) et dans les polymères solvatants aprotiques comme le polyoxyde d'éthylène. Il est aussi soluble à plus de 5% en poids dans les solvants réactifs comme le triéthylèneglycol divinyl éther, contrairement par exemple au sel de bis(trifluorométhanesulfonyl)imide du diphényliodonium.

On a testé les propriétés de photo-amorçage de ce sel en irradiant par un rayonnement U.V. à 254 nm, d'une puissance de 1900 $mW/cm^2$, un feutre non tissé de polyéthylène imbibé de triéthylèneglycol divinyl éther (79% en poids) contenant le diphényliodonium du di-2-éthylhexylaminosulfonylmalononitrile (1% en poids) du présent exemple et le 7,8-octène-3,6-oxa-1-sulfonylmalononitrile (20% en poids), obtenu à l'exemple 21. Après une période de quelques secondes sous irradiation, suivie d'une période de 10 min permettant la propagation des espèces générées dans le milieu (postcure), on a obtenu le polyélectrolyte supporté par le feutre. Ce type de composite est très intéressant pour le développement de batteries au lithium à électrolyte polymère ou gel.

**Exemple 17.**

5,91 g (20 mmoles) du sel de potassium du dibutylaminosulfonylmalononitrile, préparés selon l'exemple 14, ont été mis en solution dans 10 ml d'eau. On a ajouté, sous agitation, 2,71 g (10 mmoles) d'hydrochlorure de 2,2'-azobis(2-méthylpropionamidine) $[=NC(CH_3)_2C(=NH)NH_2]_2 \cdot 2\ HCl$ (commercialisé par Aldrich) en solution dans 10 ml

d'eau. Il s'est formé immédiatement un précipité qui a été recueilli par filtration. Après séchage sous vide à température ambiante, on a récupéré le dibutylaminosulfonylmalononitrile de 2,2'-azobis(2-méthylpropionamidine).

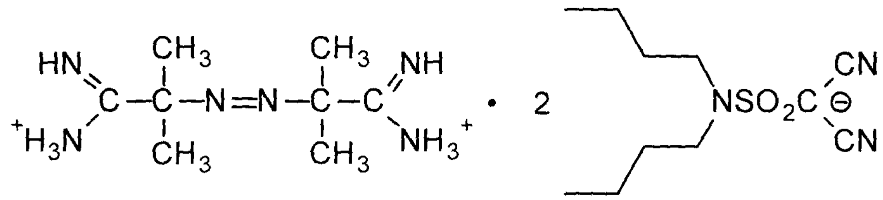

Ce sel est un amorceur de polymérisation radicalaire soluble dans la plupart des solvants organiques usuels (tétrahydrofurane, acétonitrile, diméthylformamide, acétate d'éthyle, glymes,) et dans les monomères et polymères solvatants aprotiques, contrairement à l'hydrochlorure de 2,2'-azobis(2-méthylpropionamidine).

On a préparé une solution dans l'acétonitrile de 1 partie de cet initiateur et de 100 parties d'un polymère contenant des insaturations éthyléniques, obtenu par polycondensation de polyéthylène glycol de masse 1000 avec le 3-chloro-2-chlorométhyl-1-propène selon la procédure décrite par Alloin, et al (*Solid States Ionics*, (1993), 60, 3). On a coulé la solution visqueuse obtenue sur un film de polypropylène (PP). Après évaporation du solvant, le film de polymère d'une épaisseur de 110 μm sur PP a été stocké une semaine en boîte à gants sous argon pour le sécher. La réticulation a alors été initiée en portant la température du film à 60°C. Après une nuit, on a obtenu un film présentant de bonnes propriétés mécaniques et un faible taux d'extractibles (inférieure à 1%). La solubilité de l'initiateur utilisé dans la matrice polymère permet donc d'obtenir une réticulation efficace et homogène. De plus, cet initiateur, contrairement par exemple au 2,2'-azobisisobutyronitrile, n'est pas volatil et la quantité ajoutée peut être optimisée au mieux pour chaque type de polymérisation.

**Exemple 18.**

A 4,08 g (10 mmoles) du sel de potassium du di-2-éthylhexylaminosulfonylmalononitrile, obtenu à l'exemple 14, en solution dans 10 ml de nitrométhane anhydre, on a ajouté en boîte à gants 1,17 g (10 mmoles) de tétra-fluoroborate de nitrosonium $NOBF_4$ (commercialisé par Aldrich). Après une heure, le milieu réactionnel a été filtré pour éliminer le tétrafluoroborate de potassium insoluble, et on a obtenu une solution 1 M du sel de nitrosonium du di-2-éthylhexylaminosulfonylmalononitrile dans le nitrométhane.

Par un procédé similaire, on a préparé une solution 1 M dans le nitrométhane du sel de nitrosonium du dibutylaminosulfonylmalononitrile, à partir du sel de potassium du dibutylaminosulfonylmalononitrile. Ces sels sont particulièrement intéressants pour le dopage de polymères conjugués (polythiophène, polypyrrole, polyaniline,) auxquels ils confèrent une conductivité électronique appréciable.

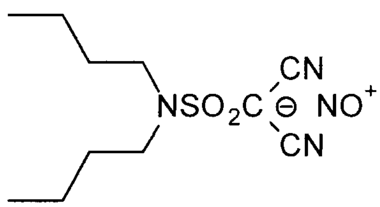
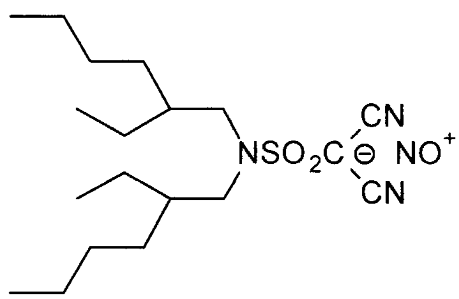

On a réalisé deux dépôts de poly(3-hexylthiophène) stéréorégulier (commercialisé par Aldrich, Milwaukee, USA)) sur des plaques de verre à partir d'une solution dans le chloroforme. Après séchage, ces dépôts ont été dopés respectivement par l'un et l'autre des sels ci-dessus en solution dans le nitrométhane. Après dopage, chacun des films de poly (3-hexylthiophène) ainsi obtenus présentait une conductivité électronique supérieure à 1 $S.cm^{-1}$ et une bonne stabilité en milieu humide. Ces dépôts sont intéressants pour la réalisation de masques pour l'industrie des semi-conducteurs.

**Exemple 19.**

Dans 100 ml de tétrahydrofurane anhydre sous argon à 0°C, on a fait réagir 20,27 g (100 mmoles) de chlorure de 4-styrènesulfonyle $CH_2=CHC_6H_4SO_2Cl$ (commercialisé par Monomer-Polymer & Dajac Laboratories), 10,42 g (100 mmoles) de malononitrile et 22,44 g (200 mmoles) de DABCO. Après 2 heures à 0°C et 48 heures à l'ambiante, la solution a été filtrée pour éliminer l'hydrochlorure de DABCO formé, puis traitée par 4,24 g de chlorure de lithium anhydre (100 mmoles), stocké et pesé en boîte à gants. Il s'est formé immédiatement un précipité d'hydrochlorure de DABCO et le milieu réactionnel a alors été de nouveau filtré après une agitation de 6 heures. Après évaporation et séchage sous vide pendant 24 heures à l'ambiante, on a récupéré 31,16 g du sel de lithium du 4-styrènesulfonylmalononitrile, ayant une pureté, caractérisée par RMN du proton et du carbone supérieure, à 97%.

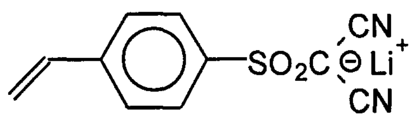

Ce sel peut être homo- ou copolymérisé par une polymérisation initiée par voie anionique, cationique ou radicalaire. On peut également le greffer par irradiation sur une matrice polymère telle que le polyfluorure de vinylidène.

L'homopolymère, obtenu par polymérisation radicalaire dans l'eau désaérée initiée par l'acide cyanovalérique à 60°C, est soluble dans les solvants organiques usuels et dans les polymères solvatants aprotiques. Dans le polyoxyde d'éthylène à une concentration O/Li de 16/1, ce sel présente une conductivité ≈ $5{,}2.10^{-4}$ $S.cm^{-1}$ à 100°C.

De plus, en solution concentrée dans l'acétone (≈ 1 M en cation lithium), cet homopolymère du sel de lithium du 4-styrènesulfonylmalononitrile peut être utilisé comme catalyseur pour des réactions de Diels-Alder.

**Exemple 20.**

Suivant un procédé similaire à celui décrit dans l'exemple 19, on a obtenu le sel de lithium du vinyisulfonylmalononitrile, à partir de 11,01 g (100 mmoles) de fluorure d'éthylènesulfonyle (commercialisé par Fluka, Buchs, Suisse), ayant une pureté, caractérisée par RMN du proton et du carbone, supérieure à 98%.

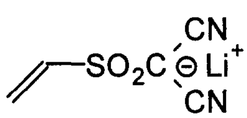

Ce sel peut être homo- ou copolymérisé par une polymérisation initiée par voie radicalaire.

Ainsi, 6,6 g d'un copolymère d'oxyde d'éthylène contenant des doubles liaisons allyliques et ayant une masse $M_W = 2{,}5.10^5$ ont été mis en solution dans l'acétonitrile. On a ajouté 1,52 g du sel lithium du vinylsulfonylmalonitrile et 50 mg de l'initiateur radicalaire préparé selon l'exemple 17. La solution a été évaporée dans une coupelle de PTFE à fond plat, et le récipient a été mis dans un four sous vide primaire à 80°C pendant 12 heures. On a obtenu un élastomère réticulé sur lequel sont fixés des groupements $-SO_2C(CN)_2Li$. Ce matériau, qui constitue un électrolyte à anions fixés, possède une conduction par les ions lithium de $6{,}7.10^{-4}$ $S.cm^{-1}$ à 60°C, et un nombre de transport cationique de 0,92.

**Exemple 21.**

A 2,2 g (25 mmoles) de l'éther vinylique de l'éthylène glycol $CH_2=CHO(CH_2)_2OH$ dans 60 ml de diméthylformamide anhydre, on a ajouté 4,05 g du sel de lithium du vinylsulfonylmalononitrile, obtenu à l'exemple 20, 5,87 g (42,5 mmoles) de carbonate de potassium $K_2CO_3$ anhydre et 330 mg (1,25 mmoles) de 18-Crown-6 (agissant comme complexant du cation $K^+$). Le milieu réactionnel a alors été agité sous argon à 85°C. Après 48 heures, le milieu réactionnel a été filtré sur un verre fritté de porosité N°3, puis on a éliminé le solvant par évaporation sous pression réduite. Après séchage, le composé résiduel a été recristallisé dans 10 ml d'eau contenant 1,86 g (25 mmoles) de chlorure de potassium KCl anhydre. Après filtration et séchage, on a récupéré le sel de potassium du 7,8-octène-3,6-oxa-1-sulfonylmalononitrile, ayant une pureté, caractérisée par RMN du proton et du carbone, supérieure à 98%.

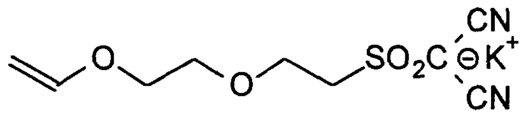

On a obtenu quantitativement le sel de lithium par traitement du sel de potassium dans le tétrahydrofurane anhydre par la quantité stoechiométrique de chlorure de lithium anhydre, filtration du milieu réactionnel, évaporation du solvant et séchage sous vide.

Ce sel peut être homo- ou copolymérisé par une polymérisation initiée par voie cationique, par polymérisation alternée avec un monomère insaturé initiée par voie radicalaire. L'homopolymère préparé par une polymérisation dans l'acétonitrile anhydre initiée par voie cationique par la bis(trifluorométhanesulfonyl)imide présente une conductivité à une concentration de 0,8 M dans un mélange de diméthylcarbonate et de carbonate d'éthylène (2:1) de $6.10^{-3}$ $S.cm^{-1}$ à 30°C. De plus, cet homopolymère est soluble dans la plupart des solvants organiques usuels (tétrahydrofurane, acétonitrile, diméthylformamide, acétate d'éthyle, glymes,...) et dans les polymères solvatants aprotiques comme le polyoxyde d'éthylène. Cet homopolymère constitue par conséquent un bon matériau à conduction ionique.

**Exemple 22.**

A une solution dans 10 ml de tétrahydrofurane anhydre à 0°C de 4,49 g (40 mmoles) de DABCO et de 1,32 g (20 mmoles) de malononitrile, on a ajouté 6,05 g (20 mmoles) de chlorure de 4-iodobenzènesulfonyle $IC_6H_4SO_2Cl$ (commercialisé par Aldrich) dilués dans 5 ml de tétrahydrofurane anhydre. Après deux heures à 0°C, la réaction s'est poursuivie durant 24 heures à l'ambiante. L'hydrochlorure de DABCO formé au cours de la réaction a été éliminé par filtration sur un verre fritté de porosité N°4. Après évaporation de l'acétonitrile de la solution filtrée, le produit a été repris dans 15 ml d'eau froide et l'on a ajouté lentement 1,49 g (20 mmoles) de chlorure de potassium anhydre en solution dans 5 ml d'eau. Il est apparu un précipité qui a été recueilli par filtration sur un verre fritté de porosité N°4. Après séchage, on a récupéré le sel de potassium du 4-iodobenzènesulfonylmalononitrile.

Ce composé a été oxydé en iodosoacétate $K[(NC)_2CSO_2C_6H_4I(O_2CCH_3)_2]$ par le mélange acide acétique, anhydride acétique et peroxyde d'hydrogène selon la méthode de Yamada & al *(Die Makromolecular Chemie,* (1972), 152, 153-162). 4,88 g (10 mmoles) dudit iodosoacétate ont été mis en suspension dans un mélange de 15 ml d'acide méthanesulfonique et de 4,51 g (30 mmoles) de butoxybenzène maintenu à 0°C pendant 4 heures. Le produit de réaction a été versé dans 300 ml d'éther et le précipité séparé par filtration, lavé par de l'éther et séché. On a ainsi obtenu 3,9 g du zwitterion (4-butoxybenzène)-(4-phénylsulphonylmalononitrile] iodonium (75% de rendement) ayant une pureté, caractérisée par RMN du proton et du carbone, supérieure à 97%.

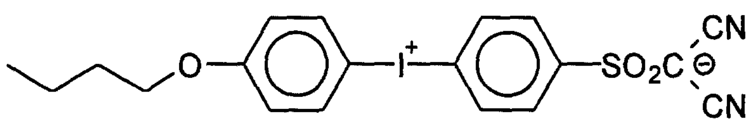

Ce zwitterion permet d'amorcer sous l'effet d'un rayonnement actinique (lumière, rayons γ, faisceaux d'électrons) la réaction de réticulation cationique de monomères riches en électrons (éthers vinyliques, propènyléthers vinyliques,...).

Il a une bonne solubilité dans la plupart des solvants organiques usuels (tétrahydrofurane, acétonitrile, diméthylformamide, acétate d'éthyle, glymes,...) et dans les monomères et les polymères solvatants aprotiques comme le polyoxyde d'éthylène. Il est aussi soluble à plus de 5% en poids dans les solvants réactifs comme le triéthylèneglycol divinyl éther.

On a testé les propriétés de photo-amorçage de ce sel en irradiant par un rayonnement U.V. à 254 nm, d'une puissance de 1900 $mW/cm^2$, une solution de triéthylèneglycol divinyl éther contenant à 1% en poids dudit sel d'iodonium. Après quelques secondes sous irradiation, le solvant réactif a pris en masse, cette réaction étant très exothermique.

**Exemple 23.**

Une solution de 2,22 g (5 mmoles) de tétra-fluoroborate de tétrakis(acétonitrile)palladium(II) $(CH_3CN)_4Pd(BF_4)_2$ (commercialisé par Aldrich), dans 30 ml de tétrahydrofurane, a été traitée par 4,08 g (10 mmoles) de sel de potassium du di-2-éthylhexylaminosulfonylmalononitrile. Après 24 heures sous agitation, le milieu réactionnel a été

filtré pour éliminer le précipité de tétrafluoroborate de potassium $KBF_4$, puis le solvant évaporé. On a obtenu quantitativement le di-2-éthylhexylaminosulfonylmalononitrile de tétrakis-(acétonitrile)palladium(II).

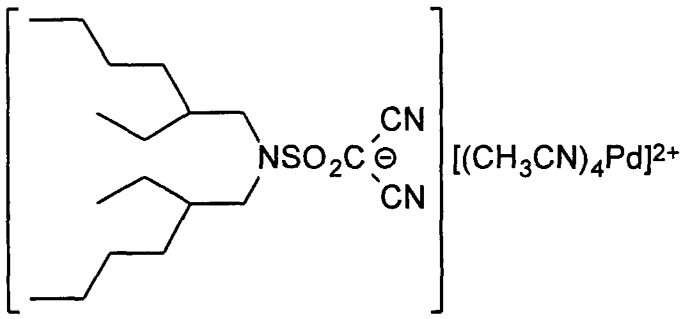

Ce sel est utile comme catalyseur de polymérisation vinylique du norbornène. Ainsi on a réalisé la polymérisation à température ambiante du norbornène dans le nitrométhane en présence de 300 ppm de ce sel. Après 2 heures, on a reprécipité le milieu réactionnel dans le méthanol. On a obtenu un polynorbornène ayant une masse moyenne en nombre de 420000, avec un rendement de 82%.

**Exemple 24.**

En opérant dans une boîte à gants sous argon, on a introduit dans un flacon en polypropylène (Nalgène®) de 30 ml, 1,8 g de phénazine (10 mmoles) et 139 mg de lithium métallique. On a alors ajouté 20 ml de tétrahydrofurane anhydre et des billes en agate. Le flacon fermé a ensuite été mis en rotation sur lui-même, à l'extérieur de la boîte à gants, sur l'axe d'un moteur. Le tétrahydrofurane a rapidement pris une couleur mauve foncé caractéristique de la phénazine mono-lithiée. Après 24 heures, on a obtenu une suspension d'un précipité orange de 9,10-di-Li-dihydrophénazine. A 8,61 g (20 mmoles) de ce composé, on a ensuite ajouté 4,89 g (40 mmoles) de 1,3-propane sultone. Après 8 heures de broyage à température ambiante, le milieu réactionnel a été filtré pour éliminer les billes en agate et on a ajouté sous argon, deux gouttes de diméthylformamide à la solution filtrée, puis lentement 5,08 g (40 mmoles) de chlorure d'oxalyle ClCOCOCl en solution dans 15 ml de dichlorométhane anhydre. Après 4 heures à température ambiante, on a ajouté 4,65 g (40 mmoles) de sel de potassium du malononitrile. La réaction s'est poursuivie pendant 24 heures, puis le milieu réactionnel a été filtré pour éliminer le précipité de chlorure de potassium. Après évaporation du solvant, on a récupéré le di-sel de lithium de la 9-10-(propylsulfonylmalononitrile)phénazine, ayant une pureté, caractérisée par RMN du proton et du carbone, supérieure à 98%.

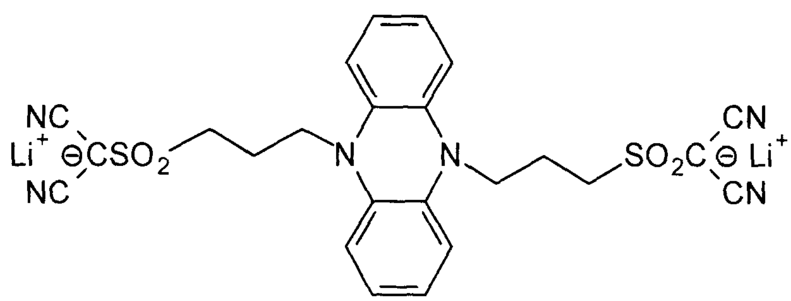

Ce composé est soluble dans la plupart des solvants organiques usuels (tétrahydrofurane, acétonitrile, diméthylformamide, acétate d'éthyle, glymes,) et dans les polymères polaires.
Ce composé présente deux couples rédox réversibles. Dans le polyoxyde d'éthylène, on a pu mettre en évidence, sur une électrode de platine d'un diamètre de 125 µm, un premier couple rédox d'un potentiel ≈ 3,2 V et un second couple rédox d'un potentiel ≈3,8 V, ces potentiels étant mesurés vis à vis d'une électrode de lithium.
Lorsqu'il est dissout dans un électrolyte liquide, gel ou polymère, ce composé permet une protection en surcharge dans les batteries au lithium, jouant ainsi un rôle de navette rédox.
Ce composé permet également de réaliser des systèmes électrochromes à colorants. On a ainsi réalisé un vitrage électrochrome en déposant sur une plaque de verre, recouverte d'une couche conductrice d'ITO (oxyde d'indium

et d'étain), une solution dans l'acétone de ce composé et de poly (benzodiimide-co-oxyde d'éthylène) d'une masse molaire ≈1100 g/mole, obtenu par un procédé similaire à celui décrit dans la demande de brevet Français FR 93/01117. Après évaporation du solvant et séchage, on a assemblé en boîte à gants, sur le polymère préalablement déposé, une deuxième électrode de verre recouverte d'une couche conductrice d'ITO. Après avoir scellé l'ensemble pour le rendre étanche, on a appliqué à l'extérieur un potentiel de 1250 mV à l'aide d'un potentiostat. Le système s'est alors coloré d'une couleur bleu intense. En appliquant un potentiel de -500 mV, on a pu observé une décoloration relativement rapide du système (inférieur à 60 s). Un tel système électrochrome est facile à mettre en oeuvre, même pour des systèmes de grande taille (supérieure au $m^2$) qui utilisent aussi bien du verre qu'un polymère convenablement traité comme électrode transparente conductrice. De plus, l'énergie nécessaire pour conserver la coloration est relativement faible, inférieure à 1 $W/m^2$.

**Exemple 25.**

Dans un réacteur chimique Parr, on a mis en solution dans 60 ml d'acétonitrile anhydre, 5,21 g (50 mmoles) du sel de potassium du malononitrile et 264 mg d'un éther-couronne, le 18-Crown-6. Après avoir fermé le réacteur, on a effectué un balayage d'argon pendant 15 min avant de l'isoler. On a alors introduit 6,41 g (50 mmoles) de dioxyde de soufre $SO_2$ (commercialisé par Fluka), puis, après 10 min, 9,52 g (50 mmoles) de vinyltriéthoxysilane (commercialisé par Fluka) en solution dans 20 ml de d'acétonitrile anhydre. Après 6 heures à température ambiante, le réacteur a été porté à 40°C et maintenu à cette température pendant 48 heures, puis le solvant a été évaporé. Après avoir été séché sous vide, le produit a été stocké sous argon. On a récupéré quantitativement le sel de potassium du 2-(triéthoxysilyl) éthylsulfonylmalononitrile $[(C_2H_5O)_3Si\ (CH_2)_2SO_2C(CN)_2)]K$, ayant une pureté, caractérisée par RMN du proton et du carbone supérieure, à 99%.

On a obtenu le sel de lithium par échange ionique avec le chlorure de lithium dans le tétrahydrofurane.

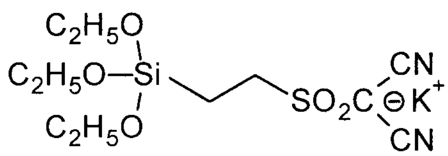

Ces sels permettent de former des réseaux organosiliciés par un mécanisme d'hydrolyse-polycondensation. Ils permettent également d'ensimer les matériaux à base de verre (fibre, vitrage,) afin de modifier leur état de surface et en particulier de leur conférer des propriétés antistatiques.

En outre, on peut obtenir les homopolymères ou des copolymères avec divers alkoxysilanes en milieu protique, éventuellement en présence d'un catalyseur (acide, base, fluorure,). On a préparé un copolymère en polycondensant le sel de potassium du 2-(triéthoxysilyl)éthylsulfonylmalononitrile avec le O-[2-(triméthoxysilyl)éthyl]-O'-méthylpolyéthylène glycol de masse 5000 (commercialisé par Shearwaters Polymers) (5:1) dans un mélange eau/méthanol, en utilisant comme catalyseur une trace d'acide perchlorique. Après quelques heures, la solution a été concentrée. On a alors imprégné un feutre de carbone activé, préalablement dégazé, d'une surface spécifique de 1500 $m^2/g$ (commercialisé par la société Actitex), avec le liquide visqueux obtenu. Après séchage, l'opération a été renouvelée pour parfaire l'imprégnation. Après une semaine dans une étuve à 50°C, on a découpé à l'emporte-pièces deux pastilles d'un diamètre de 2 cm. On a alors impregné une feuille de papier à cigarette (commercialisée par Bolloré Technologies) par un liquide visqueux identique à celui utilisé pour imprégner le feutre de carbone précité. Cette feuille a été déposée entre les deux électrodes de carbone préalablement découpées. Après une semaine dans une étuve à 50°C, puis 2 jours sous vide à 60°C, on a obtenu une supercapacité électrochimique "tout-solide". Cette supercapacité présente les caractéristiques suivantes à 40°C : une densité d'énergie de 15 Wh/l (soit une capacité de 96 F/g de carbone pour une électrode), une puissance maximum de 700 W/kg et de bons résultats en cyclage (plus de 10000 cyles de charge/décharge entre 0 et 2 V). Ce type de supercapacité est particulièrement intéressant pour l'électronique du fait de l'absence de liquides volatils.

**Exemple 26.**

Suivant un procédé similaire à celui décrit dans l'exemple 25, on a synthétisé le sel de lithium du bis[3-(triméthoxysilyl)propyl]aminosulfonylmalononitrile, à partir de 12,54 g (40 mmoles) de bis[3-(triméthoxysilyl)propyl]amine $[(CH_3O)_3Si\ (CH_2)_3]_2NH)$, (commercialisé par Fluka). Le composé obtenu avait une pureté, caractérisée par RMN du proton et du carbone,supérieure à 98%.

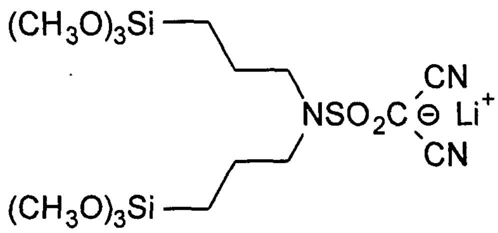

Ce composé présente des propriétés analogues à celles du composés de l'exemple 25 et peut être utilisé pour les mêmes applications.

On a effectué une polycondensation de ce composé dans un mélange eau/méthanol, en utilisant une goutte d'acide chlorhydrique concentré comme catalyseur. Après quelques heures, les solvants ont été évaporés et le liquide visqueux obtenu a été coulé sur une plaque de Téflon®. Après une semaine dans une étuve à 50°C, le matériau obtenu a été séché sous vide à 100°C pendant 48 heures, puis broyé sous argon jusqu'à obtenir une taille de particules de l'ordre du micron. On a alors préparé un électrolyte composite en mélangeant cette poudre avec du polyoxyde d'éthylène de masse 300000 dans l'acétonitrile. Après avoir coulé cette dispersion dans un anneau en verre et évaporé l'acétonitrile, on a obtenu un film d'électrolyte composite de bonne tenue mécanique, d'une épaisseur de 220 µm. Cette électrolyte présente une conductivité ionique supérieure à $10^{-5}$ $S.cm^{-1}$ à 60°C, et un nombre de transport cationique de 0,92.

**Exemple 27.**

Une solution dans 100 ml de tétrahydrofurane anhydre de 10,81 g (40 mmoles) du sel de lithium du 4-styrènesulfonylmalononitrile préparé comme à l'exemple 19, de 3,18 g d'acrylonitrile (40 mmoles) et de 100 mg de 1,1'-azobis-(cyclohexanecarbonitrile) a été dégazée par un balayage d'argon sec. On a alors effectué sous argon la copolymérisation de l'acrylonitrile avec le dérivé du styrène en chauffant le milieu réactionnel à 60°C pendant 48 heures. Après refroidissement, la solution a été concentrée, puis le polymère récupéré par reprécipitation dans l'éther. Après filtration et séchage, on a obtenu le sel de lithium du poly-(acrylonitrile-co-4-styrènesulfonylmalononitrile) (PANSSM).

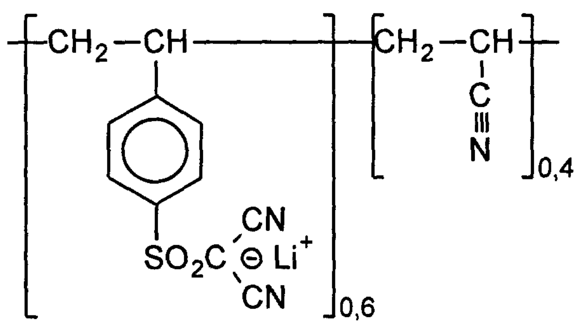

Ce polymère permet de réaliser des électrolytes polymères gélifiés à anions fixes. Il constitue la matrice permettant d'obtenir le gel et il présente les propriétés d'un polyélectrolyte.

On a préparé un électrolyte gélifié (30% en poids de PANSSM, 35% de carbonate d'éthylène, 35% de carbonate de propylène). Ce gel présente de bonnes propriétés mécaniques et une conductivité de $7{,}9.10^{-4}$ $S.cm^{-1}$ à 30°C. Le nombre de transport cationique de cet électrolyte a été estimé à 0,95. On a assemblé un générateur électrochimique en utilisant ledit électrolyte gélifié, une anode composite constituée par un coke de carbone (80% en volume) mélangé avec le copolymère (PANSSM) comme liant (20% en volume), et une cathode composite constituée par du noir de carbone (6% en volume), $LiCoO_2$ (75% en volume) et du copolymère (PANSSM) (20% en volume). Ce générateur a permis d'effectuer 1000 cycles de charge/décharge entre 3 et 4,2 V en conservant une capacité supérieure à 80% de la capacité au premier cycle, lors d'un cyclage à 25°C. Il présente de très bonnes performances lors d'appel de puissance du fait de l'utilisation d'anions fixes. L'utilisation d'anions fixes a également permis d'améliorer l'évolution de la résistance d'interface.

**Exemple 28.**

13,44 g (50 mmoles) de chlorure de 1-dodécanesulfonyle $C_{12}H_{25}SO_2Cl$ (commercialisé par Lancaster) en solution dans 30 ml de tétrahydrofurane anhydre à -20°C ont été traités par 8,8 g (100 mmoles) du sel de sodium du malononitrile. Après 1 heure à 0°C, puis 24 heures à température ambiante, le solvant a été évaporé et le produit repris dans 30 ml d'eau. L'addition de 3,73 g (50 mmoles) de chlorure de potassium KCl anhydre a permis d'obtenir un précipité qui a été recristallisé, puis récupéré par filtration et enfin séché. On a obtenu le sel de potassium du 1-dodécanesulfonylmalononitrile, sous forme d'une poudre cristallisée, ayant une pureté, déterminée par RMN du proton et du carbone supérieure, à 99%.

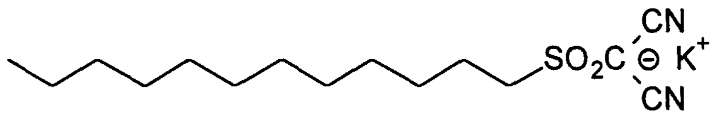

On a obtenu par un procédé similaire, les sels de potassium du 1-butanesulfonylmalononitrile et du 1-octylsulfonylmalononitrile à partir respectivement du chlorure de 1-butanesulfonyle et du chlorure de 1-octanesulfonyle.

Les sels de lithium de ces trois dérivés ont été préparés quantitativement par échange ionique entre le sel de potassium et le chlorure de lithium dans le tétrahydrofurane anhydre.

Le sel de lithium du 1-dodécanesulfonylmalononitrile dissous dans une matrice de polyoxyde d'éthylène à une concentration O/Li = 16/1 a un nombre de transport cationique ≈0,55. Il en résulte que, lorsque ce composé est utilisé dans l'électrolyte d'une batterie au lithium à électrolyte polymère, les gradients de concentration qui apparaissent au cours du fonctionnement de la batterie sont diminués de façon substantielle. Les performances lors d'appels de puissance sont ainsi améliorées.

Les sels du 1-dodécanesulfonylmalononitrile présentaient un intérêt indéniable comme additif pour le laminage du lithium et pour l'extrusion de polymères, en particulier l'extrusion du polyoxyde d'éthylène.

**Exemple 29.**

Dans 100 ml d'eau à 0°C, on a ajouté 23,01 g (100 mmoles) d'hexafluoropropanesultone (commercialisé par Fluorochem). Après 2 heures sous agitation, la phase aqueuse a été extraite par deux fractions de 20 ml de dichlorométhane, puis les phases organiques ont été réunies et séchées par du sulfate de magnésium. Après évaporation du dichlorométhane et distillation du liquide recueilli, on a obtenu 16,94 g de fluorure de 1-fluoro-2,2,2-trifluoroéthanesulfonyle $CF_3CHFSO_2F$ (rendement : 94% ; pureté, déterminée par RMN du proton et du fluor, supérieure à 99%).

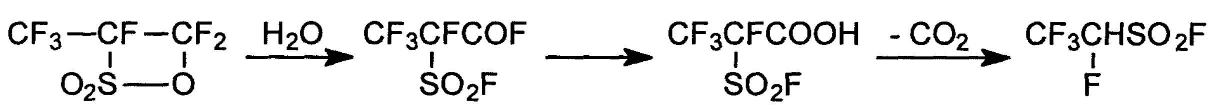


En opérant dans une boîte à gants sous argon, on a mis en solution 9,2 g (50 mmoles) du composé ainsi préparé dans 10 ml de tétrahydrofurane anhydre. Après avoir porté cette solution à -20°C, on a ajouté lentement 50 ml d'une solution 1 M (50 mmoles) dans le tétrahydrofurane de tert-butoxide de potassium $(CH_3)_3COK$ (commercialisé par Aldrich). Après 15 min, on a ajouté 12,46 g (50 mmoles) de 1-bromododécane. La réaction s'est poursuivie pendant 2 heures à -20°C, puis pendant 24 heures à température ambiante. On a alors ajouté 8,8 g (100 mmoles) du sel de sodium du malononitrile. Après 48 heures, le solvant a été évaporé et le résidu recristallisé dans 50 ml d'eau contenant 7,46 g (100 mmoles) de chlorure de potassium KCl. Après filtration et séchage, on a obtenu le sel de potassium du 1-dodécane-2,2,2-trifluoroéthanesulfonylmalononitrile ayant une pureté, caractérisée par RMN du proton, du carbone et du fluor, supérieure à 99%.

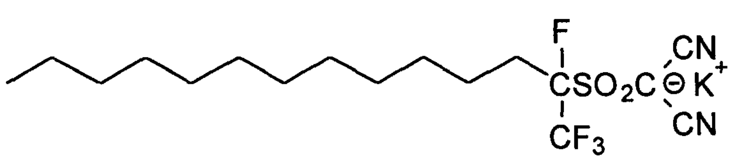

On a obtenu quantitativement le sel de lithium en traitant le sel de potassium dans le tétrahydrofurane anhydre par la quantité stoechiométrique de chlorure de lithium anhydre, filtration du milieu réactionnel, évaporation du solvant et séchage sous vide.
Ces composés peuvent être utilisés comme additif pour le laminage du lithium et pour l'extrusion de polymères, en particulier l'extrusion du polyoxyde d'éthylène. Ils présentent aussi des propriétés plastifiantes et antistatiques.

**Exemple 30.**

A 14,41 g (100 mmoles) du sel de sodium de l'acide 2-propène-sulfonique $CH_2=CHCH_2SO_3Na$ en suspension dans 60 ml d'acétonitrile anhydre à -20°C, on a ajouté goutte à goutte pendant 2 heures 11,9 g (100 mmoles) de chlorure de thionyle $SOCl_2$ dilués dans 20 ml de benzène. Après une nuit à -20°C, le milieu réactionnel a été centrifugé pour éliminer le chlorure de sodium formé et les solvants ont été évaporés à l'aide d'un évaporateur rotatif muni d'une pompe à membrane. Le liquide obtenu a alors été distillé sous vide sur une colonne courte pour donner 10,97 g du chlorure de 2-propène-sulfonyle $CH_2=CHCH_2SO_2Cl$ (rendement 78%) caractérisé par RMN du proton. On a alors fait réagir, sous argon, 7,03 g (50 mmoles) de ce composé avec 10,42 g (100 mmoles) du sel de potassium du malononitrile dans 60 ml d'acétonitrile anhydre à -20°C pendant 2 heures, puis à l'ambiante pendant 24 heures. Après évaporation du solvant, le produit a été recristallisé dans 15 ml d'eau. Après filtration et séchage, on a récupéré le sel de potassium du 2-propènesulfonylmalononitrile, ayant une pureté, caractérisée par RMN du proton et du carbone supérieure, à 98%.

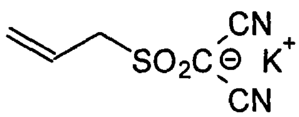

On a obtenu quantitativement le sel de lithium en traitant le sel de potassium dans le tétrahydrofurane anhydre par la quantité stoechiométrique de chlorure de lithium anhydre, filtration du milieu réactionnel, évaporation du solvant et séchage sous vide.
Ces sels peuvent être utilisés dans les réactions chimiques propres aux liaisons éthyléniques. Ils peuvent en particulier être homo- ou copolymérisés par une polymérisation initiée par voie radicalaire ou par un catalyseur de polymérisation coordiné tel un zircanocène.

**Exemple 31.**

A 8,33 g du sel de potassium du 2-propènesulfonylmalononitrile (40 mmoles), obtenu confomément à l'exemple 30, dans 100 ml d'eau, on a ajouté 6,9 g de l'acide 3-chloroperoxybenzoïque (40 mmoles), obtenu suivant la procédure décrite par Scwartz & Blumbergs (*J. Org. Chem.*, (1964), 1976). Après 1 heure sous forte agitation, le solvant a été évaporé puis le résidu a été recristallisé dans 10 ml d'éthanol. Après filtration et séchage, on a récupéré le sel de potassium du 2,3-époxypropane-1-sulfonylmalononitrile, ayant une pureté caractérisée par RMN du proton et du carbone supérieure à 98%.

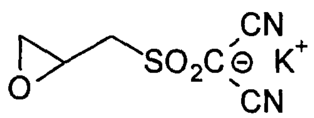

On a obtenu le sel de lithium en traitant le sel de potassium dans le tétrahydrofurane anhydre par la quantité stoechiométrique de chlorure de lithium anhydre, filtration du milieu réactionnel, évaporation du solvant et séchage sous vide.
Ces sels peuvent être homo- ou copolymérisés par une polymérisation initiée par voie anionique ou cationique. Plus généralement, ils peuvent subir les réactions chimiques propres aux oxétanes.
On a préparé l'homopolymère du sel de potassium du 2,3-époxybutane-1-sulfonylmalononitrile par une polymérisation dans le tétrahydrofurane initiée par voie anionique par le tert-butoxyde de potassium, puis le polysel de lithium par échange ionique dans le THF avec du chlorure de lithium anhydre. Ce dernier présente une conductivité en milieu gélifié (21% en poids de polyacrylonitrile, 38% de carbonate d'éthylène, 33% de carbonate de propylène, 8% de l'homopolymère) de $1{,}2.10^{-3}$ $S.cm^{-1}$ à 30°C. Le nombre de transport cationique dans cette électrolyte est de

0,76. De plus, cet homopolymère est soluble dans la plupart des solvants organiques usuels (tétrahydrofurane, acétonitrile, diméthylformamide, acétate d'éthyle, glymes,) et dans les polymères solvatants aprotiques.

**Exemple 32.**

50,74 g (300 mmoles) de 2-amino-5-trifluorométhyl-1,3,4-thiadiazole (commercialisé par Aldrich) ont été ajoutés sous agitation à un mélange de 100 ml d'acide chlorhydrique concentré et de 30 ml d'acide acétique glacial. On a porté le milieu réactionnel à -10°C et on a alors ajouté lentement 22,42 g (325 mmoles) de nitrite de sodium $NaNO_2$ dans 35 ml d'eau. La réaction de diazotation s'est poursuivie pendant 1 heure. Dans le même temps, on a fait passer à travers un fritté, un courant de dioxyde de soufre $SO_2$ (commercialisé par Fluka) dans 300 ml d'acide acétique glacial jusqu'à saturation. On a alors ajouté 7,5 g de chlorure de cuivre(I) CuCl et on a poursuivi l'addition de dioxyde de soufre jusqu'à ce que la couleur du milieu réactionnel passe du jaune-vert au bleu-vert. Après avoir porté le milieu réactionnel à une température < 10°C, on a ajouté sur une période de 30 min, le diazonium préparé au préalable. On a ajouté un peu d'éther pour diminuer la quantité de mousse qui se forme après chaque addition. Après la fin de l'addition du diazonium, on a versé le milieu réactionnel dans un 1 litre d'un mélange eau-glace (1:1). Après fusion de la glace, on a séparé une huile jaune dans une ampoule à décanter, puis on a extrait la phase aqueuse par deux fractions de 100 ml d'éther. Après addition de la phase éthérée à l'huile recueillie, la solution a été lavée par une solution concentrée de bicarbonate de sodium jusqu'à neutralité, puis avec de l'eau, et enfin séchée par du sulfate de magnésium. Après évoparation du solvant, on a récupéré après distillation sous vide 46,99 g du chlorure de 2-sulfonyl-5-trifluorométhyl-1,3,4-thiadiazole (62% de rendement) avec une pureté, caractérisée par RMN du proton et du fluor, supérieure à 98%.

Ensuite, par un procédé similaire à celui décrit dans l'exemple 19, on a obtenu le sel de lithium du 5-trifluorométhyl-1,3,4-thiadiazole-2-sulfonylmalononitrile, avec une pureté, caractérisée par RMN du carbone et du fluor, supérieure à 98%.

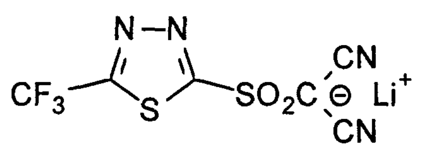

Le sel de potassium a été obtenu en traitant le sel de lithium, dans le minimum d'eau, par le fluorure de potassium KF. Après filtration, évaporation et séchage, on a récupéré quantitativement le sel de potassium.

Ces sels sont solubles dans la plupart des solvants organiques usuels (tétrahydrofurane, acétonitrile, diméthylformamide, acétate d'éthyle, glymes,) et dans les polymères solvatants aprotiques comme le polyoxyde d'éthylène.

Ces sels ont un potentiel d'oxydation, à une concentration 0,5 M dans l'acétonitrile, supérieure à 4,5 V vis à vis d'une anode de lithium. Le sel de lithium, seul ou en mélange avec le sel de potassium, peut être utilisé pour les batteries Li-Ion à électrolytes liquides ou électrolytes gels, et les batteries au lithium à électrolytes polymères.

On a assemblé une pile en utilisant une anode constituée par un mélange de coke de carbone (80% en volume) et de poly(fluorure de vinylidène) (PVDF, commercialisé par la société Montedison) comme liant (20% en volume), un électrolyte composé d'un mélange de carbonate d'éthylène et de diméthylcarbonate (2:1), gélifié par du PVDF, contenant le sel de lithium du 5-trifluorométhyl-1,3,4-thiadiazole-2-sulfonylmalononitrile à une concentration 1 M et une cathode composite constituée par un mélange de noir de carbone (6% en volume), de $Li_2MnO_4$ (75% en volume) et de PVDF comme liant (20% en volume). La pile a été soumise à un cyclage à 25°C. Après 1000 cycles de charge/décharge entre 2 et 4,7 V, la pile conservait une capacité représentant environ 50% de la capacité au premier cycle.

**Exemple 33.**

13,21 g (200 mmoles) de malononitrile ont été mis en solution dans 150 ml de THF à -20°C. On a alors ajouté, sous argon, 44,87 g (400 mmoles) de DABCO et 21,18 g (200 mmoles) de bromure de cyanogène BrCN. Après 4 heures sous agitation à -20°C, puis 24 heures à température ambiante, le milieu réactionnel a été filtré pour éliminer l'hydrochlorure de DABCO. On a alors ajouté 8,48 g de chlorure de lithium anhydre (200 mmoles). Après 24 heures sous agitation, le milieu réactionnel a été filtré pour éliminer l'hydrochlorure de DABCO. Après évaporation du solvant et séchage, on a récupéré 19 g (98% de rendement) du sel de lithium du tricyanométhane $LiC(CN)_3$, avec une pureté caractérisée par RMN du proton et du carbone supérieure à 98%.

On a obtenu l'acide en solution éthérée, en ajoutant 100 ml d'acide chlorhydrique 1 M à 0°C (100 mmoles)

à une suspension de 9,7 g du sel de lithium du tricyanométhane (100 mmoles) dans 30 ml d'éther. Après quelques minutes sous agitation, on a récupéré le tricyanométhane dans la phase éthérée. Après avoir séché la phase organique par du sulfate de magnésium, on a ajouté 7,15 g d'imidazole (105 mmoles). Il s'est immédiatement formé un précipité qui a été récupéré par filtration et séché. On a récupéré 15,23 g (96% de rendement) de l'imidazolium du tricyanométhane, ayant une pureté, caractérisée par RMN du proton et du carbone, supérieure à 99%.

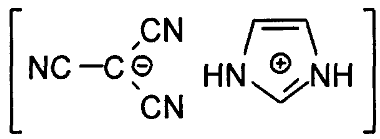

Le broyage en boîte à gants d'un mélange molaire de 7 imidazoles pour deux sels d'imidazolium a permis d'obtenir dans le mortier un liquide. Ce sel fondu présente une conductivité protonique élevée supérieure à $10^{-3}$ S.$cm^{-1}$ à 60°C. Ce sel fondu peut être utilisé pour préparer un électrolyte polymère, conducteur protonique anhydre, en ajoutant du polyoxyde d'éthylène, préférentiellement de haute masse ou pouvant être ultérieurement réticulé, au sel fondu sans que cela nuise à la conductivité. Ces électrolytes polymères sont particulièrement intéressants pour la réalisation de systèmes de modulation de la lumière telles que les vitrages électrochromes incluant les systèmes électrochromes à colorants.

Un électrolyte polymère composé du sel fondu à 80% en poids et de 20% en poids de polyoxyde d'éthylène de masse $5.10^6$ a été utilisé pour préparer une membrane optiquement transparente dans le visible ayant une bonne tenue mécanique. On a ensuite réalisé un système électrochrome en boîte à gants en utilisant cette membrane enfermée entre une première électrode constituée par le dépôt sur une plaque de verre d'une couche d'oxyde d'iridium hydrogéné $H_xIrO_2$ et d'une sous couche conductrice d'oxyde d'étain et une seconde électrode constituée d'une couche de trioxyde de tungstène $WO_3$ et d'une sous-couche conductrice d'oxyde d'étain. Cet électrochrome a permis une variation de l'absorption optique entre 80% (état décoloré) et 30% (état coloré) et de bonnes performances en cyclage (plus de 20 000 cycles).

**Exemple 34.**

A 6,61 g de malononitrile (100 mmoles) en solution dans 50 ml de THF à -20°C, on a ajouté par portions 795 mg d'hydrure de lithium LiH. Après deux heures à -20°C, on a ajouté 20,14 g (100 mmoles) de 1-(trifluorométhanesulfonyl)-imidazole (commercialisé par Fluka). La réaction s'est poursuivie pendant 4 heures à -20°C, puis 48 heures à température ambiante. Le solvant a alors été évaporé et le résidu repris dans 60 ml d'eau. On a ensuite ajouté 14,66 g (100 mmoles) du chlorure de 1-éthyl-3-méthyl-1*H*-imidazolium (commercialisé par Aldrich) à la solution aqueuse. Il s'est immédiatement formé une phase plus dense que l'eau. Cette phase a été récupérée par extraction au dichlorométhane. Après évaporation du dichlorométhane et séchage sous vide à 40°C du liquide obtenu, on a récupéré le sel fondu de 1-éthyl-3-méthyl-1*H*-imidazolium du trifluorométhanesulfonylmalononitrile, avec une pureté, caractérisée par RMN du proton, du carbone et du fluor, supérieure à 98%.

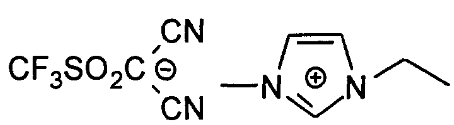

Ce sel fondu présente une conductivité de $4,5.10^{-3}$ S.$cm^{-1}$ et un point de congélation inférieure à -20°C. Son large domaine de stabilité rédox en fait un électrolyte particulièrement intéressant pour les générateurs électrochimiques tels que les batteries au lithium, les supercapacités, les systèmes de modulation de la lumière, les cellules photovoltaïques.

On a réalisé une supercapacité électrochimique en utilisant le sel fondu de l-éthyl-3-méthyl-1*H*-imidazolium du trifluorométhanesulfonylmalononitrile comme électrolyte et des composites carbone/aluminium comme électrodes. Les électrodes d'une épaisseur de 150 µm ont été placées de part et d'autre d'un polyéthylène microporeux ayant une épaisseur de 40 µm et le système complet a été scellé en boîte à gants dans un boitier de pile bouton après avoir été imbibé du sel fondu liquide. La supercapacité obtenue a permis de réaliser plus de 100.000 cycles de charge/décharge entre 0 et 2,5 V pour une densité d'énergie supérieure à 25 Wh/l et une puissance délivrée supérieure à 1500 W/l.

**Exemple 35.**

Dans 30 ml de THF, on a fait réagir 6,76 g (40 mmoles) de pentafluoropyridine (commercialisée par Aldrich) avec 4,17 g (40 mmoles) du sel de potassium du malononitrile $KHC(CN)_2$ en présence de 4,49 g (40 mmoles) de DABCO. Après 48 heures sous agitation, le solvant a été évaporé et le résidu recristallisé dans 15 ml d'eau additionée de 4 g de chlorure de potassium. Après filtration et séchage, on a obtenu 5,29 g du sel de potassium de la 4-malononitrilepentafluoropyridine (73% de rendement), ayant une pureté, déterminée par RMN du carbone, supérieure à 99%.
Suivant le même procédé, on a préparé le sel de potassium du 2-malononitrile-3,5-dinitrobenzotrifluorure à partir de 10,82 g (40 mmoles) du 2-chloro-3,5-dinitrobenzotrifluorure (commercialisé par Aldrich), ayant une pureté, déterminée par RMN du fluor, du proton et du carbone, supérieure à 99%.
On a obtenu les sels de lithium par échange ionique avec le chlorure de lithium dans le THF.
Ces sels sont solubles dans la plupart des solvants organiques usuels (tétrahydrofurane, acétonitrile, diméthylformamide, acétate d'éthyle, glymes,) et dans les polymères solvatants aprotiques.

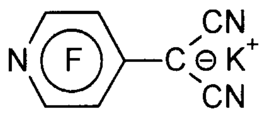

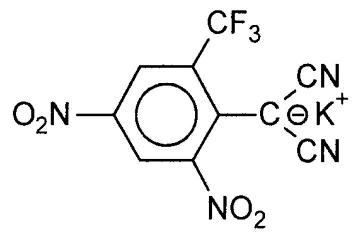

Ces exemples illustrent le greffage de l'anion du malononitrile sur un noyau aromatique comportant des substituants activés par la présence de groupements électroattracteurs et/ou d'hétéroatomes dans le cycle aromatique.

**Exemple 36.**

On a introduit dans un ballon tricol équipé d'un réfrigérant, d'une agitation mécanique et d'une entrée de gaz neutre (Argon), 9,5 g d'un copolymère de diméthylsiloxane et d'(hydrogéno)(méthyl)-siloxane (HMS 301 25% SiH, $M_w$ 1900, commercialisé par Gelest Inc., Tullytown, PA, USA) en solution dans du tétrahydrofurane. On a ensuite ajouté 6,04 g du sel de lithium du vinylsulfonylmalononitrile et 70 mg d'acide chloroplatinique $H_2PtCl_6$. Le mélange a été chauffé au reflux pendant 4 heures. Le polymère a ensuite été reprécipité dans l'éthanol.
On a ainsi obtenu le copolymère de diméthylsiloxane et du sel de lithium de 1' (éthylsulfonylmalononitrile) (méthyl)-siloxane, qui est soluble dans la plupart des solvants organiques, y compris à des teneurs > 2% dans les huiles ou les matériaux siliconés, auxquels il confère des propriétés antistatiques.

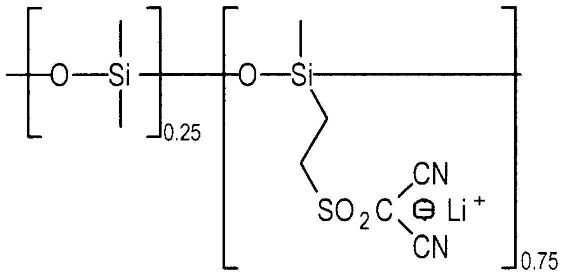

**Exemple 37.**

Suivant un procédé similaire à celui décrit dans l'exemple 28, on a obtenu le sel de potassium du (1*R*)-(-)-10-camphorsulfonylmalononitrile à partir du chlorure de (1*R*)-(-)-10-camphorsulfonyle (commercialisé par Aldrich) et le sel de potassium du (1*S*)-(+)-camphorsulfonylmalononitrile à partir du chlorure de (1*S*)-(+)-10-camphorsulfonyle (commercialisé par Aldrich) avec des rendements supérieurs à 70% et une pureté, déterminée par RMN du proton et du carbone, supérieure à 99%.

Les sels de lithium correspondants ont été obtenus par échange ionique (métathèse) dans le tétrahydrofurane avec le chlorure de lithium.

Les sels de scandium ont été obtenus en traitant les sels de potassium correspondants par une quantité stoechiométrique de tétrafluoroborate de scandium $Sc(BF_4)_3$ dans l'acétonitrile. Après filtration pour éliminer le précipité de tétrafluoroborate de potassium $KBF_4$ et évaporation du solvant, on a récupéré quantitativement les sels de scandium des deux énantiomères.

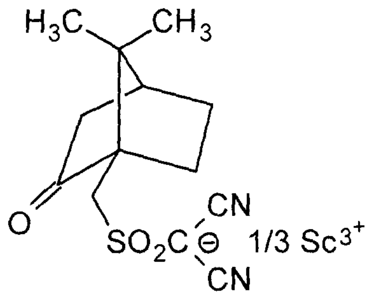

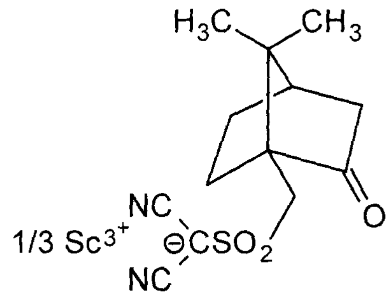

Ces sels sont solubles dans la plupart des solvants organiques polaires (acétonitrile, tétrahydrofurane, DMF,) et dans les polymères solvatants aprotiques.

**Exemple 38.**

Les deux énantiomères du sel de lithium du (N-Méthoxybutyl-N-2-butyl-3-méthyl)aminosulfonylmalononitrile ont été obtenus par un procédé similaire à celui décrit dans l'exemple 14, à partir des deux énantiomères de la N-Méthoxybutyl-N-2-butyl-3-méthyl-amine (commercialisé par Air Products) avec une pureté supérieure à 97%.

Les sels de potassium ont été obtenus en traitant les sels de lithium par le fluorure de potassium KF dans l'eau. Après filtration, évaporation et séchage, on a récupéré quantitativement les sels de potassium.

Les sels de scandium ont été obtenus en traitant les sels de potassium par une quantité stoechiométrique de tétrafluoroborate de scandium $Sc(BF_4)_3$ dans l'acétonitrile. Après filtration pour éliminer le précipité de tétrafluoroborate de potassium $KBF_4$ et évaporation du solvant, on a récupéré quantitativement les sels de scandium des deux énantiomères.

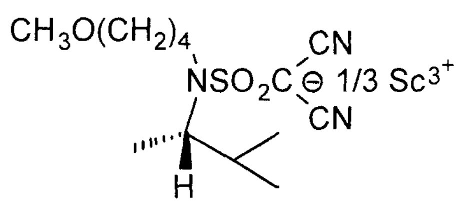

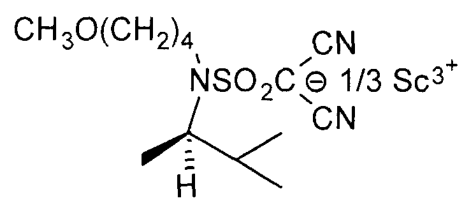

Ces sels sont solubles dans la plupart des solvants organiques polaires (acétonitrile, tétrahydrofurane, DMF,) et dans les polymères solvatants aprotiques.

**Exemple 39.**

1,82 g (10 mmoles) de l'acide (1*S*)-(+)-kétopinique (commercialisé par Aldrich) ont été mis en solution dans 10 ml de pyridine et on a ajouté 1,04 g (10 mmoles) de sel de potassium du malononitrile et 2,06 g (10 mmoles)de dicyclohexylcarbodiimide. Après 48 h sous agitation, le mélange a été filtré pour éliminer la dicyclohexylurée. Après évaporation du filtrat, on a obtenu le sel de potassium du (1*S*) - (+)-kétopinique-acétylmalononitrile avec une pureté, déterminée par RMN du proton et du carbone, supérieure à 97%.

Le sel de scandium a été obtenu en traitant le sel de potassium par une quantité stoechiométrique de tétrafluoroborate de scandium $Sc(BF_4)_3$ dans l'acétonitrile. Après filtration pour éliminer le précipité de tétrafluoroborate de potassium $KBF_4$ et évaporation du solvant, on a récupéré quantitativement le sel de scandium du (1*S*)-(+)-kétopiniqueacétylmalononitrile.

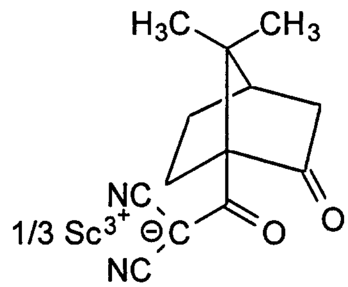

**Exemple 40.**

A 18,11 g (100 mmoles) de 6-bromo-1-hexanol et 11,22 g (100 mmoles) de DABCO dans 100 ml de THF anhydre à -20°C, on a ajouté lentement 19,06 g (100 mmoles) de chlorure de tosyle. Après 24 heures sous agitation à -20°C, le milieu réactionnel a été filtré pour éliminer le précipité d'hydrochlorure de DABCO. Après évaporation du solvant, on a récupéré quantitativement le tosylate du 6-bromo-1-hexanol $CH_3FSO_2O(CH_2)_6Br$. Ce composé a ensuite été dissous dans 200 ml de THF avec 40 g d'aniline $FNH_2$ (200 mmoles) et cette solution a été portée au reflux pendant une nuit. Après refroidissement, on a ajouté 300 ml d'eau et la phase organique a été extraite avec de l'éther. Après avoir été lavée avec de l'eau, la phase éthérée a été séchée par du sulfate de magnésium. On a obtenu après évaporation et séchage, 23 g de la N-(6-bromohexyl)aniline.

En opérant dans une boîte à gants sous argon, on a mis en solution 9,2 g de fluorure de 1-fluoro-2,2,2-trifluoroéthanesulfonyle $CF_3CHFSO_2F$ (50 mmoles), préparé conformément à l'exemple 29, dans 10 ml de tétrahydrofurane anhydre. Après avoir porté cette solution à -20°C, on a ajouté lentement 50 ml d'une solution 1 M dans le tétrahydrofurane de tert-butoxide de potassium $(CH_3)_3COK$ (commercialisé par Aldrich). Après 15 min, on a ajouté 12,81 g (50 mmoles) de N-(6-bromohexyl)aniline. La réaction s'est poursuivie pendant deux heures à -20°C, puis pendant 24 heures à température ambiante. On a alors ajouté 8,8 g (50 mmoles) du sel de sodium du malononitrile et 5,61 g de DABCO. Après 48 heures, le milieu réactionnel a été filtré pour éliminer le précipité d'hydrochlorure de DABCO, puis le solvant a été évaporé et le résidu recristallisé dans 50 ml d'eau contenant 7,46 g (100 mmoles) de chlorure de potassium KCl. Après filtration et séchage, on a obtenu le sel de potassium du 1-(6-anilino-1-hexane)-2,2,2-trifluoroéthanesulfonylmalononitrile avec une pureté, caractérisée par RMN du proton, du carbone et du fluor, supérieure à 99%.

8,87 g de ce composé (20 mmoles) ont ensuite été dissous dans 200 ml d'une solution 1 M d'acide chlorhydrique, et on a ajouté lentement sur une période de 3 heures, 4,56 g (20 mmoles) de persulfate d'ammonium $(NH_4)_2S_2O_8$. La réaction s'est poursuivie pendant 14 heures. Le milieu réactionnel a alors été neutralisé par de l'hydroxyde de potassium, puis concentré à un volume de ≈40 ml. On a récupéré après filtration et séchage 5,9 g d'une poudre noire du composé suivant :

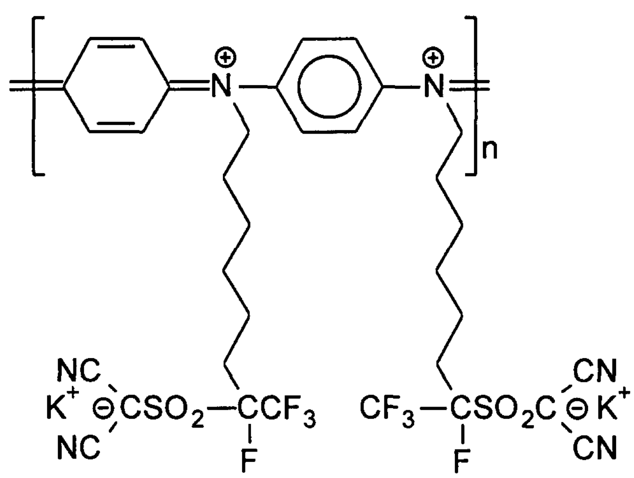

Ce composé polymère qui comprend un anion dopant très délocalisé dans sa structure, présente des propriétés de conducteur électronique (PCE). La faible basicité de cet anion améliore la stabilité du polymère. En milieu

humide, cette conductivité déterminée par une mesure quatres points est initialement de l'ordre de 4 $S.cm^{-1}$.

On a testé ce matériau comme cathode de batterie. La batterie avait la constitution suivante :

- une cathode composite constituée par 40% en volume du composé polymère obtenu dans le présent exemple et 60% en volume de polyoxyde d'éthylène de masse $3.10^5$ ;
- un électrolyte constitué par un film de polyoxyde d'éthylène de masse $5.10^6$ contenant le sel de lithium de du butanesulfonylmalononitrile, obtenu à l'exemple 28, à une concentration à une concentration O/Li = 20/1 ;
- une anode de lithium métallique.

Après assemblage de l'ensemble dans un boîtier de pile bouton, la batterie obtenue a été cyclée à une température de 60°C entre 3 V et 3,9 V. Plus de 1000 cycles de charge/décharge ont pu être effectués en conservant 80% de la capacité du premier cycle.

En outre, le composé polymère du présent exemple est un bon inhibiteur de corrosion des métaux ferreux et permet de réaliser des dépôts sur des plastiques traités par effet Corona.

**Exemple 41.**

A 11,8 g (0,1 mole) d'oxalate de méthyle dans 30 ml de THF on a ajouté 20,8 g (0,2 moles) du sel de potassium du malononitrile en solution dans 80 ml de THF. Un précipité s'est formé en quelques minutes. Le mélange a été maintenu sous agitation en atmosphère neutre pendant deux heures, puis filtré et lavé avec deux portions de 50 ml d'éther. On a obtenu le sel de potassium de l'anion suivant, sous forme d'un solide beige :

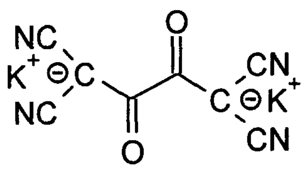

Ce composé forme des complexes anioniques avec les métaux comme l'aluminiumn, le zinc, le magnesium, le fer, le Chrome. Ces complexes sont solubles dans les solvants aprotiques ou les polymères solvatants et sont utiles comme transporteurs véhiculaires des métaux complexés (Mg, Al) ou comme couple rédox stables (Fe, Cr).

**Exemple 42.**

A 1,98 g (10 mmoles) de sulfonyldiimidazole dans 10 ml d'acétonitrile ont été ajoutés 1,42 g (20 mmoles) de sel de sodium du malononitrile en solution dans 20 ml d'éther. Un précipité s'est formé immédiatement. Le mélange a été maintenu sous agitation en atmosphère neutre pendant deux heures, puis filtré et lavé avec deux portion de 20 ml d'ether. On a obtenu, sous forme d'un solide blanc, le sel de lithium de l'anion

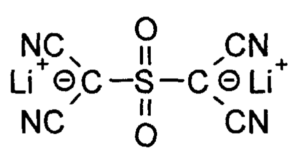

Ce sel est soluble dans les solvant polaires tels que le carbonate de propylène ou les polymères solvatants à base d'oxyde d'éthylène et donne des conductivités de l'ordre de $10^{-4}$ $S.cm^{-1}$ à 90°C avec une excellente stabilité de l'interface avec le lithium.

**Exemple 43**

Le sel de lithium du trifluorométhanesulfonylmalononitrile obtenu à l'exemple 2 et le polysel de lithium du 2,3-époxypropane-1-sufonylmalononitrile obtenu à l'exemple 31 ont été testés dans des générateurs électrochimiques selon la technologie lithium-polymère.

Pour chaque sel, on a réalisé une batterie en superposant les couches suivantes :

- un collecteur de courant en acier inoxydable ayant une épaisseur de 2 mm ;
- une cathode constituée par une pastille d'un film de matériau composite ayant une épaisseur de 72 µm et constitué par du dioxyde de vanadium (45% en volume), du noir de Shawinigan (5% en volume) et un polyoxyde d'éthylène de masse $M_w = 3.10^5$ (50% en volume) ;
- un électrolyte constitué par une pastille d'un film de polyoxyde d'éthylène de masse $M_w = 5.10^6$ contenant un des deux sels de lithium à une concentration O/Li = 15/1 ;
- une anode constituée par une feuille de lithium métallique ayant une épaisseur de 50 µm ;
- un collecteur de courant analogue au collecteur précité.

Les pastilles constituant les électrodes et l'électrolyte ont été découpées en boîte à gants et empilées dans l'ordre indiqué ci-dessus. Les collecteurs ont ensuite été déposés de part et d'autre de l'empilement obtenu.

On a scellé le tout dans un boîtier de pile bouton, qui permet à la fois de protéger le générateur de l'atmosphère et d'excercer une contrainte mécanique sur les films. La batterie a alors été placée dans une enceinte sous argon installée dans une étuve à une température de 60°C. Elle a ensuite été cyclée entre 1,8 et 3,3 V à un régime de charge et de décharge de C/10 (capacité nominale chargée ou déchargée en 10 heures).

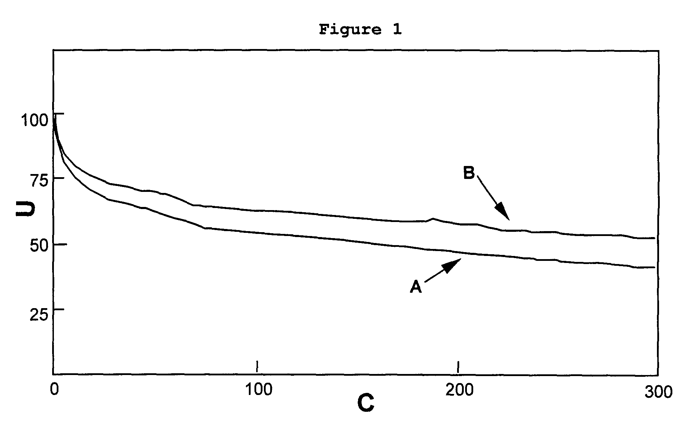

Les courbes de cyclage obtenues sont représentées sur la figure 1 (sel de lithium du trifluorométhanesulfonylmalononitrile : courbe A ; polysel de lithium du 2,3-époxypropane-1-sufonylmalononitrile : courbe B). Sur cette figure, l'utilisation, U, exprimée en % est donnée en ordonnée, et le nombre de cycles C est donné en abscisse.

## Revendications

1. Matériau à conduction ionique comprenant un composé ionique en solution dans un solvant, ledit composé ionique étant un dérivé du malononitrile comprenant une partie anionique associée à au moins une partie cationique $M^{m+}$ en nombre suffisant pour assurer la neutralité électronique de l'ensemble, **caractérisé en ce que** M est un hydroxonium, un nitrosonium $NO^+$, un ammonium $-NH_4^+$, un cation métallique ayant la valence m, un cation organique ayant la valence m ou un cation organométallique ayant la valence m, et que la partie anionique répond à l'une des formules $R_D\text{-}Y\text{-}C(C\equiv N)_2^-$ ou $Z\text{-}C(C\equiv N)_2^-$ dans lesquelles :

   - Z représente un radical électro-attracteur ayant un paramètre de Hammett au moins égal à celui d'un radical phényle, choisi parmi :

     j) -CN, $-NO_2$, -SCN, $-N_3$, $-CF_3$, $FSO_2$-, $R'_FCH_2$- ($R'_F$ étant un radical perfluoré), les radicaux fluoroalkyloxy, fluoroalkylthioxy, fluoroalkényloxy, fluoroalkénylthioxy ;
     jj) les radicaux comprenant un ou plusieurs noyaux aromatiques contenant éventuellement au moins un atome d'azote, d'oxygène, de soufre ou de phosphore, les dits noyaux pouvant être éventuellement des noyaux condensés et/ou lesdits noyaux pouvant éventuellement porter au moins un substituant choisi parmi les halogènes, -CN, $-NO_2$, -SCN, $-N_3$, $CF_2$=CF-O-, les radicaux $R_F$- et $R_F$-$CH_2$- dans lesquels $R_F$ représente un radical perfluoroalkyle ayant de 1 à 12 atomes de carbone, les groupes fluoroalkyloxy, les groupes fluoroalkylthioxy, les radicaux alkyles, alkényles, oxaalkyles, oxaalkényles, azaalkyles, azaalkényles, thiaalkyles, thiaalkényles, les radicaux polymères, les radicaux possédant au moins un groupement ionophore cationique et/ou au moins un groupement ionophore anionique ;

   - Y représente un groupement carbonyle, un groupement thiocarbonyle, un groupement sulfonyle, un groupement sulfinyle ou un groupement phosphonyle et :
   - $R_D$ est un radical choisi parmi :

     a) les radicaux alkyle ou alkényle ayant de 1 à 24 atomes de carbone, et les radicaux aryle, arylalkyle, alkylaryle ou alkénylaryle ayant de 5 à 24 atomes de carbone, les radicaux alicycliques ou hétérocycliques, y compris les radicaux polycycliques ;
     b) les radicaux alkyle ou alkényle ayant de 1 à 12 atomes de carbone et comprenant éventuellement au moins un hétéroatome O, N ou S dans la chaîne principale ou dans une chaîne latérale, et/ou comprenant au moins un groupe fonctionnel éther, thioéther, amine, imine, amide, carboxyle, carbonyle, isocyanate, isothiocyanate, thiocarbonyle, hydroxy,; les radicaux oxa-alkyle, oxa-alkényle, aza-alkyle, aza-alkényle, thia-alkyle ou thia-alkényle ayant de 1 à 24 atomes de carbone ;
     c) les radicaux aryle, arylalkyle, arylalkényle, alkylaryle ou alkénylaryle, dans lesquels les noyaux aromatiques et/ou au moins un substituant du noyau comprennent des hétéroatomes tels que l'azote, l'oxygène,

le soufre ;
d) les radicaux comprenant des cycles aromatiques condensés qui comprennent éventuellement au moins un hétéroatome choisi parmi l'azote, l'oxygène, le soufre ;
e) les radicaux halogénés ou perhalogénés alkyle, alkényle, aryle, arylalkyle, alkylaryle, lesdits radicaux comprenant éventuellement des groupes fonctionnels éther, thioéther, imine, amine, carboxyle, carbonyle ou hydroxy ;
f) les radicaux $R_cC(R')(R'')$-O- dans lesquels $R_C$ est un radical alkylperfluoré et R' et R'' sont indépendamment l'un de l'autre un atome d'hydrogène ou un radical tel que défini en a), b), c) ou d) ci-dessus;
g) les radicaux $(R_B)_2N$-, dans lesquels les radicaux $R_B$ identiques ou différents sont tels que définis en a), b), c), d) et e) ci-dessus, l'un des $R_B$ pouvant être un atome d'hydrogène, ou bien les deux radicaux $R_B$ forment ensemble un radical divalent qui forme un cycle avec N ;
h) les radicaux poly(oxyalkylène) et poly(oxyalkylène)-alkyléther,
i) les radicaux possédant un ou plusieurs groupements ionophores cationiques et/ou un ou plusieurs groupements ionophores anioniques ;

étant entendu que :

- un substituant Z peut être un radical monovalent, une partie d'un radical ayant une valence égale à 2 portant deux groupements $-C^{-}(C{\equiv}N)_{2'}$ ou un segment d'un polymère ;
- un substituant $R_D$ peut être un radical monovalent, une partie d'un radical ayant une valence supérieure à 2 portant plusieurs groupements $-Y-C^{-}(C{\equiv}N)_2$' ou un segment d'un polymère ;
- lorsque Y est un carbonyle et $R_D$ est un radical perfluoroalkyle ayant de 1 à 3 atomes de carbone, ou lorsque Z est -CN, M est différent d'un métal alcalin ;
- "segment d'un polymère" signifie unité récurrente éthylène portant éventuellement un substituant alkylène ou un substituant oxyalkénylène, unité récurrente oxyalkylène portant éventuellement un groupe méthylène, unité récurrente styrène portant éventuellement un substituant polyoxyéthylène, unité récurrente de 2,3-époxypropane, unité récurrente azaéthylène, unité récurrente 2-(triéthoxysilyl)éthyl-co-O-[2-(triméthoxysilyl)éthyl] -O'-méthylpolyéthylène glycol, unité récurrente bis[3-triméthoxysilyl)propyl]amino, unité récurrente méthyléthylsiloxane, unité récurrente pyrrole, unité récurrente acrylonitrile-co-4-styrényle, ou unité récurrente aniline portant un substituant fluoroalkyle ;
- "groupement ionophore cationique" signifie un groupement iodonium, sulfonium, oxonium, ammonium, amidinium, guanidinium, pyridinium, imidazolium, imidazolinium, triazolium, phosphonium ou carbonium, ledit groupement ionique jouant totalement ou partiellement le rôle du cation $M^+$ ;
- "groupement ionophore anionique" signifie une fonction carboxylate ($-CO_2^-$), une fonction sulfonate ($-SO_3^-$), une fonction sulfonimide ($-SO_2NSO_2$-) ou une fonction sulfonamide ($-SO_2N$-).

**2.** Matériau à conduction ionique selon la revendication 1, **caractérisé en ce que** le cation organique est un cation onium choisi dans le groupe constitué par les cations $R_3O^+$ (oxonium), $NR_4^-$ (ammonium), $RC(NR_2)_2^+$ (amidinium), $C(NR_2)_3$+ (guanidinium), $C_5R_6N^+$ (pyridinium), $C_3R_5N_2^+$ (imidazolium), $C_3R_7N_2^+$ (imidazolinium), $C_2R_4N_3^+$ (triazolium), $SR_3^+$ (sulfonium), $PR_4^+$ (phosphonium), $IR_2^+$ (iodonium), $(C_6R_5)_3C^+$ (carbonium), les radicaux R représentant de manière indépendante un H ou un radical choisi dans le groupe constitué par :

- les radicaux alkyles, alkényles, oxa-alkyles, oxa-alkényles, aza-alkyles, aza-alkényles, thia-alkyles, thia-alkényles, aryles, arylalkyles, alkylaryles, alkényl-aryles, les radicaux dialkylamino et les radicaux dialkylazo ;
- les radicaux cycliques ou hétérocycliques comprenant éventuellement au moins une chaîne latérale comprenant des hétéroatomes tels que l'azote, l'oxygène, le soufre ;
- les radicaux cycliques ou hétérocycliques comprenant éventuellement des hétéroatomes dans le noyau aromatique ;
- les groupes comprenant plusieurs noyaux aromatiques ou hétérocycliques, condensés ou non, contenant éventuellement au moins un atome d'azote, d'oxygène, de soufre ou de phosphore ;

étant entendu que plusieurs radicaux R peuvent former ensemble des cycles aromatiques ou non englobant éventuellement le centre portant la charge cationique.

**3.** Matériau à conduction ionique selon la revendication 2, **caractérisé en ce que** le cation onium fait partie du radical Z ou du radical $R_D$.

**4.** Matériau à conduction ionique selon la revendication 2, **caractérisé en ce que** le cation onium fait partie d'une

unité récurrente d'un polymère.

5. Matériau à conduction ionique selon la revendication 2, **caractérisé en ce que** le cation $M^+$ est un hétérocycle cationique à caractère aromatique, comportant au moins un atome d'azote alkylé dans le cycle.

6. Matériau à conduction ionique selon la revendication 5, **caractérisé en ce que** le cation est un-imidazolium, un triazolium, un pyridinium, un 4-diméthylamino-pyridinium, lesdits cations portant éventuellement un substituant sur les atomes de carbone du cycle.

7. Matériau à conduction ionique selon la revendication 2, **caractérisé en ce que** le cation M est un groupement possédant une liaison -N=N, $-N=N^+$, un groupement sulfonium, un groupement phosphonium, un groupement iodonium, ou un cation arène-ferrocénium substitué ou non, éventuellement incorporé dans une trame polymérique.

8. Matériau à conduction ionique selon la revendication 2, **caractérisé en ce que** le cation est un cation diaryliodonium, un cation dialkylaryliodonium, un cation triarylsulfonium, un cation trialkylaryle sulfonium, ou un cation phénacyl-dialkyl sulfonium substitué ou non.

9. Matériau à conduction ionique selon la revendication 2, **caractérisé en ce que** le cation aryliodonium, ou le cation alkylaryliodonium, ou le cation triarylsulfonium, ou le cation alkylaryle sulfonium, ou le cation phénacyl-dialkyl sulfonium font partie d'une chaîne polymère.

10. Matériau à conduction ionique selon la revendication 2, **caractérisé en ce que** M est un cation organique incorporant un groupement 2,2'[Azobis(2-2'-imidazolinio-2-yl)propane]$^{2+}$ ou 2,2'-Azobis(2-amidiniopropane)$^{2+}$.

11. Matériau à conduction ionique selon la revendication 1, **caractérisé en ce que** le cation M est un cation métallique choisi dans le groupe constitué par les cations de métaux alcalins, les cations de métaux alcalino-terreux, les cations de métaux de transition, les cations de métaux trivalents et les cations de terres rares.

12. Matériau à conduction ionique selon la revendication 1, **caractérisé en ce que** le cation est un métallocénium, choisi dans le groupe constitué par les cations dérivés du ferrocène, du titanocène et du zirconocène, les cations indénocénium, les cations arène métallocénium, les cations des métaux de transition complexés par des ligands de type phosphine possédant éventuellement une chiralité et les cations organométalliques possédant un ou plusieurs groupements alkyles ou aryles fixés d'une manière covalente à un atome ou un groupe d'atome, lesdits cations faisant éventuellement partie d'une chaîne polymère.

13. Matériau à conduction ionique selon la revendication 1, **caractérisé en ce que** le substituant Z est choisi dans le groupe constitué par $-OC_nF_{2n+1}$, $-OC_2F_4H$, $-SC_nF_{2n+1}$ et $-SC_2F_4H$, $-OCF=CF_2$, $-SCF=CF_2$, n étant un nombre entier de 1 à 8.

14. Matériau à conduction ionique selon la revendication 1, **caractérisé en ce que** Z est un radical $C_nF_{2n+1}CH_2$-, n étant un nombre entier de 1 à 8.

15. Matériau à conduction ionique selon la revendication 1, **caractérisé en ce que** $R_D$ est partie d'un radical poly (oxyalkylène) ou d'un radical polystyrène.

16. Matériau à conduction ionique selon l'une des revendications 1, **caractérisé en ce que** $R_D$ comporte au moins une insaturation éthylénique et/ou un groupe condensable et/ou un groupe dissociable par voie thermique, par voie photochimique ou par dissociation ionique.

17. Matériau à conduction ionique selon l'une des revendications 1, **caractérisé en ce que** $R_D$ représente un groupe mésomorphe ou un groupe chromophore ou un polymère conducteur électronique autodopé ou un alcoxysilane hydrolysable.

18. Matériau à conduction ionique selon la revendication 1, **caractérisé en ce que** $R_D$ représente une unité récurrente d'une chaîne polymère.

19. Matériau à conduction ionique selon la revendication 1, **caractérisé en ce que** $R_D$ comporte un groupement susceptible de piéger les radicaux libres.

**20.** Matériau à conduction ionique selon la revendication 1, **caractérisé en ce que** $R_D$ comporte ou un dipôle dissociant.

**21.** Matériau à conduction ionique selon la revendication 1, **caractérisé en ce que** $R_D$ comporte un couple rédox.

**22.** Matériau à conduction ionique selon la revendication 1, **caractérisé en ce que** $R_D$ comporte ou un ligand complexant.

**23.** Matériau à conduction ionique selon la revendication 1, **caractérisé en ce que** $R_D$-Y- est optiquement actif.

**24.** Matériau à conduction ionique selon la revendication 1, **caractérisé en ce que** $R_D$CO représente un acide aminé, ou un polypeptide optiquement ou biologiquement actif.

**25.** Matériau à conduction ionique selon la revendication 1, **caractérisé en ce que** $R_D$ représente un radical ayant une valence v supérieure à deux, comportant au moins un groupe **-Y-**$C(C{\equiv}N)_2^- M^+$.

**26.** Matériau à conduction ionique selon la revendication 1, **caractérisé en ce que** le substituant $R_D$ du composé ionique est un alkyle ou un alkényle qui contient au moins un hétéroatome choisi parmi O, N et S ; un alkyle ou un alkényle portant un groupe hydroxy, un groupe carbonyle, un groupe amine, un groupe carboxyle ; un aryle, un arylalkyle, un alkylaryle ou un alkénylaryle dans lesquels les chaînes latérales et/ou les noyaux aromatiques comprennent des hétéroatomes.

**27.** Matériau selon la revendication 1, **caractérisé en ce que** le substituant $R_D$ est une unité récurrente d'un polymère.

**28.** Matériau à conduction ionique selon la revendication 1 , **caractérisé en ce que** le solvant est soit un solvant liquide aprotique, choisi parmi les éthers linéaires et les éthers cycliques, les esters, les nitriles, les dérivés nitrés, les amides, les sulfones, les sulfolanes, les alkylsulfamides et les hydrocarbures partiellement halogénés, soit un polymère polaire, soit un de leurs mélanges.

**29.** Matériau à conduction ionique selon la revendication 28 , **caractérisé en ce que** le solvant est un polymère solvatant, réticulé ou non, portant ou non des groupements ioniques greffés.

**30.** Matériau à conduction ionique selon la revendication 29 , **caractérisé en ce que** le polymère solvatant est choisi parmi les polyéthers de structure linéaire, peigne ou à blocs, formant ou non un réseau, à base de poly(oxyde d'éthylène), les copolymères contenant le motif oxyde d'éthylène ou oxyde de propylène ou allylglycidyléther, les polyphosphazènes, les réseaux réticulés à base de polyéthylène glycol réticulé par des isocyanates, les réseaux obtenus par polycondensation et portant des groupements qui permettent l'incorporation de groupements réticulables et les copolymères à blocs dans lesquels certains blocs portent des fonctions qui ont des propriétés rédox.

**31.** Matériau à conduction ionique selon la revendication 1 , **caractérisé en ce que** le solvant est constitué essentiellement par un solvant liquide aprotique et un solvant polymère polaire comprenant des unités contenant au moins un hétéroatome choisi parmi le soufre, l'oxygène, l'azote et le fluor.

**32.** Matériau à conduction ionique selon la revendication 31 , **caractérisé en ce que** le polymère polaire contient principalement des unités dérivées de l'acrylonitrile, du fluorure de vinylidène, de la N-vinylpyrrolidone ou du méthacrylate de méthyle.

**33.** Matériau à conduction ionique selon la revendication 1 , **caractérisé en ce qu'**il contient en outre un second sel.

**34.** Matériau à conduction ionique selon la revendication 1 , **caractérisé en ce qu'**il contient en outre une charge minérale ou organique sous forme de poudre ou de fibres.

**35.** 44. Générateur électrochimique comprenant une électrode négative et une électrode positive séparées par un électrolyte, **caractérisé en ce que** l'électrolyte est un matériau selon l'une des revendications 1 à 34

**36.** Générateur selon la revendication 35, **caractérisé en ce que** l'électrode négative est constituée par du lithium

métallique, ou par l'un de ses alliages, éventuellement sous forme de dispersion manométrique dans de l'oxyde de lithium, ou par un nitrure double de lithium et d'un métal de transition, ou par un oxyde à bas potentiel ayant pour formule générale $Li_{1+y+x/3}Ti_{2-x/3}O_4$ ($0 \leq x \leq 1$, $0 \leq y \leq 1$), ou par le carbone et les produit carbonés issus de la pyrolyse de matières organiques.

**37.** Générateur selon la revendication 35, **caractérisé en ce que** l'électrode positive est choisie parmi les oxydes de vanadium $VO_x$ ($2 \leq x \leq 2{,}5$), $LiV_3O_8$, $Li_yNi_{1-x}Co_xO_2$, ($0 \leq x \leq 1$ ; $0 \leq y \leq 1$), les spinelles de manganèse $Li_yMn_{1-x}M_xO_2$ (M = Cr, Al, V, Ni, $0 \leq x \leq 0{,}5$ ; $0 \leq y \leq 2$), les polydisulfures organiques, FeS, $FeS_2$, le sulfate de fer $Fe_2(SO_4)_3$, les phosphates et phosphosilicates de fer et de lithium de structure olivine, ou leurs produits de substitution du fer par le manganèse, utilisés seuls ou en mélanges.

**38.** Générateur selon la revendication **35**, **caractérisé en ce que** le collecteur de la cathode est en aluminium.

**39.** Supercapacité utilisant au moins une électrode de carbone à haute surface spécifique, ou une électrode contenant un polymère rédox, dans laquelle l'électrolyte est un matériau selon l'une des revendications 1 à 34

**40.** Utilisation d'un matériau selon l'une des revendications 1 à 34 pour le dopage p ou n d'un polymère à conduction électronique.

**41.** Dispositif électrochrome, dans lequel l'électrolyte est un matériau selon l'une des revendications 1 à 34.

**42.** Matériau à conduction électronique, **caractérisé en ce qu'**il comprend un composé ionique selon la revendication 1.

**43.** Matériau à conduction électronique selon la revendication 42 , **caractérisé en ce que** la partie cationique du composé ionique est un polycation constitué par un polymère conjugué dopé "p".

**44.** Matériau à conduction électronique selon la revendication 42, **caractérisé en ce que** le substituant Z du composé ionique comprend une chaîne alkyle ayant de 6 à 20 atomes de carbone.

## Claims

**1.** Ionically conducting material comprising an ionic compound in solution in a solvent, the said ionic compound being derived from malononitrile and comprising an anionic part associated to at least one cationic part $M^{m+}$ in sufficient number to provide electronic neutrality of the assembly, **characterized in that** M is an hydroxonium, a nitrosonium $NO^+$, an ammonium $-NH_4^+$, a metallic cation having a valency m, an organic cation having a valency m or an organometallic cation having a valency m, and **in that** the anionic part corresponds to one of the formulae $R_D$-Y-C(C≡N)$_2^-$ or Z-C(C≡N)$_2^-$ in which :

- Z represents an electroattractor radical having a Hammett parameter at least equal to that of a phenyl radical, selected from :

  j) -CN, $-NO_2$, -SCN, $-N_3$, $-CF_3$, $FSO_2$-, $R'_FCH_2$- ($R'_F$ being a perfluorinated radical), fluoroalkyloxy, fluoroalkylthioxy, fluoroalkenyloxy, fluoroalkenylthioxy radicals ;
  jj) radicals comprising one or more aromatic nuclei optionally containing at least one nitrogen, oxygen, sulfur or phosphorus atom, said nuclei optionally being condensed nuclei and/or said nuclei optionally carrying at least one substituent selected from halogens, -CN, $-NO_2$, -SCN, $-N_3$, $CF_2$=CF-O- radicals, $R_F$- and $R_F$-$CH_2$- in which $R_F$ represents a perfluoroalkyl radical having 1 to 12 carbon atoms, fluoroalkyloxy groups, fluoroalkylthioxy groups, alkyl, alkenyl, oxa-alkyl, oxa-alkenyl, aza-alkyl, aza-alkenyl, thia-alkyl, thia-alkenyl radicals, polymer radicals, radicals having at least one cationic ionophorous group and/or at least one anionic ionophorous group ;

- Y represents a carbonyl group, a thiocarbonyl group, a sulfonyl group, a sulfinyl group or a phosphonyl group and :
- $R_D$ is a radical selected from :

  a) alkyl or alkenyl radicals having from 1 to 24 carbon atoms, and aryl, arylalkyl, alkylaryl or alkenylaryl

radicals having from 5 to 24 carbon atoms, alicyclic or heterocyclic radicals, including polycyclic radicals ;
b) alkyl or alkenyl radicals having from 1 to 12 carbon atoms and comprising optionally at least one heteroatom O, N or S in the main chain or in the side chain, and/or comprising at least one functional ether, thioether, amine, imine, amide, carboxyl, carbonyl, isocyanate, isothiocyanate, thiocarbonyl or hydroxy group ; oxa-alkyl, oxa-alkenyl, aza-alkyl, aza-alkenyl, thia-alkyl, or thia-alkenyl radicals having from 1 to 24 carbon atoms;
c) aryl, arylalkyl, arylalkenyl, alkylaryl or alkenylaryl radicals, in which the aromatic nuclei and/or at least one substituent of the nucleus comprises heteroatoms such as nitrogen, oxygen, sulfur;
d) radicals comprising condensed aromatic cycles which optionally comprise at least one heteroatom selected from nitrogen, oxygen, sulfur ;
e) halogenated or perhalogenated alkyl, alkenyl, aryl, arylalkyl, alkylaryl radicals, said radicals optionally comprising functional ether, thioether, imine, amine, carboxyl, carbonyl or hydroxy groups ;f) radicals $R_CC(R')(R")$-O- in which $R_C$ is an perfluorinated alkyl radical and R' and R" are independently from one another an hydrogen atom or a radical such as defined in a), b), c) or d) above ;
g) radicals $(R_B)_2N$-, in which the radicals $R_B$ which are identical or different are such as defined in a), b), c), d) and e) above, one of the $R_B$ may be a halogen atom, or the two radicals $R_B$ together form a divalent radical which constitutes a cycle with N ;
h) poly(oxyalkylene) and poly(oxyalkylene)alkylether radicals,
i) radicals having one or more cationic ionophorous groups and/or one or more anionic ionophorous groups ;

with the proviso that:

- a substituent Z may be a monovalent radical, part of a radical having a valency of 2 and carrying two $-C^-(C\equiv N)_2$ groups, or a segment of a polymer ;
- a substituent $R_D$ may be a monovalent radical, part of a multivalent radical carrying a plurality of $-Y-C^-(C\equiv N)_2$ groups or a segment of a polymer ;when Y is a carbonyl and $R_D$ is a perfluoroalkyl radical having 1 to 3 carbon atoms, or when Z is -CN, M is different from an alkali metal;
- "segment of a polymer" means an ethylene recurring unit optionally carrying an alkylene substituent or an oxyalkylene substituent, an oxyalkylene recurring unit optionally carrying a methylene group, a styrene recurring unit optionally carrying a polyoxyethylene substituent, a 2,3-epoxypropane recurring unit, an azaethylene recurring unit, a 2-(triethoxysilyl)ethyl-co-O-[2-(trimethoxysilyl)ethyl]-O'-methylpolyethylene glycol recurring unit, a bis([3-trimethoxy-silyl)-propyl]amino recurring unit, a methylethylsiloxane recurring unit, a pyrrole recurring unit, an acrylonitrile-co-4-styrenyle recurring unit, or an aniline recurring unit carrying a fluoroalkyl substituent;
- "cationic ionophoric group" means an iodonium, sulfonium, oxonium, ammonium, amidinium, guanidinium, pyridinium, imidazolium, imidazolinium, triazolium, phosphonium or carbonium group, said ionic group acting totally or partly as the cation $M^+$ ;
- "anionic ionophoric group" means a carboxylate function ($-CO_2^-$), a sulfonate function ($-SO_3^-$), a sulfonimide function ($-SO_2NSO_2$-) or a sulfonamide function ($-SO_2N$-).

**2.** Ionically conducting material according to claim 1, **characterized in that** the organic cation is an onium cation selected from the group consisting of $R_3O^+$ (oxonium), $NR_4^+$ (ammonium), $RC(NR_2)_2^+$ (amidinium), $C(NR_2)_3^+$ (guanidinium), $C_5R_6N^+$ (pyridinium), $C_3R_5N_2^+$ (imidazolium), $C_3R_7N_2^+$ (imidazolinium), $C_2R_4N_3^+$ (triazolium), $SR_3^+$ (sulfonium), $PR_4^+$ (phosphonium), $IR_2^+$ (iodonium), $(C_6R_5)_3C^+$ (carbonium), the radicals R representing independently from one another a H or a radical selected from the group consisting of :

- alkyl, alkenyl, oxa-alkyl, oxa-alkenyl, aza-alkyl, aza-alkenyl, thia-alkyl, thia-alkenyl, aryl, arylalkyl, alkylaryl, alkenylaryl radicals, dialkylamino radicals and dialkylazo radicals;
- cyclic or heterocyclic radicals optionally comprising at least one lateral chain comprising heteroatoms such as nitrogen, oxygen, sulfur ;
- cyclic or heterocyclic radicals optionally comprising heteroatoms in the aromatic nucleus ;
- groups comprising a plurality of aromatic or heterocyclic nuclei, condensed or non-condensed, optionally containing at least one nitrogen, oxygen, sulfur or phosphorus atom;

with the proviso that a plurality of radicals R may together form aliphatic or aromatic cycles, optionally enclosing the center carrying the cationic charge.

3. Ionically conducting material according to claim 2, **characterized in that** the onium cation is part of the radical Z or the radical $R_D$.

4. Ionically conducting material according to claim 2, **characterized in that** the onium cation is part of a recurring unit of a polymer.

5. Ionically conducting material according to claim 2, **characterized in that** the cation $M^+$ is a cationic heterocycle with aromatic character, having at least one alkylated nitrogen atom in the cycle.

6. Ionically conducting material according to claim 5, **characterized in that** the cation is an imidazolium, a triazolium, a pyridinium, a 4-dimethyl-amino-pyridinium, said cations optionally carrying a substituent on the carbon atoms of the cycle.

7. Ionically conducting material according to claim 2, **characterized in that** the cation M is a group having a -N=N, $-N=N^+$ bond, a sulfonium group, a phosphonium group, an iodonium group, or an arene-ferrocenium cation which is substituted or non-substituted, optionally incorporated in a polymeric network.

8. Ionically conducting material according to claim 2, **characterized in that** the cation is a diaryliodonium cation, a dialkylaryliodonium cation, a triarylsulfonium cation, a trialkylaryl sulfonium cation, or a substituted or non-substituted phenacyl-dialkyl sulfonium cation.

9. Ionically conducting material according to claim 2, **characterized in that** the aryliodonium cation, or the alkylaryliodonium cation, or the triarylsulfonium cation, or the alkylaryl sulfonium cation, or the phenacyl-dialkyl sulfonium cation are part of a polymer chain.

10. Ionically conducting material according to claim 2, **characterized in that** M is an organic cation incorporating a 2,2'[Azobis(2-2'-imidazolinio-2-yl)propane]$^{2+}$ or a 2,2'-Azobis(2-amidiniopropane)$^{2+}$ group.

11. Ionically conducting material according to claim 1, **characterized in that** the cation M is a metallic cation selected from the group consisting of cations of alkali metals, cations of alkali-earth metals, cations of transition metals, cations of trivalent metals and cations of rare earths.

12. Ionically conducting material according to claim 1, **characterized in that** the cation is a metallocenium, selected from the group consisting of cations derived from ferrocene, titanocene and zirconocene, indenocenium cations, arene metallocenium cations, cations of transition metals complexed with phosphine ligands optionally having a chirality, and organometallic cations having one or more alkyl or aryl groups covalently bound to an atom or a group of atoms, said cations optionally being part of a polymer chain.

13. Ionically conducting material according to claim I, **characterized in that** the substituent Z is selected from the group consisting of-$OC_nF_{2n+1}$, $-OC_2F_4H$, $-SC_nF_{2n+1}$ and $-SC_2F_4H$, -O-CF=$CF_2$, -SCF=$CF_2$, n being a whole number from 1 to 8.

14. Ionically conducting material according to claim 1, **characterized in that** Z is a radical $C_nF_{2n+1}CH_2$-, n being a whole number from 1 to 8.

15. Ionically conducting material according to claim 1, **characterized in that** $R_D$ is part of a poly (oxyalkylene) radical or a polystyrene radical.

16. Ionically conducting material according to claim 1, **characterized in that** $R_D$ includes at least one ethylenic unsaturation and/or a condensable group and/or a group which is dissociable by thermal means, by photochemical means or by ionic dissociation.

17. Ionically conducting material according to claim 1, **characterized in that** $R_D$ represents a mesomorphous group or a chromophore group or a self-doped electronically conductive polymer or a hydrolysable alkoxysilane.

18. Ionically conducting material according to claim 1, **characterized in that** $R_D$ represents a recurring unit of a polymer chain.

**19.** Ionically conducting material according to claim 1, **characterized in that** $R_D$ includes a group capable of trapping free radicals.

**20.** Ionically conducting material according to claim 1, **characterized in that** $R_D$ includes a dissociating dipole.

**21.** Ionically conducting material according to claim 1, **characterized in that** $R_D$ includes a redox couple.

**22.** Ionically conducting material according to claim 1, **characterized in that** $R_D$ includes a complexing ligand.

**23.** Ionically conducting material according to claim 1, **characterized in that** RD-Y- is optically active.

**24.** ionically conducting material according to claim 1, **characterized in that** $R_D$-CO- represents an amino acid, or an optically or biologically active polypeptide.

**25.** Ionically conducting material according to claim 1, **characterized in that** $R_D$ represents a radical having a valency v higher than 2, having at least one $-Y-C(C\equiv N)_2^{-}M^{+}$ group.

**26.** Ionically conducting material according to claim 1, **characterized in that** the substituent $R_D$ of the ionic compound is an alkyl or an alkenyl comprising at least one heteroatom selected from O, N and S ; an alkyl or an alkenyl carrying a hydroxy group, a carbonyl group, an amino group, a carboxyl group ; an aryl, an arylalkyl, an alkylaryl or an alkenylaryl in which the side chains and/or the aromatic nuclei comprise heteroatoms.

**27.** Material according to claim 1, **characterized in that** the substituent $R_D$ is a recurring unit of a polymer.

**28.** Ionically conducting material according to claim 1, **characterized in that** the solvent is either a liquid aprotic solvent selected form linear ethers and cyclic ethers, esters, nitriles, nitro derivatives, amides, sulfones, sulfolanes, alkylsulfamides and partly halogenated hydrocarbons, or a polar polymer, or a mixture thereof.

**29.** Ionically conducting material according to claim 28, **characterized in that** the solvent is a solvating polymer, cross-linked or non cross-linked, optionally carrying grafted ionic groups.

**30.** Ionically conducting material according to claim 29, **characterized** the solvating polymer is selected from polyethers of linear, comb or blocks structure, which may form a network based on poly(ethylene oxide), copolymers containing the ethylene oxide or propylene oxide or allylglycidylether unit, polyphosphazenes, cross-linked networks based on polyethylene glycol cross-linked with isocyanates, networks obtained by polycondensation and carrying groups enabling the incorporation of cross-linkable groups and block copolymers in which some blocks carry functions which have redox properties.

**31.** Ionically conducting material according to claim 1, **characterized in that** the solvent consists essentially of an aprotic liquid solvent and a polar polymer solvent comprising units containing at least one heteroatom selected from sulfur, oxygen, nitrogen and fluorine.

**32.** Ionically conducting material according to claim 31, **characterized in that** the polar polymer mainly contains units derived from acrylonitrile, vinylidene fluoride, N-vinylpyrrolidone or methyl methacrylate.

**33.** Ionically conducting material according to claim 1, **characterized in that** it additionally contains at least one second salt.

**34.** Ionically conducting material according to claim 1, **characterized in that** it additionally contains a mineral or organic charge in the form of powder or fibres.

**35.** Electrochemical generator comprising a negative electrode and a positive electrode separated by an electrolyte, **characterized in that** the electrolyte is a material according to one of claims 1 to 34.

**36.** Generator according to claim 35, **characterized in that** the negative electrode consists of metallic lithium, or an alloy thereof, optionally in the form of a nanometric dispersion in lithium oxide, or a double nitride of lithium and a transition metal, or an oxide with low potential having the general formula $Li_{1+y+x/3}Ti_{2-x/3}O_4$ ($0 \leq x \leq 1$, $0 \leq y \leq 1$), or carbon or carbonated products derived from pyrolysis of organic materials.

**37.** Generator according to claim 35, **characterized in that** the positive electrode is selected from vanadium oxides $VO_x$ ($2 \le x \le 2{,}5$), $LiV_3O_8$, $Li_yNi_{1-x}Co_xO_2$, ($0 \le x \le 1$ ; $0 \le y \le 1$), spinels of manganese $Li_yMn_{1-x}M_xO_2$ (M = Cr, Al, V, Ni, $0 \le x \le 0{,}5$ ; $0 \le y \le 2$), organic polydisulfides, FeS, $FeS_2$, iron sulfate $Fe_2(SO_4)_3$, phosphates and phosphosilicates of iron and lithium of olivine structure, or products thereof in which the iron is substituted by manganese, used alone or in mixtures.

**38.** Generator according to claim 35, **characterized in that** the cathode collector is made of aluminium.

**39.** Supercapacitor utilizing at least one carbon electrode of high specific surface, or an electrode containing a redox polymer, in which the electrolyte is a material according to one of claims 1 to 34.

**40.** Utilization of a material according to one of claims 1 to 34, for doping p or n in an electronically conductive polymer.

**41.** Electrochrome device in which the electrolyte is a material according to one of claims 1 to 34.

**42.** Electronically conducting material, **characterized in that** it comprises an ionic compound according to claim 1.

**43.** Electronically conducting material according to claim 42, **characterized in that** the cationic part of the ionic compound is a polycation consisting of a doped "p" conjugated polymer.

**44.** Electronically conducting material according to claim 42, **characterized in that** the substituent Z of the ionic compound comprises an alkyl chain having 6 to 20 carbon atoms.

## Patentansprüche

**1.** Ionenleitendes Material, das eine ionische Verbindung in Lösung in einem Lösungsmittel umfasst, wobei die ionische Verbindung ein Malononitril-Derivat ist, das einen anionischen Anteil umfasst, der mit zumindest einem kationischen Anteil $M^{+m}$ in ausreichender Zahl assoziiert ist, um elektrische Neutralität des Ganzen zu gewährleisten, **dadurch gekennzeichnet, dass** M ein Hydroxonium-, ein Nitrosonium $NO^+$, ein Ammonium- $-NH_4^+$, ein Metallkation mit der Wertigkeit m, ein organisches Kation mit der Wertigkeit m oder ein organometallisches Kation mit der Wertigkeit m ist, sowie dadurch, dass der anionische Anteil einer der Formeln $R_D\text{-}Y\text{-}C(C{\equiv}N)_2^-$ oder $Z\text{-}C(C{\equiv}N)_2^-$ entspricht, worin:

- Z einen Elektronen-anziehenden Rest mit einem Hammett-Parameter darstellt, der zumindest dem eines Phenylrests entspricht, ausgewählt aus:

  j) -CN, $-NO_2$, -SCN, $-N_3$, $-CF_3$, $FSO_2$-, $R'_FCH_2$- (wobei $R'_F$ ein perfluorierter Rest ist), Fluoralkyloxy-, Fluoralkylthioxy-, Fluoralkenyloxy- und Fluoralkenylthioxy-Resten;
  jj) Resten mit einem oder mehreren aromatischen Kernen, die gegebenenfalls zumindest ein Stickstoff-, Sauerstoff-, Schwefel- oder Phosphoratom enthalten, wobei die Kerne gegebenenfalls kondensierte Kerne sind und/oder die Kerne gegebenenfalls zumindest einen Substituenten aufweisen, der aus Halogenen, -CN, $-NO_2$, -SCN, $-N_3$, $CF_2$=CF-O-, den Resten $R_F$- und $R_F$-$CH_2$-, worin $R_F$ für einen Perfluoralkylrest mit 1 bis 12 Kohlenstoffatomen steht, Fluoralkyloxygruppen, Fluoralkylthioxygruppen, Alkyl-, Alkenyl, Oxaalkyl-, Oxaalkenyl-, Azaalkyl-, Azaalkenyl-, Thiaalkyl-, Thiaalkenylresten, Polymerresten, sowie Resten, die zumindest eine kationische ionophore Gruppe und/oder zumindest eine anionische ionophore Gruppe aufweisen;

- Y für eine Carbonylgruppe, eine Thiocarbonylgruppe, eine Sulfonylgruppe, eine Sulfinylgruppe oder eine Phosphonylgruppe steht, und
- $R_D$ ein aus Folgenden ausgewählter Rest ist:

  a) Alkyl- oder Alkenylresten mit 1 bis 24 Kohlenstoffatomen und Aryl-, Arylalkyl-, Alkylaryl- oder Alkenylarylresten mit 5 bis 24 Kohlenstoffatomen, alizyklischen oder heterozyklischen Resten, einschließlich polyzyklischen Resten;
  b) Alkyl- oder Alkenylresten mit 1 bis 12 Kohlenstoffatomen, die gegebenenfalls zumindest ein Heteroatom O, N oder S in der Hauptkette oder in einer Seitenkette enthalten und/oder die zumindest eine funktionelle Ether-, Thioether-, Amino-, Imino-, Amid-, Carboxyl-, Carbonyl-, Isocyanat-, Isothiocyanat-, Thiocarbonyl-

oder Hydroxygruppe umfassen; Oxaalkyl-, Oxaalkenyl-, Azaalkyl-, Azaalkenyl-, Thiaalkyl- oder Thiaalkenylresten mit 1 bis 24 Kohlenstoffatomen;

c) Aryl-, Arylalkyl-, Arylalkenyl-, Alkylaryl- oder Alkenylarylresten, worin die aromatischen Kerne und/oder zumindest ein Kernsubstituent Heteroatome, wie z.B. Stickstoff, Sauerstoff oder Schwefel, umfassen;

d) Resten, die kondensierte aromatische Ringe umfassen, die gegebenenfalls zumindest ein aus Stickstoff, Sauerstoff und Schwefel ausgewähltes Heteroatom umfassen,

e) halogenierten oder perhalogenierten Alkyl-, Alkenyl-, Aryl-, Arylalkyl- oder Alkylarylresten, wobei die Reste gegebenenfalls funktionelle Ether-, Thioether-, Imino-, Amino-, Carboxyl-, Carbonyl oder Hydroxygruppen umfassen;

f) Resten $R_CC(R')(R'')$-O-, worin $R_C$ ein perfluorierter Alkylrest ist und R' und R'' unabhängig voneinander ein Wasserstoffatom oder ein Rest wie zuvor unter a), b), c) oder d) definiert ist;

g) Resten $(R_B)_2N$-, worin die Reste $R_B$, die identisch oder unterschiedlich sein können, wie zuvor unter a), b), c), d) und e) definiert sind, wobei ein $R_B$ ein Wasserstoffatom sein kann, oder die beiden Reste $R_B$ gemeinsam einen zweiwertigen Rest bilden, der mit N einen Ring bildet;

h) Poly(oxyalkylen)- und Poly(oxyalkylen)alkyletherresten,

i) Resten, die eine oder mehrere kationische ionophore Gruppen und/oder eine oder mehrere anionische ionophore Gruppen aufweisen;

mit der Maßgabe, dass

- ein Substituent Z ein einwertiger Rest, ein Teil eines Rests mit der Wertigkeit 2, der zwei Gruppen $-C^-(C{\equiv}N)_2$ trägt, oder ein Segment eines Polymers sein kann;
- ein Substituent $R_D$ ein einwertiger Rest, ein Teil eines Rests mit einer Wertigkeit von über 2, der mehrere Gruppen $-Y-C^-(C{\equiv}N)_2$ aufweist, oder ein Polymersegment sein kann;
- wenn Y ein Carbonyl ist und $R_D$ ein Perfluoralkylrest mit 1 bis 3 Kohlenstoffatomen ist oder wenn Z -CN ist, M kein Alkalimetall ist;
- "Polymersegment" eine Ethylen-Grundeinheit, die gegebenenfalls einen Alkylensubstituenten oder einen Oxyalkenylensubstituenten aufweist, eine Oxyalkylen-Grundeinheit, die gegebenenfalls eine Methylengruppe aufweist, eine Styrol-Grundeinheit, die gegebenenfalls einen Polyoxyethylensubstituenten aufweist, eine 2,3-Epoxypropan-Grundeinheit, eine Azaethylen-Grundeinheit, eine 2-(Triethoxysilyl)ethyl-co-O-[2-(trimethoxysilyl)ethyl]-O'-methylpolyethylenglykol-Grundeinheit, eine Bis[3-trimethoxysilyl)propyl]amino-Grundeinheit, eine Methylethylsiloxan-Grundeinheit, eine Pyrrol-Grundeinheit, eine Acrylnitril-co-4-styrenyl-Grundeinheit oder eine Anilin-Grundeinheit ist, die einen Fluoralkylsubstituenten aufweist;
- "kationische ionophore Gruppe" eine Iodonium-, Sulfonium- Oxonium-, Ammonium-, Amidinium-, Guanidinium-, Pyridinium- Imidazolium-, Imidazolinium, Triazolium-, Phosphonium- oder Carboniumgruppe bezeichnet, wobei diese ionischen Gruppen vollständig oder teilweise die Rolle von Kation $M^+$ übernehmen;
- "ionophore anionische Gruppe" eine Carboxylat-Funktionalität ($-CO_2^-$), eine Sulfonat-Funktionalität ($-SO_3$), eine Sulfonimid-Funktionalität ($-SO_2NSO_2-$) oder eine Sulfonamid-Funktionalität ($-SO_2N-$) bezeichnet.

**2.** Ionenleitendes Material nach Anspruch 1, **dadurch gekennzeichnet, dass** das organische Kation ein Oniumkation ist, das aus der aus den Kationen $R_3O^+$ (Oxonium), $NR_4^+$ (Ammonium), $RC(NR_2)_2^+$ (Amidinium), $C(NR_2)_3^+$ (Guanidinium), $C_5R_6N^+$ (Pyridinium), $C_3R_5N_2^+$ (Imidazolium), $C_3R_7N_2^+$ (Imidazolinium), $C_2R_4N_3^+$ (Triazolium), $SR_3^+$ (Sulfonium), $PR_4^+$ (Phosphonium), $IR_2^+$ (Iodonium) und $(C_6R_5)_3C^+$ (Carbonium) bestehenden Gruppe ausgewählt ist, wobei die Reste R unabhängig voneinander ein H oder einen Rest darstellen, der aus der aus Folgenden bestehenden Gruppe ausgewählt ist:

- Alkyl-, Alkenyl-, Oxaalkyl-, Oxaalkenyl-, Azaalkyl-, Azaalkenyl-, Thiaalkyl-, Thiaalkenyl-, Aryl-, Arylalkyl-, Alkylaryl-, Alkenylarylresten, Dialkylaminoresten und Dialkylazoresten;
- zyklischen oder heterozyklischen Resten, die gegebenenfalls zumindest eine Seitenkette umfassen, die Heteroatome, wie z.B. Stickstoff, Sauerstoff oder Schwefel, umfasst;
- zyklischen oder heterozyklischen Resten, die gegebenenfalls Heteroatome im aromatischen Kern umfassen;
- Gruppen, die mehrere kondensierte oder nichtkondensierte, aromatische oder heterozyklische Kerne umfassen, die gegebenenfalls zumindest ein Stickstoff-, Sauerstoff-, Schwefel- oder Phosphoratom enthalten;

mit der Maßgabe, dass mehrere Reste R gemeinsam aromatische Ringe bilden können, die gegebenenfalls das die kationische Ladung tragende Zentrum umfassen.

**3.** Ionenleitendes Material nach Anspruch 2, **dadurch gekennzeichnet, dass** das Oniumkation Bestandteil von Rest

Z oder von Rest $R_D$ ist.

4. Ionenleitendes Material nach Anspruch 2, **dadurch gekennzeichnet, dass** das Oniumkation Bestandteil einer Grundeinheit eines Polymers ist.

5. Ionenleitendes Material nach Anspruch 2, **dadurch gekennzeichnet, dass** das Kation $M^+$ ein kationischer Heterozyklus mit aromatischer Beschaffenheit ist, der im Ring zumindest ein alkyliertes Stickstoffatom aufweist.

6. Ionenleitendes Material nach Anspruch 5, **dadurch gekennzeichnet, dass** das Kation ein Imidazolium, Triazolium, Pyridinium oder 4-Dimethylaminopyridinium ist, wobei die Kationen an den Kohlenstoffatomen des Rings gegebenenfalls einen Substituenten tragen.

7. Ionenleitendes Material nach Anspruch 2, **dadurch gekennzeichnet, dass** das Kation M eine Gruppe ist, die eine -N=N- oder $-N=N^+$-Bindung, eine Sulfoniumgruppe, eine Phosphoniumgruppe, eine Iodoniumgruppe oder ein substituiertes oder unsubstituiertes Aren-Ferrocen-Kation ist, das gegebenenfalls in einem Polymereinschluss enthalten ist.

8. Ionenleitendes Material nach Anspruch 2, **dadurch gekennzeichnet, dass** das Kation ein substituiertes oder unsubstituiertes Diaryliodoniumkation, Dialkylaryliodoniumkation, Triarylsulfoniumkation, Trialkylarylsulfoniumkation oder Phenacyldialkylsulfoniumkation ist.

9. Ionenleitendes Material nach Anspruch 2, **dadurch gekennzeichnet, dass** das Aryliodoniumkation oder das Alkylaryliodoniumkation oder das Triarylsulfoniumkation oder das Alkylarylsulfoniumkation oder das Phenacyldialkylsulfoniumkation Teil einer Polymerkette sind.

10. Ionenleitendes Material nach Anspruch 2, **dadurch gekennzeichnet, dass** M ein organisches Kation ist, das eine. Gruppe 2,2'-[Azobis(2,2'-imidazolinio-2-yl)propan]$^{2+}$ oder 2,2'-Azobis(2-amidiniopropan)$^{2+}$ umfasst.

11. Ionenleitendes Material nach Anspruch 1, **dadurch gekennzeichnet, dass** das Kation M ein Metallkation ist, das aus der aus Alkalimetallkationen, Erdalkalimetallkationen, Übergangsmetallkationen, dreiwertigen Metallkationen und Seltenderdkationen bestehenden Gruppe ausgewählt ist.

12. Ionenleitendes Material nach Anspruch 1, **dadurch gekennzeichnet, dass** das Kation ein Metallocenium ist, das aus der aus den von Ferrocen, Titanocen und Zirconocen abgeleiteten Kationen, Indenoceniumkationen, Arenmetalloceniumkationen, Kationen von Übergangsmetallen, die mit Liganden vom Phosphintyp komplexiert sind, die gegebenenfalls Chiralität aufweisen, und organometallischen Kationen, die eine oder mehrere Alkyl- oder Arylgruppen aufweisen, die kovalent an ein Atom oder eine Atomgruppe gebunden sind, bestehenden Gruppe ausgewählt ist, wobei die Kationen gegebenenfalls Teil einer Polymerkette sind.

13. Ionenleitendes Material nach Anspruch 1, **dadurch gekennzeichnet, dass** der Substituent Z aus der aus $-OC_nF_{2n+1}$, $-OC_2F_4H$, $-SC_nF_{2n+1}$ und $-SC_2F_4H$, $-OCF=CF_2$ und $-SCF = CF_2$ bestehenden Gruppe ausgewählt ist, wobei n eine ganze Zahl von 1 bis 8 ist.

14. Ionenleitendes Material nach Anspruch 1, **dadurch gekennzeichnet, dass** Z ein Rest $C_nF_{2n+1}CH_2$- ist, wobei n eine ganze Zahl von 1 bis 8 ist.

15. Ionenleitendes Material nach Anspruch 1, **dadurch gekennzeichnet, dass** $R_D$ Teil eines Poly(oxyalkylen)rests oder eines Polystyrolrests ist.

16. Ionenleitendes Material nach Anspruch 1, **dadurch gekennzeichnet, dass** $R_D$ zumindest eine ethylenische Unsättigung und/oder eine kondensierbare Gruppe und/oder eine thermisch, photochemisch oder durch ionische Dissoziation dissoziierbare Gruppe umfasst.

17. Ionenleitendes Material nach Anspruch 1, **dadurch gekennzeichnet, dass** $R_D$ eine mesomorphe Gruppe oder eine chromophore Gruppe oder ein eigendotiertes elektronenleitendes Polymer oder ein hydrolysierbares Alkoxysilan darstellt.

18. Ionenleitendes Material nach Anspruch 1, **dadurch gekennzeichnet, dass** $R_D$ eine Grundeinheit einer Polymerkette darstellt.

19. Ionenleitendes Material nach Anspruch 1, **dadurch gekennzeichnet, dass** $R_D$ eine als Radikalfänger dienende Gruppe umfasst.

20. Ionenleitendes Material nach Anspruch 1, **dadurch gekennzeichnet, dass** $R_D$ einen dissoziierbaren Dipol umfasst.

21. Ionenleitendes Material nach Anspruch 1, **dadurch gekennzeichnet, dass** $R_D$ ein Redoxpaar umfasst.

22. Ionenleitendes Material nach Anspruch 1, **dadurch gekennzeichnet, dass** $R_D$ einen komplexierenden Liganden umfasst.

23. Ionenleitendes Material nach Anspruch 1, **dadurch gekennzeichnet, dass** $R_D$-Yoptisch aktiv ist.

24. Ionenleitendes Material nach Anspruch 1, **dadurch gekennzeichnet, dass** $R_DCO$ eine Aminosäure oder ein optisch oder biologisch aktives Polypeptid darstellt.

25. Ionenleitendes Material nach Anspruch 1, **dadurch gekennzeichnet, dass** $R_D$ einen Rest mit einer Wertivkeit v von über 2 darstellt, der zumindest eine Gruppe $-Y-C(C{\equiv}N)_2^-M^+$ umfasst.

26. Ionenleitendes Material nach Anspruch 1, **dadurch gekennzeichnet, dass** der Substituent $R_D$ der ionischen Verbindung ein Alkyl oder ein Alkenyl ist, das zumindest ein aus O, N und S ausgewähltes Heteroatom enthält; ein Alkyl oder ein Alkenyl, das eine Hydroxygruppe, eine Carbonylgruppe, eine Aminogruppe oder eine Carboxylgruppe aufweist; ein Aryl, ein Arylakyl, ein Alkylaryl oder ein Alkenylaryl, worin die Seitenketten und/oder die aromatischen Kerne Heteroatome umfassen.

27. Material nach Anspruch 1, **dadurch gekennzeichnet, dass** der Substituent $R_D$ eine Grundeinheit eines Polymers ist.

28. Ionenleitendes Material nach Anspruch 1, **dadurch gekennzeichnet, dass** das Lösungsmittel entweder ein flüssiges, aprotisches Lösungsmittel, das aus linearen und zyklischen Ethern, Estern, Nitrilen, Nitroderivaten, Amiden, Sulfonen, Sulfolanen, Alkylsulfamiden und Kohlenwasserstoffen ausgewählt ist, die teilweise halogeniert sind, oder ein polares Polymer oder ein Gemisch davon ist.

29. Ionenleitendes Material nach Anspruch 28, **dadurch gekennzeichnet, dass** das Lösungsmittel ein gegebenenfalls vernetztes, solvatisierendes Polymer ist, das gegebenenfalls aufgepfropfte ionische Gruppen aufweist.

30. Ionenleitendes Material nach Anspruch 29, **dadurch gekennzeichnet, dass** das solvatisierende Polymer aus Polyethern mit linearer, kammförmiger oder blockförmiger Struktur, die gegebenenfalls ein Netzwerk bilden, auf Basis von Poly(ethylenoxid), Copolymeren, die eine Ethylenoxid- oder Propylenoxid- oder Allylglycidylether-Einheit enthalten, Polyphosphazenen, vernetzten Netzwerken auf Basis von Polyethylenglykol, das durch Isocyanate vernetzt ist, Netzwerken, die durch Polykondensation erhalten werden und Gruppen aufweisen, die den Einbau vernetzbarer Gruppen ermöglichen, und Blockcopolymeren ausgewählt sind, in denen bestimmte Blöcke Funktionalitäten enthalten, die Redoxeigenschaften aufweisen.

31. Ionenleitendes Material nach Anspruch 1, **dadurch gekennzeichnet, dass** das Lösungsmittel im Wesentlichen aus einem flüssigen aprotischen Lösungsmittel und einem polaren Polymerlösungsmittel besteht, das Einheiten umfasst, die zumindest ein aus Schwefel, Sauerstoff, Stickstoff und Fluor ausgewähltes Heteroatom enthalten.

32. Ionenleitendes Material nach Anspruch 31, **dadurch gekennzeichnet, dass** das polare Polymer vorwiegend Einheiten enthält, die von Acrylnitril, Vinylidenfluorid, N-Vinylpyrrolidon oder Methylmethacrylat herrühren.

33. Ionenleitendes Material nach Anspruch 1, **dadurch gekennzeichnet, dass** es weiters ein zweites Salz enthält.

34. Ionenleitendes Material nach Anspruch 1, **dadurch gekennzeichnet, dass** es weiters einen mineralischen oder organischen Füllstoff in Pulver- oder Faserform enthält.

**35.** Elektrochemischer Generator, der eine negative Elektrode und eine positive Elektrode umfasst, die durch einen Elektrolyten voneinander getrennt sind, **dadurch gekennzeichnet, dass** der Elektrolyt ein Material nach einem der Ansprüche 1 bis 34 ist.

**36.** Generator nach Anspruch 35, **dadurch gekennzeichnet, dass** die negative Elektrode aus metallischem Lithium oder einer Legierung davon, gegebenenfalls in Form einer manometrischen Dispersion in Lithiumoxid, oder aus einem Lithium-Übergangsmetall-Doppelnitrid oder aus einem Oxid mit niedrigem Potential der allgemeinen Formel $Li_{1+y+x/3}Ti_{2-x/3}O_4$ ($0 \leq x \leq 1$, $0 \leq y \leq 1$), oder aus Kohlenstoff und kohlenstoffhältigen Produkten der Pyrolyse organischer Materialien besteht.

**37.** Generator nach Anspruch 35, **dadurch gekennzeichnet, dass** die positive Elektrode aus Vanadiumoxiden $VO_x$ ($2 \leq x \leq 2{,}5$), $LiV_3O_8$, $Li_yNi_{1-x}Co_xO_2$ ($0 \leq x \leq 1$; $0 \leq y \leq 1$), Manganspinellen $Li_yMn_{1-x}M_xO_2$ (M = Cr, Al, V, Ni, $0 \leq x \leq 0{,}5$; $0 \leq y \leq 2$), organischen Polydisulfiden, FeS, $FeS_2$, Eisensulfat $Fe_2(SO_4)_3$, Eisen- und Lithiumphosphaten und -phosphosilikaten mit Olivinstruktur oder Produkten davon, die durch Ersetzen von Eisen durch Mangan, alleine oder als Gemisch ausgewählt ist.

**38.** Generator nach Anspruch 35, **dadurch gekennzeichnet, dass** der Stromabnehmer der Kathode aus Aluminium besteht.

**39.** Superkondensator, bei dem zumindest eine Kohlenstoffelektrode mit großer spezifischer Oberfläche oder eine Elektrode die ein Redoxpolymer enthält eingesetzt wird, worin der Elektrolyt ein Material nach einem der Ansprüche 1 bis 34 ist.

**40.** Verwendung eines Materials nach einem der Ansprüche 1 bis 34 zur p- oder n-Dotierung eines elektronenleitenden Polymers.

**41.** Elektrochrome Vorrichtung, in der der Elektrolyt ein Material nach einem der Ansprüche 1 bis 34 ist.

**42.** Elektronenleitendes Material, **dadurch gekennzeichnet, dass** es ein ionenleitendes Material nach Anspruch 1 enthält.

**43.** Elektronenleitendes Material nach Anspruch 42, **dadurch gekennzeichnet, dass** der kationische Anteil der ionischen Verbindung ein Polykation ist, das aus einem p-dotierten konjugierten Polymer besteht.

**44.** Elektronenleitendes Material nach Anspruch 42, **dadurch gekennzeichnet, dass** der Substituent Z der ionischen Verbindung eine Alkylkette mit 6 bis 20 Kohlenstoffatomen umfasst.

Figure 1